(19) **Europäisches Patentamt** / **European Patent Office** / **Office européen des brevets**

(11) **EP 3 222 284 A1**

(12) **EUROPEAN PATENT APPLICATION**

(43) Date of publication:
**27.09.2017 Bulletin 2017/39**

(21) Application number: **17166614.2**

(22) Date of filing: **13.02.2012**

(51) Int Cl.:
*A61K 38/16* (2006.01)    *A61K 9/16* (2006.01)
*A61K 9/00* (2006.01)    *A61K 31/4422* (2006.01)
*A61K 9/06* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.02.2011 US 201161441695 P**

(62) Document number(s) of the earlier application(s) in accordance with Art. 76 EPC:
**12744708.4 / 2 672 983**

(71) Applicant: **Edge Therapeutics, Inc.**
**Berkeley Heights, NJ 07922 (US)**

(72) Inventors:
• LEUTHNER, Brian A.
  **Summit, New Jersey 07091 (US)**
• MACDONALD, Loch R.
  **Scottsdale, Arizona 85259 (US)**

(74) Representative: **Beresford Crump LLP**
**16 High Holborn**
**London**
**WC1V 6BX (GB)**

Remarks:
This application was filed on 13-04-2017 as a divisional application to the application mentioned under INID code 62.

(54) **COMPOSITIONS AND METHODS FOR IMPROVING PROGNOSIS OF A HUMAN WITH SUBARACHNOID HEMORRHAGE**

(57)    The described invention provides a pharmaceutical composition, delivery system and a method for treating a delayed complication associated with a brain injury in a mammal and improving its prognosis in a mammal by implanting a therapeutically effective amount of the pharmaceutical composition containing a voltage-gated calcium channel blocker and a pharmaceutically acceptable carrier into a predetermined location in the brain, which reduces signs or symptoms of at least one delayed complication associated with brain injury.

FIG. 1

EP 3 222 284 A1

**Description**

**CROSS REFERENCE TO RELATED APPLICATIONS**

This application claims priority to U.S. provisional application No. 61/441,695 filed February 11, 2011, and is a continuation in part of U.S. Application No. 12/137,320, filed June 11, 2008, which claims priority to U.S. Provisional Application No. 60/976,902 filed October 29, 2007 and U.S. Provisional Application No. 60/943,124 filed June 11, 2007. Each of these applications is incorporated by reference herein in its entirety.

**FIELD OF INVENTION**

The described invention relates to compositions, systems and methods for treating adverse consequences of a subarachnoid hemorrhage.

**BACKGROUND OF THE INVENTION**

The human brain constitutes only about 2% of the total weight of the body, but it receives about 15% of cardiac output, and its oxygen consumption is approximately 20% of that for the total body. These values indicate the high metabolic rate and oxygen requirement of the brain that are compensated by a correspondingly high rate of blood flow per unit brain weight. Cerebral circulation is supplied by the internal carotid arteries and the vertebral arteries. The total blood flow to the brain is about 750-1000 ml/min; of this amount about 350 ml flows through each internal carotid artery and about 100-200 ml flows through the vertebral basilar system. The venous outflow is drained by the internal jugular veins and the vertebral veins.

The term "stroke" or "cerebrovascular accident" as used herein refers to the neurological symptoms and signs, usually focal and acute, that result from diseases involving blood vessels. Strokes are either occlusive (due to closure of a blood vessel) or hemorrhagic (due to bleeding from a vessel). The term "ischemia" as used herein refers to a lack of blood supply and oxygen that occurs when reduced perfusion pressure distal to an abnormal narrowing (stenosis) of a blood vessel is not compensated by autoregulatory dilation of the resistance vessels. When ischemia is sufficiently severe and prolonged, neurons and other cellular elements die; this condition is referred to as "infarction."

Hemorrhage may occur at the brain surface (extraparenchymal), for example from the rupture of congenital aneurysms at the circle of Willis, causing subarachnoid hemorrhage (SAH). Hemorrhage also may be intraparenchymal, for example from rupture of vessels damaged by long-standing hypertension, and may cause a blood clot (intracerebral hematoma) within the cerebral hemispheres, in the brain stem, or in the cerebellum. Hemorrhage may be accompanied by ischemia or infarction. The mass effect of an intracerebral hematoma may compromise the blood supply of adjacent brain tissue; or subarachnoid hemorrhage may cause reactive vasospasm of cerebral surface vessels, leading to further ischemic brain damage. Infarcted tissue may also become secondarily hemorrhagic. Aneurysms occasionally can rupture into the brain, causing an intracerebral hematoma, and into the cerebral ventricles, causing intraventricular hemorrhage.

Although most occlusive strokes are due to atherosclerosis and thrombosis and most hemorrhagic strokes arc associated with hypertension or aneurysms, strokes of either type may occur at any age from many causes, including: cardiac disease, trauma, infection, neoplasm, blood dyscrasia, vascular malformation, immunological disorder, and exogenous toxins.

**CEREBRAL ARTERIES**

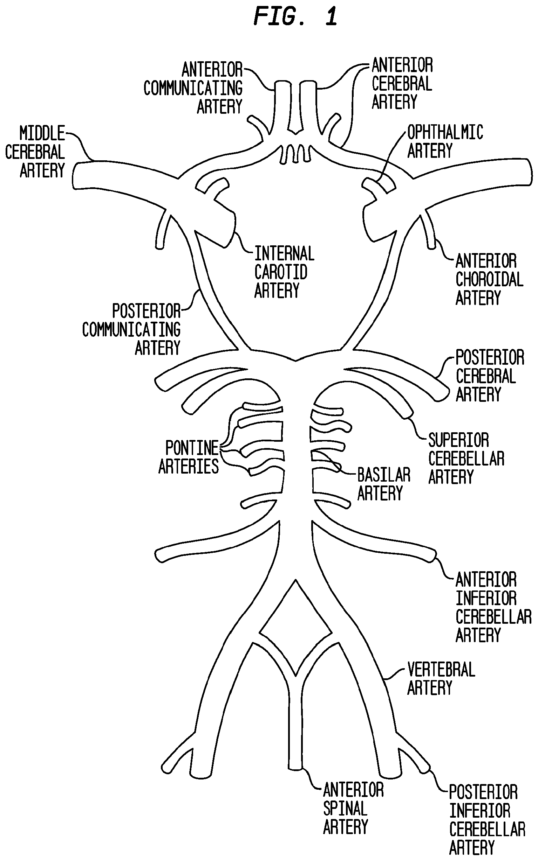

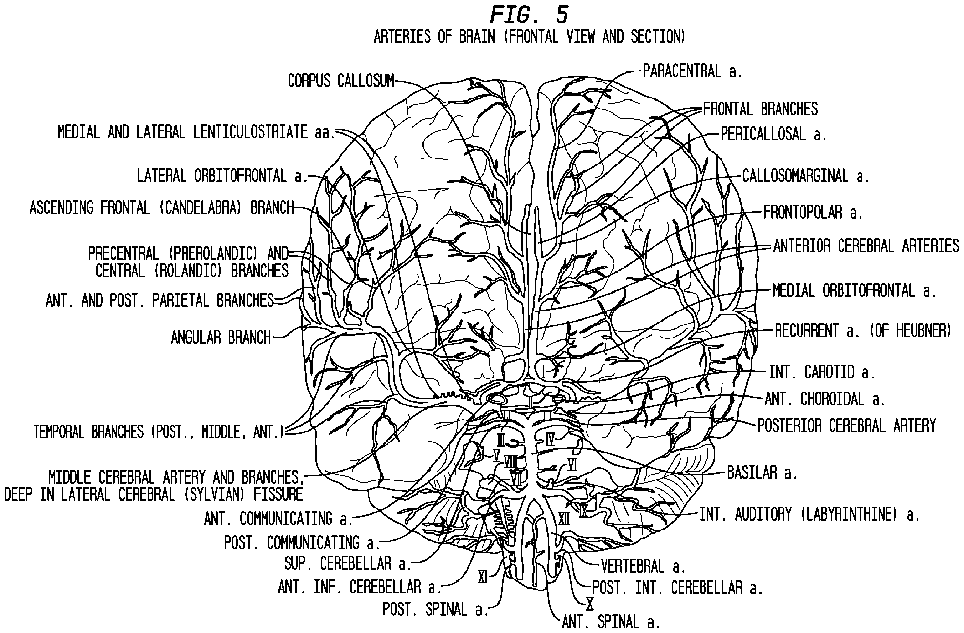

Figures 1 and 5 show schematic illustrations of the brain's blood vessels. Each cerebral hemisphere is supplied by an internal carotid artery, which arises from a common carotid artery beneath the angle of the jaw, enters the cranium through the carotid foramen, traverses the cavernosus sinus (giving off the ophthalmic artery), penetrates the dura and divides into the anterior and middle cerebral arteries. The large surface branches of the anterior cerebral artery supply the cortex and white matter of the inferior frontal lobe, the medial surface of the frontal and parietal lobes and the anterior corpus callosum. Smaller penetrating branches supply the deeper cerebrum and diencephalon, including limbic structures, the head of the caudate, and the anterior limb of the internal capsule. The large surface branches of the middle cerebral artery supply most of the cortex and white matter of the hemisphere's convexity, including the frontal, parietal, temporal and occipital lobes, and the insula. Smaller penetrating branches supply the deep white matter and diencephalic structures such as the posterior limb of the internal capsule, the putamen, the outer globus pallidus, and the body of the caudate. After the internal carotid artery emerges from the cavernous sinus, it also gives off the anterior choroidal artery, which supplies the anterior hippocampus and, at a caudal level, the posterior limb of the internal capsule. Each vertebral artery arises from a subclavian artery, enters the cranium through the foramen magnum, and gives off an anterior spinal artery and a posterior inferior cerebellar artery. The vertebral arteries join at the junction of the pons and the medulla to form the basilar artery, which at the level of the pons gives off the anterior inferior cerebellar artery and the internal auditory artery, and, at the midbrain, the superior cerebellar artery. The basilar artery then divides into the two posterior cerebral arteries. The large surface branches of the posterior cerebral arteries supply the inferior temporal and medial occipital lobes and the posterior corpus callosum; the smaller penetrating branches of these arteries supply diencephalic structures, including the thalamus and the subthalamic nuclei, as well as part of the midbrain (see Principles of Neural Sciences, 2d Ed., Eric R. Kandel and James H. Schwartz, Elsevier Science Publishing Co., Inc., New York, pp. 854-56 (1985)).

Interconnections between blood vessels (anastomoses) protect the brain when part of its vascular supply is compromised. Anastomoses are interconnections between blood vessels that protect the brain when part of its vascular supply is compromised. At the circle of Willis, the two anterior cerebral arteries are connected by the anterior communicating artery and the posterior cerebral arteries are connected to the internal carotid arteries by the posterior communicating arteries. Other important anastomoses include connections between the ophthalmic artery and branches of the external carotid artery through the orbit, and connections at the brain surface between branches of the middle, anterior, and posterior cerebral arteries (Principles of Neural Sciences, 2d Ed., Eric R. Kandel and James H. Schwartz, Elsevier Science Publishing Co., Inc., New York, pp. 854¬56 (1985)).

When referring to animals, that typically have one end with a head and mouth, with the opposite end often having the anus and tail, the head end is referred to as the cranial end, while the tail end is referred to as the caudal end. Within the head itself, rostral refers to the direction toward the end of the nose, and caudal is used to refer to the tail direction. The surface or side of an animal's body that is normally oriented upwards, away from the pull of gravity, is the dorsal side; the opposite side, typically the one closest to the ground when walking on all legs, swimming or flying, is the ventral side. On the limbs or other appendages, a point closer to the main body is "proximal"; a point farther away is "distal". Three basic reference planes are used in zoological anatomy. A "sagittal" plane divides the body into left and right portions. The "midsagittal" plane is in the midline, i.e. it would pass through midline structures such as the spine, and all other sagittal planes are parallel to it. A "coronal" plane divides the body into dorsal and ventral portions. A "transverse" plane divides the body into cranial and caudal portions. When referring to humans, the body and its parts are always described using the assumption that the body is standing upright. Portions of the body which are closer to the head end are "superior" (corresponding to cranial in animals), while those farther away are "inferior" (corresponding to caudal in animals). Objects near the front of the body are referred to as "anterior" (corresponding to ventral in animals); those near the rear of the body are referred to as "posterior" (corresponding to dorsal in animals). A transverse, axial, or horizontal plane is an X- Y plane, parallel to the ground, which separates the superior/head from the inferior/feet. A coronal or frontal plane is an Y-Z plane, perpendicular to the ground, which separates the anterior from the posterior. A sagittal plane is an X-Z plane, perpendicular to the ground and to the coronal plane, which separates left from right. The midsagittal plane is the specific sagittal plane that is exactly in the middle of the body.

Structures near the midline are called medial and those near the sides of animals are called lateral. Therefore, medial structures are closer to the midsagittal plane, lateral structures are further from the midsagittal plane. Structures in the midline of the body are median. For example, the tip of a human subject's nose is in the median line.

Ipsilateral means on the same side, contralateral means on the other side and bilateral means on both sides. Structures that are close to the center of the body are proximal or central, while ones more distant are distal or peripheral. For example, the hands are at the distal end of the arms, while the shoulders are at the proximal ends.

Cerebral ventricles, which are chambers in the brain that contain cerebrospinal fluid, include two lateral ventricles, one third ventricle, and one fourth ventricle. The lateral ventricles are in the cerebral hemispheres. They drain via the foramen of Monroe into the third ventricle, which is located between the two diencephalic structures of the brain. The third ventricle lead, by way of the aqueduct of Sylvius, to the fourth ventricle. The fourth ventricle is in the posterior fossa between the brainstem and the cerebellum. The cerebrospinal fluid drains out of the fourth ventricle through the foramenae of Luschka and Magendie to the basal cisterns. The cerebrospinal fluid then percolates through subarachnoid cisterns and drains out via arachnoid villi into the venous system.


**VASOCONSTRICTION AND VASODILATION**


The term "vasoconstriction" as used herein refers to the narrowing of the blood vessels resulting from contracting of the muscular wall of the vessels. When blood vessels constrict, the flow of blood is restricted or slowed. The term "vasodilation", which is the opposite of vasoconstriction as used herein, refers to the widening of blood vessels. The terms "vasoconstrictors," "vasopressors," or "pressors" as used herein refer to factors causing vasoconstriction. Vasoconstriction usually results in an increase of blood pressure and may be slight or severe. Vasoconstriction may result from disease, medication, or psychological conditions. Medications that cause vasoconstriction include, but are not limited to, catecholamines, antihistamines, decongestants, methylphenidate, cough and cold combinations, pseudoephedrine, and caffeine.

A vasodilator is a drug or chemical that relaxes the smooth muscle in blood vessels causing them to dilate.

Dilation of arterial blood vessels (mainly arterioles) leads to a decrease in blood pressure. The relaxation of smooth muscle relies on removing the stimulus for contraction, which depends predominately on intracellular calcium ion concentrations and phosphorylation of myosin light chain (MLC). Thus, vasodilation predominantly works either 1) by lowering intracellular calcium concentration, or 2) by dephosphorylation of MLC, which includes the stimulation of myosin light chain phosphatase and the induction of calcium symporters and antiporters (which pump calcium ions out of the intracellular compartment). The re-uptake of ions into the sarcoplasmic reticulum of smooth muscle via exchangers and expulsion of ions across the plasma membrane also helps to accomplish vasodilation. The specific mechanisms to accomplish these effects vary from vasodilator to vasodilator and may be grouped as endogenous and exogenous. The term "endogenous" as used herein refers to proceeding from within or derived internally; or resulting from conditions within the organism rather than externally caused. The term "exogenous" as used herein refers to originating from outside; derived externally; or externally caused rather than resulting from conditions within the organism.

Vasodilation directly affects the relationship between mean arterial pressure and cardiac output and total peripheral resistance (TPR). Cardiac output may be computed by multiplying the heart rate (in beats/minute) and the stroke volume (the volume of blood ejected during systole). TPR depends on several factors, including, but not limited to, the length of the vessel, the viscosity of blood (determined by hematocrit), and the diameter of the blood vessel. Blood vessel diameter is the most important variable in determining resistance. An increase in either cardiac output or TPR cause a rise in the mean arterial pressure. Vasodilators work to decrease TPR and blood pressure through relaxation of smooth muscle cells in the tunica media layer of large arteries and smaller arterioles.

Vasodilation occurs in superficial blood vessels of warm-blooded animals when their ambient environment is hot; this process diverts the flow of heated blood to the skin of the animal, where heat may be more easily released into the atmosphere. Vasoconstriction is the opposite physiological process. Vasodilation and vasoconstriction are modulated naturally by local paracrine agents produced by endothelial cells (e.g., bradykinin, adenosine), as well as by an organism's autonomic nervous system and adrenal glands, both of which secrete catecholamines, such as norepinephrine and epinephrine, respectively.

Vasodilators are used to treat conditions such as hypertension, where the patient has an abnormally high blood pressure, as well as angina and congestive heart failure, where maintaining a lower blood pressure reduces the patient's risk of developing other cardiac problems.

## CEREBRAL VENTRICLES

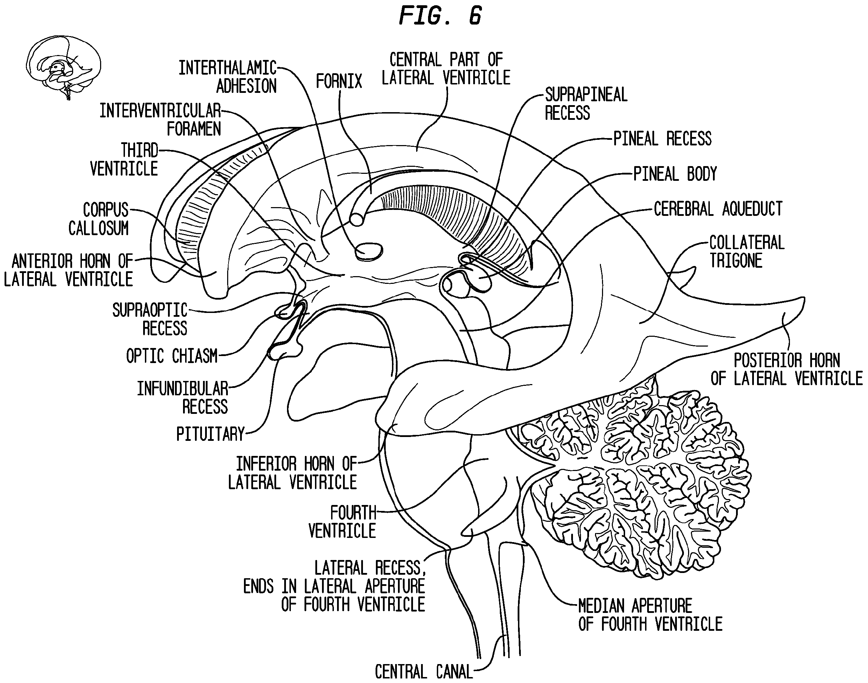

Figure 6 is a diagram of the ventricular system of the brain. The system is a series of cavities (ventricles) within the brain and is continuous with both the subarachnoid space and central canal of the spinal cord. There are four cerebral ventricles: the right and left lateral ventricles, and the midline third and fourth ventricles. The two lateral ventricles are located within the cerebrum and each connects to the third ventricle through an interventricular foramen of Monroe. The third ventricle is located in the diencephalon and is connected to the fourth ventricle by the cerebral aqueduct of Sylvius. The fourth ventricle is located in the hind brain and it is continuous with the central canal of the spinal cord, at least embryologically. Three foramina connect the fourth ventricle to the subarachnoid space: the median aperture or foramen of Magendie, and left and right lateral apertures (foramena) of Luschka.

## CSF FLOW IN THE BRAIN

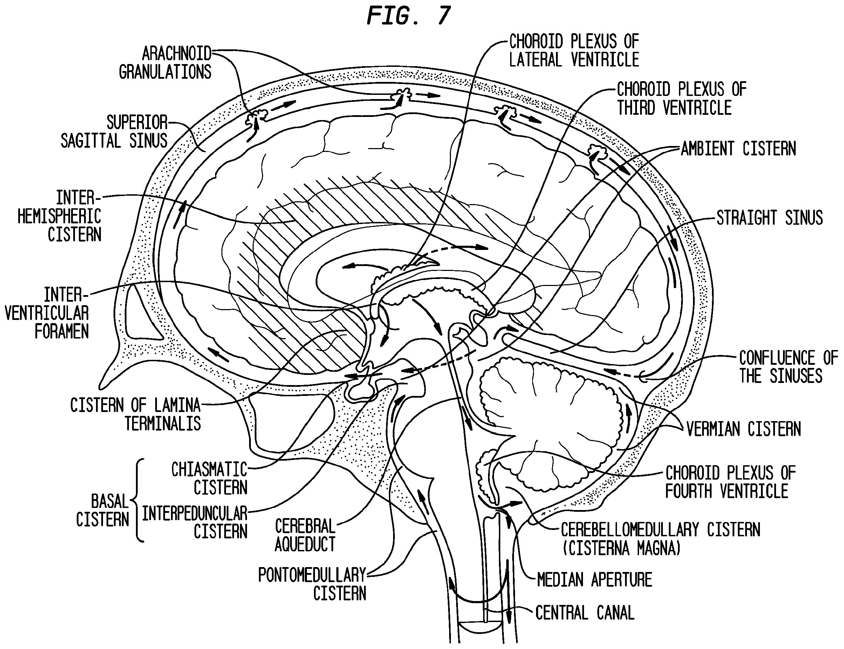

Figure 7 shows an illustrative view of the CSF flow from the ventricles to the subarachnoid space. The cerebrospinal fluid (CSF) is a clear bodily fluid that occupies the ventricular system, subarachnoid space of the brain, and central canal of the spinal cord. CSF is produced by modified ependymal cells of the choroid plexus found throughout the ventricular system. In addition, it is also formed around blood vessels and ventricular walls, presumably from the extracellular space of the brain. CSF flows from the lateral ventricles via interventricular foramina into the third ventricle. CSF then flows into the fourth ventricle through the cerebral aqueduct. CSF flows out in the subarachnoid space via the median aperture and left and right lateral apertures. Finally, the CSF is reabsorbed into the dural venous sinuses through arachnoid granulations and arachnoid villi. Arachnoid granulations consist of collections of villi. The villi are visible herniations of the arachnoid membrane through the dura and into the lumen of the superior sagittal sinus and other venous structures. The granulations appear to function as valves that allow one-way flow of CSF from the subarachnoid spaces into venous blood. All constituents of CSF leave with the fluid, including small molecules, proteins, microorganisms, and red blood cells.

CSF is produced at a rate of approximately 0.3-0.37 ml/minute or 20 ml/hour or 500 ml/day. The volume of the CSF space is about 150 mls and the CSF turns over 3.7 times a day.

The choroid plexus uses capillary filtration and epithelial secretory mechanisms to maintain the chemical stability of the CSF. While the capillaries that traverse the choroid plexus are freely permeable to plasma solutes, a barrier exists at the level of the epithelial cells that make up the choroid plexus, which is responsible for carrier-mediated active transport. CSF and extracellular fluids of the brain are in a steady state and blood plasma and CSF are in osmotic equilibrium under normal physiological conditions.

## BLOOD BRAIN BARRIER

The blood brain barrier prevents entry of blood-borne substances into the brain and maintains a stable environment for neurons to function effectively. It results from specialized properties of brain microvessel endothelial cells, the principal anatomic site of the blood brain barrier, their intercellular junctions, and a relative lack of vesicular transport, which makes such cells different from those of general capillaries. Endothelial cells of blood-brain barrier vessels also are not fenestrated; instead they are interconnected by complex arrays of tight junctions, which block diffusion across the vessel wall.

## SUBARACHNOID HEMORRHAGE

The brain is encased by three layers of membranes or meninges: the pia mater, arachnoid mater, and dura mater. The subarachnoid space is the area between the arachnoid membrane and the pia mater surrounding the brain. The term "subarachnoid hemorrhage" (also referred to as "SAH") refers to bleeding into the subarachnoid space. SAH may occur spontaneously, usually from a cerebral aneurysm, or may result from trauma. Symptoms include an intense headache with a rapid onset (sometimes referred to as a "thunderclap headache"), vomiting, and an altered level of consciousness. Diagnosis generally is made with computed tomography (CT scanning), or occasionally by lumbar puncture. Treatment is by close observation, medication and early neurosurgical investigations and treatments to prevent recurrence and complications.

SAH is a medical emergency and may lead to death or severe disability even if recognized and treated at an early stage. Half of all SAH cases are fatal, with 10-15% of patients dying before arriving at a hospital. SAH is considered a form of stroke, and causes between 1 % and 7% of all strokes. Where caused by a rupture of an intracranial aneurysm, bleeding is seen in the subarachnoid space, and less commonly in the intraventricular and intracerebral spaces. Bleeding due to SAH may result in brain damage, brain shift, decreased cerebral perfusion and hydrocephalus. It is estimated that the incidence of SAH from a ruptured intracranial aneurysm in the U.S. is 1 case per 10,000 persons, yielding approximately 27,000 - 30,000 new cases of SAH each year. These ruptured aneurysms have a 30-day mortality rate of 45%. Further, an estimated 30% of survivors will have moderate-to-severe disability.

Some studies indicate the incidence of SAH is on average 9.1 per 100,000 annually. Studies from Japan and Finland show higher rates in those countries (22.7 per 100,000 and 19.7 per 100,000, respectively), for reasons that are not entirely understood. South and Central America, in contrast, have a rate of 4.2 per 100,000 on average. The group of people at risk for SAH is younger than the population usually affected by stroke, but the risk still increases with age. Young people are much less likely than middle-aged people (risk ratio 0.1, or 10%) to suffer a SAH. The risk continues to rise with age and is 60% higher in the very elderly (over 85) than in those between 45 and 55. Risk of SAH is about 25% higher in women above 55, potentially reflecting the hormonal changes that result from the menopause.

Patients who survive SAH also are at risk of secondary complications. Among these complications are, most notably, aneurysmal re-bleeding, angiographic cerebral vasospasm and delayed cerebral ischemia (DCI).

DCI is the occurrence of focal neurological impairment (such as hemiparesis, aphasia, apraxia, hemianopia, or neglect), or a decrease in the Glasgow coma scale (either the total score or one of its individual components [eye, motor on either side, verbal]).This may or may not last for at least one hour, is not apparent immediately after aneurysm occlusion and cannot be attributed to other causes by means of clinical assessment, CT or magnetic resonance imaging (MRI) scanning of the brain, and appropriate laboratory studies. Cerebral infarction may be a consequence of DCI, and is defined as the presence of cerebral infarction on CT or MRI scan of the brain within 6 weeks after SAH, or on the latest CT or MRI scan made before death within 6 weeks, or proven at autopsy, not present on the CT or MRI scan between 24 and 48 hours after early aneurysm occlusion, and not attributable to other causes such as surgical clipping or endovascular treatment. Hypodensities on CT imaging resulting from ventricular catheter or intraparenchymal hematoma generally are not regarded as cerebral infarctions from DCI. Angiographic cerebral vasospasm is a description of a radiological test (either CT angiography [CTA], MR angiography [MRA] MRA or catheter angiography [CA]), and may be a cause of DCI. The term "angiographic cerebral vasospasm" refers to the narrowing of the large capacitance arteries at the base of the brain (i.e., cerebral arteries) following hemorrhage into the subarachnoid space, and leads to reduced perfusion of distal brain regions. Angiographic vasospasm is a consequence of SAH, but also can occur after any condition that deposits blood in the subarachnoid space.

### Symptoms

The classic symptom of SAH is thunderclap headache (a headache described as the "worst ever" or a "kick in the head," developing over seconds to minutes) although it is a symptom in only about a third of all SAH patients. Approximately 10% of patients who seek medical care with this symptom have an underlying SAH. Patients also may present with vomiting, and 1 in 14 have seizures. Neck stiffness and other signs of meningism may be present, as may confusion, decreased level of consciousness, or coma. Intraocular hemorrhage may occur in response to the raised pressure around the brain. Subhyaloid (the hyaloid membrane envelopes the vitreous body of the eye) and vitreous hemorrhage may be visible on fundoscopy. This is known as Terson syndrome (occurring in 3-13% of cases), and is more common in more severe SAH. In a patient with thunderclap headache, none of the aforementioned signs are helpful in confirming or ruling out hemorrhage, although seizures are more common if the bleeding is the result of a ruptured aneurysm as opposed to other causes. Oculomotor nerve abnormalities (affected eye movement downward and outward, inability to lift the eyelid on the same side but normal pupillary reflexes) may indicate bleeding from an aneurysm arising near the posterior communicating artery. Isolated dilation of a pupil may also reflect brain herniation as a result of rising intracranial pressure.

The body releases large amounts of adrenaline and similar hormones as a result of the bleeding, which leads to a sharp increase in the blood pressure. The heart comes under substantial strain, and neurogenic pulmonary edema, stunned myocardium, cardiac arrhythmias, electrocardiographic changes (with occasional giant inverted "cerebral" T waves) and cardiac arrest (3%) may rapidly occur after the onset of hemorrhage.

SAH also may occur in people who have suffered a head injury. Symptoms may include headache, decreased level of consciousness or hemiparesis. SAH is regarded as a severe complication of head injury, especially if it is associated with lower Glasgow Coma Scale levels.

**Diagnosis**

The initial steps for evaluating a person with a suspected SAH are the steps of obtaining a medical history and performing a physical examination. Since only 10-25% of patients admitted to a hospital with a thunderclap headache are suffering from a SAH, other possible causes usually are considered simultaneously, such as meningitis, migraine, and cerebral venous sinus thrombosis. Intracerebral hemorrhage, which is twice as common as SAH, occasionally is misdiagnosed as SAH.

A diagnosis of SAH cannot be made on clinical grounds alone. Generally, medical imaging [usually computed tomography (CT scan) which has a high sensitivity (>95% correct identification especially on the first day after the onset of bleeding)] of the brain is required to confirm or exclude bleeding. Magnetic resonance imaging (MRI scan) may be more sensitive after several days when compared to CT scan. In people with normal CT or MRI scans, lumbar puncture, in which cerebrospinal fluid (CSF) is removed with a needle from the lumbar sac, shows evidence of hemorrhage in 3% of the group in whom the CT was found to be normal; lumbar puncture is therefore regarded as mandatory if imaging is negative. The CSF sample is examined for xanthochromia, the yellow appearance of centrifugated fluid, or by using spectrophotometry for bilirubin, a breakdown product of hemoglobin in the CSF.

After an SAH is confirmed, its origin needs to be determined. CT angiography (visualizing blood vessels with radiocontrast on a CT scan) to identify aneurysms is generally the first step, although the more invasive catheter angiography (injecting radiocontrast through a catheter advanced to the brain arteries) is the gold standard test but has a higher risk of complications. The latter is useful if there are plans to obliterate the source of bleeding, such as an aneurysm, at the same time.

**Causes**

Spontaneous SAH most often is due to rupture of cerebral aneurysms (85%). Cerebral aneurysms are weaknesses in the walls of arteries of the brain that become enlarged. They tend to be located in the circle of Willis and its branches. While most cases of SAH arc due to bleeding from small aneurysms, larger aneurysms (which are rarer) are more likely to rupture. No aneurysm is detected from the first angiogram in 15-20% of cases of spontaneous SAH. Non-aneurysmal perimesencephalic hemorrhage, in which the blood is limited to the area of the midbrain, causes another 10% of SAH cases. In these, no aneurysms are generally found. The remaining 5% of cases are due to vasculitic damage to arteries, other disorders affecting the vessels, disorders of the spinal cord blood vessels, and bleeding into various tumors. Most traumatic SAHs occur near a skull fracture or intracerebral contusion.

**Classification**

Several grading scales are available for SAH. These have been derived by retrospectively matching characteristics of patients with their outcomes. In addition to the ubiquitously used Glasgow Coma Scale (GCS), three other specialized scores are in use. In all scores, a higher number is associated with a worse outcome. The first scale of severity was described by Hunt and Hess in 1968 ("Hunt and Hess scale") and categorizes the clinical condition of the patient. The Fisher Grade classifies the appearance of SAH on CT scan. The Fisher scale has been modified by Claassen and coworkers ("Claassen scale"), reflecting the additive risk from SAH size and accompanying intraventricular hemorrhage. The World Federation of Neurological Surgeons classification uses GCS and focal neurological deficit to gauge severity of symptoms. A comprehensive classification scheme has been suggested by Ogilvy and Carter to predict outcome and gauge therapy. The Ogilvy system has five grades, assigning one point for the presence or absence of each of five factors: age greater than 50; Hunt and Hess grade 4 or 5; Fischer scale 3 or 4; aneurysm size greater than 10 mm; and posterior circulation aneurysm 25 mm or more.

**Treatment**

The management of SAH consists of general measures to stabilize the patient, specific measures to prevent rebleeding by obliterating the bleeding source, prevention of vasospasm, and prevention and treatment of complications.

**General Measures**

The first priority is to stabilize the patient. Those with a depressed level of consciousness may need to be intubated and mechanically ventilated. Blood pressure, pulse, respiratory rate and Glasgow Coma Scale are monitored frequently. Once the diagnosis is confirmed, admission to an intensive care unit may be preferable, especially given that 15% of such patients have a further episode (rebleeding) in the first hours after admission. Nutrition is an early priority, with oral or nasogastric tube feeding being preferable over parenteral routes. Analgesia (pain control) generally is restricted to non-sedating agents such as codeine, as sedation may impact mental status and thus interfere with the ability to monitor the level of consciousness. Deep vein thrombosis is prevented with compression stockings, intermittent pneumatic compression of the calves, or both.

**Prevention of rebleeding**

Patients with a large hematoma associated with depressed level of consciousness or focal neurological symptoms may be candidates for urgent surgical removal of the blood and occlusion of the bleeding aneurysm. A catheter or tube may be inserted into the ventricles to treat hydrocephalus. The remainder are stabilized more extensively, and undergo a transfemoral catheter angiogram or CT angiogram later. After the first 24 hours, rebleeding risk remains about 40% over the subsequent four weeks, suggesting that interventions should be aimed at reducing this risk.
Rebleeding is hard to predict but may happen at any time and carries a dismal prognosis. Interventions to prevent rebleeding, principally clipping or coiling the ruptured aneurysm, therefore are performed as early as possible. If a cerebral aneurysm is identified on angiography, two measures are available to reduce the risk of further bleeding from the same aneurysm: neurosurgical clipping and endovascular coiling. Clipping requires a craniotomy (opening of the skull) to locate the aneurysm, followed by the placement of a clip or clips across the neck of the aneurysm. Coiling is performed through the large blood vessels: a catheter is inserted into the femoral artery in the groin, and advanced through the aorta to the arteries (both carotid arteries and both vertebral arteries) that supply the brain. When the aneurysm has been located, metallic coils are deployed that lead to formation of a blood clot in the aneurysm and obliteration. The decision as to which treatment is undertaken typically is made by a multidisciplinary team, including a neurosurgeon and a neuroradiologist.
Ancurysms of the middle cerebral artery and its related vessels arc hard to reach with angiography and tend to be amenable to clipping, while those of the basilar artery and posterior cerebral artery are hard to reach surgically and tend to be more accessible for endovascular management. The main drawback of coiling is the possibility that the aneurysm may recur; this risk is extremely small in the surgical approach. Patients who have undergone coiling are typically followed up for many years with angiography or other measures to ensure recurrence of aneurysms is identified early.

**Prognosis**

**Early morbidity and mortality**

The mortality rate for SAH is between 40% and 50%. Of those who survive initial hospitalization, treatment and complications, at least 25% have significant restrictions in their lifestyle, and less than 20% have no residual symptoms whatsoever. Delay in diagnosis of minor SAH without coma (or mistaking the sudden headache for migraine) contributes to poor outcome. Risk factors for poor outcome include higher age, poorer neurological grade, more blood and larger aneurysm on the initial CT scan, location of an aneurysm in the posterior circulation, systolic hypertension, and a previous diagnosis of heart attack, hypertension, liver disease or a previous SAH. During the hospital stay, occurrence of delayed ischemia resulting from vasospasm, development of intracerebral hematoma or intraventricular hemorrhage (bleeding into the ventricles of the brain), and presence of fever on the eighth day of admission also worsen the prognosis.

SAH that does not show an aneurysm by complete catheter angiography may be referred to as "angiogram-negative SAH." This carries a better prognosis than SAH from an aneurysm; however, it still is associated with a risk of ischemia, rebleeding and hydrocephalus. Perimesencephalic SAH (bleeding around the mesencephalon part of the brain), however, has a very low rate of rebleeding or delayed ischemia, and the prognosis of this subtype is excellent.

**Long-term outcomes**

Neurocognitive symptoms, such as fatigue, mood disturbances, and other related symptoms, are common in people who have suffered SAH. Even in those who have made a good neurological recovery, anxiety, depression, posttraumatic stress disorder and cognitive impairment are common. Over 60% report frequent headaches. Aneurysmal SAH may lead to damage of the hypothalamus and the pituitary gland, two areas of the brain that play a central role in hormonal regulation and production. Studies indicate that at least 25% of people with a previous SAH may develop deficiencies in one or more of the hypothalamic-pituitary hormones, such as growth hormone, prolactin or thyroid-stimulating hormone.

**VASOSPASM**

Angiographic cerebral vasospasm is the most common cause of focal ischemia after SAH. It adversely affects outcome in patients with SAH as it accounts for up to 23% of SAH-related disability and death. Of all types of ischemic stroke, vasospasm is unique in that it is to some degree predictable, preventable, and treatable (see Macdonald, R.L. and Weir, B. In Cerebral Vasospasm. Academic Press, Burlington, MA, USA (2001)).

Vasospasm results in decreased cerebral blood flow and increased cerebral vascular resistance. Without being limited by theory, it generally is believed that vasospasm is caused by local injury to vessels, such as that which results from atherosclerosis and other structural injury including traumatic head injury, aneurismal subarachnoid hemorrhage and other causes of subarachnoid hemorrhage. Cerebral vasospasm is a naturally occurring vasoconstriction that also may be triggered by the presence of blood in the CSF, a common occurrence after rupture of an aneurysm or following traumatic head injury. Cerebral vasospasm ultimately can lead to brain cell damage, in the form of cerebral ischemia and infarction, due to interrupted blood supply.

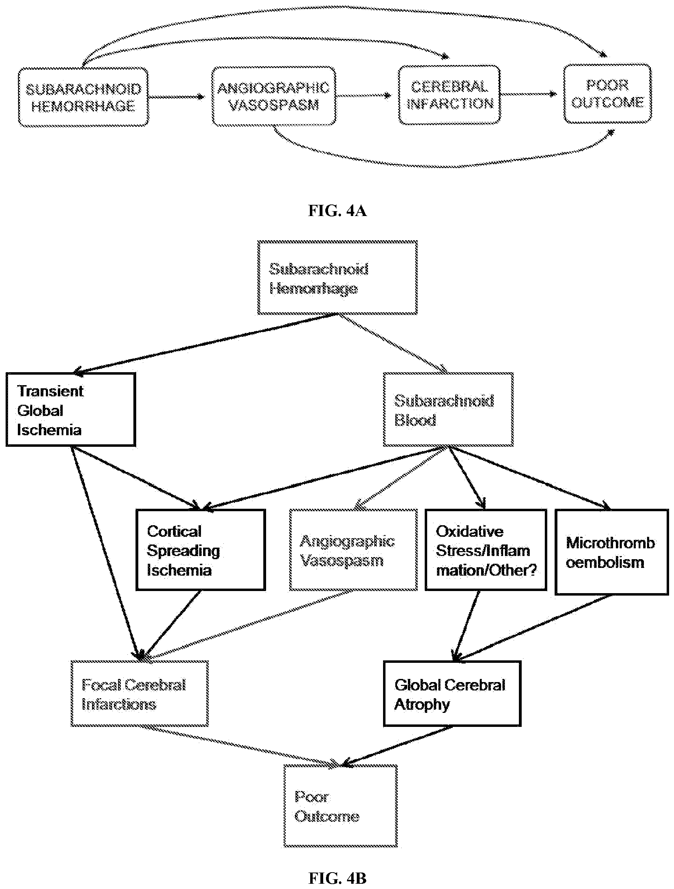

Angiographic vasospasm is one process that contributes to DCI. Other processes that may contribute to DCI are cortical spreading ischemia and microthromboemboli (Figure 4). DCI is a multifactorial process due to at least these processes, as well as to early brain injury. Cortical spreading ischemia was described in animal models of SAH as a novel mechanism that may cause DCI. It has been detected in humans with SAH and angiographic vasospasm. Another process that may contribute to DCI is formation of microthromboemboli.

Each year, about 1 in 10,000 people have an aneurysm rupture. Mortality and morbidity increase with the volume of hemorrhage and reflect the age and health status of the patient, with the chance of developing an aneurysm increasing steadily with age. Rebleeding is exceptionally adverse due to the increase in volume of SAH as well as the increased likelihood of extension into the brain and ventricles. Most deaths resulting from aneurysmal rupture occur outside of hospitals or shortly after admission due to the effects of the initial bleed or early rebleeding. Potential manifestation of symptoms from vasospasm occurs only in those patients who survive past the first few days.

The incidence of vasospasm is less than the incidence of SAH (since only some patients with SAH develop vasospasm). The incidence of vasospasm will depend on the type of patient a given hospital receives and the methods by which vasospasm is diagnosed.

The unqualified term "vasospasm" is usually used with reference to angiographically determined arterial narrowing as defined above. Clinical vasospasm most often is used synonymously with delayed cerebral ischemia (DCI). When used in another fashion, for instance, vasospasm based on increased middle cerebral artery transcranial Doppler velocities, this should be specified.

Some degree of angiographic narrowing will occur in at least two-thirds of patients having angiography between 4 and 12 days after SAH. The numbers of patients developing neurological deterioration from this DCI varies with the diligence with which the patient is monitored and the efficacy of prophylaxis, but it has been estimated at about one-third. Of hospitalized SAH patients, between 5 to 10% die from vasospasm. When compared to post-SAH patients of intermediate grade, post-SAH patients in very good condition are less likely to develop vasospasm as they have small volume SAH, while post-SAH patients in very poor condition are more likely to die earlier from the initial episode. The presence of thick, widespread subarachnoid clot which can be visualized on the computerized tomographic (CT) scan done in close proximity to the bleeding episode is a key prognostic factor. The absence of blood on the initial CT scan is indicative that vasospasm is very unlikely in the absence of rebleeding. The chance of vasospasm and consequently DCI is decreased by factors decreasing the duration of exposure to clot. Conversely, the incidence of vasospasm and DCI is increased by the utilization of antifibrinolytic drugs which prolong the exposure of arteries to clot and possibly cause ischemia by other mechanisms. Poor admission clinical grade is associated with DCI, presumably because they both indicate larger volumes of SAH. A definite relationship between age, hypertension, or sex and DCI has not been established. It is likely that smokers are more prone to vasospasm and DCI. Factors unrelated to the development of vasospasm include season, geography, contrast material, and diabetes.

Patients who develop vasospasm do worse than those who do not. If surgery or aneurysm coiling is performed earlier (within the first day or so) the outcome tends to be better than if treatment is delayed. When operations were preferentially performed during the peak period for vasospasm, outcomes were generally worse. Vasospasm does not result from early surgery or coiling; early surgery or coiling permits more vigorous treatment should vasospasm develop. If a thick clot is present, an attempt at careful removal should be made. The amount of residual clot postoperatively is a prognostic factor for DCI. Open operation exposes the patient to retractor pressure, venous sacrifice, temporary clipping ischemia, brain removal, and arterial injury. Studies have shown post operative decrease in cerebral blood flow, regional cerebral metabolic rate of oxygen, and oxygen extraction ratio.

Independent variables, such as admission neurologic grade, increasing age, and massive intracranial or intraventricular hemorrhage, are more closely linked to outcome than vasospasm. Since vasospasm is a graded process, it is expected that only the extreme cases will result in infarction in the absence of systemic hypotension, cardiac dysfunction, anoxia, and intracranial hypertension. Preexisting hypertension and advanced age also strongly influence the vulnerability of the brain to ischemia. The etiological relationship between vasospasm and infarction in fatal cases is not in dispute.

There is evidence that vasospasm may be reduced by clot removal either surgically or pharmacologically. There also are data suggesting that DCI may be lessened by hypertension and hypervolemia as well as by calcium antagonists. Vasospasm also may be abolished by mechanical or transiently by pharmacologic angioplasty.

**Incidence of Vasospasm**

The incidence of angiographic vasospasm depends on the time interval after the SAH. The peak incidence occurs 6-8 days after SAH (range, 3-12 days). In addition to the time after the SAH, other principal factors that affect the prevalence of vasospasm are the volume and distribution of subarachnoid blood.

**Prognostic Factors for Vasospasm**

Prognostic factors for vasospasm include: blood on CT scan; hypertension; anatomical and systemic factors; clinical grade; whether the patient is receiving antifibrinolytics; age and sex; smoking; physiological parameters; and hydrocephalus.

**Diagnosis**

The diagnosis of vasospasm is primarily clinical. Vasospasm can be asymptomatic; however, when the cerebral blood flow is below ischemic threshold, symptoms become apparent. Symptoms typically develop subacutely and may fluctuate. Symptoms may include excess sleepiness, lethargy, stupor, hemiparesis or hemiplegia, abulia, language disturbances, visual fields deficits, gaze impairment, and cranial nerve palsies. Although some symptoms are localized, they are not diagnostic of any specific pathological process; therefore alternative diagnoses, such as rebleeding, hydrocephalus, and seizures, should be excluded promptly using radiographic, clinical and laboratory assessments. Cerebral angiography is the gold standard for visualizing and studying cerebral arteries; transcranial Doppler ultrasonography is also utilized.

The pathophysiology of vasospasm may involve structural changes and biochemical alterations within the vascular endothelium and smooth muscle cells. The presence of blood in the subarachnoid space may initiate these changes. In addition, hypovolemia and an impaired cerebral autoregulatory function may concurrently interfere with cerebral perfusion. The cumulative effects of these processes can lead to reduction in cerebral blood flow so severe as to cause cerebral ischemia leading to infarction. Additionally, a period of severe constriction could lead to morphologic changes in the walls of the cerebral arteries, which may cause them to remain narrowed without the continued presence of vasoactive substances. The area of the brain supplied by the affected artery then would experience ischemia (meaning a restriction in blood supply).

**Other complications**

Hydrocephalus (a condition marked by an excessive accumulation of CSF resulting in dilation of the cerebral ventricles and raised intracranial pressure) may complicate SAH in both the short- and long-term, and may be detected on CT scanning. If the level of consciousness is decreased, surgical drainage of the excess fluid (for instance with a ventricular drain or shunt) is occasionally necessary.

Fluctuations in blood pressure and electrolyte disturbances, as well as pneumonia and cardiac decompensation, occur in about 50% of hospitalized patients with SAH, and may worsen prognosis. They are managed symptomatically.

Seizures occur in about a third of all cases.

## Treatments

Nimodipine, an oral calcium channel blocker, has been shown in clinical trials to reduce the chance of a poor outcome, however it may not significantly reduce the amount of vasospasm detected on angiography. Other calcium channel blockers and magnesium sulfate have been studied, but are not presently recommended. There is no evidence that shows benefit if nimodipine is given intravenously. In traumatic SAH, the efficacy of oral nimodipine remains in question.

Hemodynamic manipulation, previously referred to as "triple H" therapy, often is used as a measure to treat vasospasm. This entails the use of intravenous fluids to achieve a state of hypertension (high blood pressure), hypervolemia (excess fluid in the circulation) and hemodilution (mild dilution of the blood). Induced hypertension is believed to be the most important component of this treatment although evidence for the use of this approach is inconclusive, and no sufficiently large randomized controlled trials ever have been undertaken to demonstrate its benefits.

If symptomatic vasospasm is resistant to medical treatment, angiography may be attempted to identify the sites of vasospasm and to administer vasodilator medication (drugs that relax the blood vessel wall) directly into the artery (pharmacological angioplasty), and mechanical angioplasty (opening the constricted area with a balloon) may be performed.

## VOLTAGE-GATED ION CHANNELS

Voltage-gated ion channels are a class of integral membrane proteins that allow the passage of selected inorganic ions across the cell membrane by opening and closing in response to changes in transmembrane voltage. (Sands, Z. et al., "Voltage-gated ion channels," Current Biology, 15(2): R44-R47 (2005)). These types of ion channels are especially critical in neurons, but are common in many types of cells. They have an important role in excitable neuronal and muscle tissues as they allow a rapid and coordinated depolarization in response to triggering voltage change. Positioned along the axon and at the synapse, voltage-gated ion channels directionally propagate electrical signals.

## Structure

Voltage-gated potassium, sodium and calcium ion channels are thought to have similar overall architectures. (Sands, Z. et al., "Voltage-gated ion channels," Current Biology, 15(2): R44-R47 (2005)). Voltage-gated ion channels generally are composed of several subunits arranged such that there is a central pore through which ions can travel down their electrochemical gradients. The channels tend to be quite ion-specific, although similarly sized and charged ions may also travel through them to some extent.

## Mechanism

Crystallographic structural studies of a potassium channel, assuming that this structure remains intact in the corresponding plasma membrane, suggest that when a potential difference is introduced over the membrane, the associated electromagnetic field induces a conformational change in the potassium channel. The conformational change distorts the shape of the channel proteins sufficiently such that the channel, or cavity, opens to admit ion influx or efflux to occur across the membrane, down its electrochemical gradient. This subsequently generates an electrical current sufficient to depolarize the cell membrane.

Voltage-gated sodium channels and calcium channels are made up of a single polypeptide with four homologous domains. Each domain contains 6 membrane spanning alpha helices. The voltage sensing helix, S4, has multiple positive charges such that a high positive charge outside the cell repels the helix and induces a conformational change such that ions may flow through the channel. Potassium channels function in a similar way, with the exception that they are composed of four separate polypeptide chains, each comprising one domain. The voltage-sensitive protein domain of these channels (the "voltage sensor") generally contains a region composed of S3b and S4 helices, known as the "paddle" due to its shape, which appears to be a conserved sequence.

## VOLTAGE-DEPENDENT CALCIUM CHANNELS

Voltage-dependent calcium channels (VDCC) are a group of voltage-gated ion channels that control calcium entry into cells in response to membrane potential changes. (Van Petegem F. et al., Biochemical Society Transactions, 34(5): 887-893 (2006)). Voltage-dependent calcium channels are found in excitable cells (e.g., muscle, glial cells, neurons, etc.). At physiologic or resting membrane potential, VDCCs are normally closed. They are activated (i.e., opened) at depolarized membrane potentials. Activation of particular VDCCs allows $Ca^{2+}$ entry into the cell; muscular contraction, excitation of neurons, upregulation of gene expression, or release of hormones or neurotransmitters results, depending upon the cell type. (Catterall W. A. et al., "International Union of Pharmacology. XLVIII. Nomenclature and structure-function relationships of voltage-gated calcium channels," Pharmacol. Rev., 57(4): 411-25 (2005); Yamakage M. et al, "Calcium channels--basic aspects of their structure, function and gene encoding; anesthetic action on the channels--a review," Can. J. Anaesth., 49(2): 151-64 (2002)).

Voltage-dependent calcium channels are formed as a complex of several different subunits: $\alpha_1$, $\alpha_2\delta$, $\beta_{1-4}$, and $\gamma$. The alpha subunit forms the ion conducting pore while the associated subunits have several functions including modulation of gating. (Dolphin A. C. "A short history of voltage-gated calcium channels," Br. J. Pharmacol., 147 (Suppl 1): S56-62 (2006))

### $\alpha_1$ Subunit

The $\alpha_1$ subunit pore (about 190 kDa in molecular mass) is the primary subunit necessary for channel functioning in the VDCC, and consists of the characteristic four homologous I-IV domains containing six transmembrane $\alpha$-helices each. The alpha subunit forms the $Ca^{2+}$ selective pore, which contains voltage-sensing machinery and the drug/toxin-binding sites. Ten alpha subunits that have been identified in humans. (Dolphin A. C. "A short history of voltage-gated calcium channels," Br. J. Pharmacol., 147 (Suppl 1): S56-62 (2006))

### $a_2\delta$ Subunit

The $\alpha_2\delta$ gene encodes two subunits, $\alpha_2$ and $\delta$. They are linked to each other via a disulfide bond and have a combined molecular weight of 170 kDa. The $\alpha_2$ is the extracellular glycosylated subunit that interacts the most with the $\alpha 1$ subunit. The $\delta$ subunit has a single transmembrane region with a short intracellular portion, which serves to anchor the protein in the plasma membrane. There are 4 $\alpha_2\delta$ genes: CACNA2D1 (CACNA2D1), (CACNA2D2), (CACNA2D3), and (CACNA2D4). Co-expression of the $\alpha_2\delta$ enhances the level of expression of the $\alpha 1$ subunit and causes an increase in current amplitude, faster activation and inactivation kinetics and a hyperpolarizing shift in the voltage dependence of inactivation. Some of these effects are observed in the absence of the beta subunit, whereas, in other cases, the co-expression of beta is required. The $\alpha_2\delta$-1 and $\alpha2\delta$-2 subunits are binding sites for at least two anticonvulsant drugs, gabapentin and pregabalin, that also find use in treating chronic neuropathic pain. (Dolphin A. C. "A short history of voltage-gated calcium channels," Br. J. Pharmacol., 147 (Suppl 1): S56-62 (2006))

### $\beta$ Subunit

The intracellular $\beta$ subunit (55 kDa) is an intracellular membrane-associated guanylate kinase (MAGUK)-like protein containing a guanylate kinase (GK) domain and an SH3 (src homology 3) domain. The guanylate kinase domain of the $\beta$ subunit binds to the alpha subunit I-II cytoplasmic loop and regulates HVGCC activity. There are four known isoforms of the $\beta$ subunit: CACNB1, CACNB2, CACNB3, and CACNB4. (Dolphin A. C. "A short history of voltage-gated calcium channels," Br. J. Pharmacol., 147 (Suppl 1): S56-62 (2006))

Without being limited by theory, it is postulated the cytosolic $\beta$ subunit has a major role in stabilizing the final alpha subunit conformation and delivering it to the cell membrane by its ability to mask an endoplasmic reticulum retention signal in the alpha subunit. The endoplasmic retention brake is contained in the I-II loop of the alpha subunit that becomes masked when the $\beta$ subunit binds. Therefore the $\beta$ subunit functions initially to regulate the current density by controlling the amount of alpha subunit expressed at the cell membrane.

In addition to this potential trafficking role, the $\beta$ subunit has the added important functions of regulating activation and inactivation kinetics, and hyperpolarizing the voltage- dependence for activation of the alpha subunit pore, so that more current passes for smaller depolarizations. The $\beta$ subunit acts as an important modulator of channel electrophysiological properties. The interaction between a highly conserved 18-amino acid region on the $\alpha 1$ subunit intracellular linker between domains I and II (the Alpha Interaction Domain, AIDBP) and a region on the GK domain of the $\beta$ subunit (Alpha Interaction Domain Binding Pocket) is responsible for the regulatory effects exerted by the $\beta$ subunit. Additionally, the SH3 domain of the $\beta$ subunit also gives added regulatory effects on channel function, indicating that the P subunit may have multiple regulatory interactions with the $\alpha 1$ subunit pore. The alpha interaction domain sequence does not appear to contain an endoplasmic reticulum retention signal; this may be located in other regions of the I-II $\alpha$1 subunit linker.

**$\gamma$ Subunit**

The $\gamma$1 subunit is known to be associated with skeletal muscle VGCC complexes, but the evidence is inconclusive regarding other subtypes of calcium channel. The $\gamma$1 subunit glycoprotein (33 kDa) is composed of four transmembrane spanning helices. The $\gamma$1 subunit does not affect trafficking, and, for the most part, is not required to regulate the channel complex. However, $\gamma$2, $\gamma$3, $\gamma$4 and $\gamma$8 also are associated with $\alpha$-amino-3-hydroxy-S-methyl-4-isoxazolepropionic acid (AMPA) glutamate receptors, non-NMDA-type ionotropic transmembrane receptors for glutamate that mediate fast synaptic transmissions in the CNS. An NMDA-type receptor is a receptor to which NMDA (N-methyl-D-aspartate) binds specifically. There are 8 genes for gamma subunits: $\gamma$1 (CACNG1), $\gamma$2 (CACNG2), $\gamma$3 (CACNG3), $\gamma$4 (CACNG4), (CACNG5), (CACNG6), (CACNG7), and (CACNG8). (Chu P. J. et al., "Calcium channel gamma subunits provide insights into the evolution of this gene family," Gene, 280 (1-2): 37-48 (2002)).

Voltage dependent calcium channels vary greatly in structure and form. Calcium channels are classified as L-, N-, P/Q, T- and R-type according to their pharmacological and electrophysiological properties. These channel subtypes have distinct physiological functions. Molecular cloning has clarified the $\alpha$1 subunit sequence of each channel. The $\alpha$1 subunit has a specific role in eliciting activity in an individual channel. Nonetheless, selective blockers for these channel subtypes are required for defining specific channels involved in each activity. The neural N-type channels are blocked by $\omega$-conotoxin GVIA; the R-type channels are resistant to other blockers and toxins, are blocked by SNX-482, and may be involved in processes in the brain; the closely related P/Q-type channels are blocked by co-agatoxins. The dihydropyridine-sensitive L-type channels are responsible for excitation-contraction coupling of skeletal, smooth, and cardiac muscle and for hormone secretion in endocrine cells and also are antagonized by phenylalkylamines and benzothiazepines.

**TYPES OF VOLTAGE-GATED CALCIUM CHANNELS**

**L-type Calcium Channels**

L-type voltage-gated calcium channels are opened when a smooth muscle cell is depolarized. This depolarization may be brought about by stretching of the cell, by an agonist-binding its G protein-coupled receptor (GPCR), or by autonomic nervous system stimulation. Opening of the L-type calcium channel causes influx of extracellular $Ca^{2+}$, which then binds calmodulin. The activated calmodulin molecule activates myosin light-chain kinase (MLCK), which phosphorylates the myosin in thick filaments. Phosphorylated myosin is able to form crossbridges with actin thin filaments, and the smooth muscle fiber (i.e., cell) contracts via the sliding filament mechanism. (Yamakage M. et al, "Calcium channels--basic aspects of their structure, function and gene encoding; anesthetic action on the channels--a review," Can. J. Anaesth., 49(2): 151-64 (2002))

L-type calcium channels also are enriched in the t-tubules of striated muscle cells, such as, skeletal and cardiac myofibers. As in smooth muscle, L-type calcium channels open when these cells are depolarized. In skeletal muscle, since the L-type calcium channel and the calcium-release channel (ryanodine receptor, or RYR) are mechanically gated to each other with the latter located in the sarcoplasmic reticulum (SR), the opening of the L-type calcium channel causes the opening of the RYR. In cardiac muscle, opening of the L-type calcium channel permits influx of calcium into the cell. The calcium binds to the calcium release channels (RYRs) in the SR, opening them (referred to as "calcium-induced calcium release" or "CICR"). $Ca^{2+}$ is released from the SR and is able to bind to troponin C on the actin filaments regardless of how the RYRs are opened, either through mechanical-gating or CICR. The muscles then contract through the sliding filament mechanism, causing shortening of sarcomeres and muscle contraction.

**R-type voltage dependent calcium channels**

R-type voltage dependent calcium channels (VDCC) are involved in regulating calcium flow. The R-type VDCCs play an important role in decreased cerebral blood flow observed following SAH. Without being limited by theory, R-type voltage-dependent $Ca^{2+}$ channels that may be located within small diameter cerebral arteries may regulate global and local cerebral blood flow, since the concentration of intracellular free calcium ions determines the contractile state of vascular smooth muscle. Yamakage M. et al, "Calcium channels--basic aspects of their structure, function and gene encoding; anesthetic action on the channels--a review," Can. J. Anaesth., 49(2): 151-64 (2002).

R-type voltage dependent calcium channel inhibitors are calcium entry blocking drugs whose main pharmacological effect is to prevent or slow the entry of calcium into cells via R-type voltage-gated calcium channels. The gene $Ca_v2.3$ encodes the principal pore-forming unit of R-type voltage-dependent calcium channels being expressed in neurons.

## N-type Calcium Channels

N-type ('N' for "Neural-Type") calcium channels are found primarily at presynaptic terminals and are involved in neurotransmitter release. Strong depolarization by an action potential causes these channels to open and allow influx of $Ca^{2+}$, initiating vesicle fusion and release of stored neurotransmitter. N-type channels are blocked by $\omega$-conotoxin. Yamakage M. et al, "Calcium channels--basic aspects of their structure, function and gene encoding; anesthetic action on the channels--a review," Can. J. Anaesth., 49(2): 151-64 (2002).

## P/Q- type Calcium Channels

P-type ('P' for cerebellar Purkinje cells) calcium channels play a similar role to the N-type calcium channel in neurotransmitter release at the presynaptic terminal, and in neuronal integration in many neuronal types. They also are found in Purkinje fibers in the electrical conduction system of the heart (Winds, R., et al., J. Physiol. (Lond.) 305: 171-95 (1980); Llinds, R. et al., Proc. Natl. Acad. Sci. U.S.A. 86 (5): 1689-93 (1989)). Q-type calcium channel blockers appear to be present in cerebellar granule cells. They have a high threshold of activation and relatively slow kinetics. Yamakage M. et al, "Calcium channels-basic aspects of their structure, function and gene encoding; anesthetic action on the channels--a review," Can. J. Anaesth., 49(2): 151-64 (2002).

## T-type Calcium Channels

T-type ('T' for transient) calcium channel blockers are low voltage-activated. They most often are found in neurons and cells that have pacemaker activity and in osteocytes. Mibefradil shows some selectivity for T-type over other types of VDCC. Yamakage M. et al, "Calcium channels--basic aspects of their structure, function and gene encoding; anesthetic action on the channels--a review," Can. J. Anaesth., 49(2): 151-64 (2002).

## BLOCKERS AND INHIBITORS OF CALCIUM CHANNELS

Calcium channel blockers are a class of drugs and natural substances having effects on many excitable cells of the body, such as the muscle of the heart, smooth muscles of the vessels or neuron cells. The main action of calcium channel blockers is to decrease blood pressure.

Most calcium channel blockers decrease the force of contraction of the myocardium. This is known as the "negative inotropic effect" of calcium channel blockers. Most calcium channel blockers are not the preferred choice of treatment in individuals with cardiomyopathy due to their negative inotropic effects.

Many calcium channel blockers slow the conduction of electrical activity within the heart by blocking the calcium channel during the plateau phase of the action potential of the heart. This "negative dromotropic effect" causes a lowering of the heart rate and may cause heart blocks (which is known as the "negative chronotropic effect" of calcium channel blockers). The negative chronotropic effects of calcium channel blockers make them a commonly used class of agents for control of the heart rate in individuals with atrial fibrillation or flutter.

Calcium channel blockers act upon voltage-gated calcium channels (VGCCs) in muscle cells of the heart and blood vessels. By blocking the calcium channel they prevent large increases of the calcium levels in the cells when stimulated, which subsequently leads to less muscle contraction. In the heart, a decrease in calcium available for each beat results in a decrease in cardiac contractility. In blood vessels, a decrease in calcium results in less contraction of the vascular smooth muscle and therefore an increase in blood vessel diameter. The resultant vasodilation decreases total peripheral resistance, while a decrease in cardiac contractility decreases cardiac output. Since blood pressure is in part determined by cardiac output and peripheral resistance, blood pressure drops.

Calcium channel blockers do not decrease the responsiveness of the heart to input from the sympathetic nervous system. Since blood pressure regulation is carried out by the sympathetic nervous system (via the baroreceptor reflex), calcium channel blockers allow blood pressure to be maintained more effectively than do $\beta$-blockers. However, because calcium channel blockers result in a decrease in blood pressure, the baroreceptor reflex often initiates a reflexive increase in sympathetic activity leading to increased heart rate and contractility. The decrease in blood pressure also likely reflects a direct effect of antagonism of VDCC in vascular smooth muscle, leading to vasodilation. A $\beta$-blocker may be combined with a calcium channel blocker to minimize these effects.

The blockers for L, N, and P/Q-types of calcium channels are utilized in distinguishing channel subtypes. For the R-type calcium channel subtype, $\omega$-agatoxin IIIA shows blocking activity, even though its selectivity is rather low. This peptide binds to all of the high voltage-activated channels including L, N, and P/Q subtypes (J. Biol. Chem., 275, 21309 (2000)). A putative R-type (or class $\alpha$1E) selective blocker, SNX-482, a toxin from the tarantula *Hysterocrates*

*gigas,* is a 41 amino acid residue peptide with 3 disulfide linkages (1-4, 2-5 and 3-6 arrangement) (Biochemistry, 37, 15353 (1998), Peptides 1998, 748 (1999)). This peptide blocks the class E calcium channel (IC50=15 nM to 30 nM) and R-type calcium current in the neurohypophysial nerve endings at 40 nM concentration. R-type (class E) calcium channel blocking activity is highly selective; no effect is observed on $K^+$ and $Na^+$ currents, and L, P/Q and T-type calcium currents. N-type calcium current is blocked only weakly 30-50% at 300 nM to 500 nM. Regionally, different sensitivity of R-type current to SNX-482 is observed; no significant effect on R-type current occurs in preparations of the neuronal cell body, retinal ganglion cells and hippocampal pyramidal cells. Using SNX-482, three alpha E- calcium subunits with distinct pharmacological properties are recognized in cerebellar R-type calcium channels (J. Neurosci., 20, 171 (2000)). Similarly, it has been shown that secretion of oxytocin, but not vasopressin, is regulated by R-type calcium current in neurohypophysial terminals (J. Neurosci., 19, 9235 (1999)).

Dihydropyridine calcium channel blockers often are used to reduce systemic vascular resistance and arterial pressure, but are not used to treat angina (with the exception of amlodipine, which carries an indication to treat chronic stable angina as well as vasospastic angina) since the vasodilation and hypotension can lead to reflex tachycardia. This calcium channel blocker class is easily identified by the suffix "-dipine."

Phenylalkylamine calcium channel blockers are relatively selective for myocardium. They reduce myocardial oxygen demand and reverse coronary vasospasm. They have minimal vasodilatory effects compared with dihydropyridines. Their action is intracellular.

Benzothiazepine calcium channel blockers are an intermediate class between phenylalkylamine and dihydropyridines in their selectivity for vascular calcium channels. Benzothiazepines are able to reduce arterial pressure without producing the same degree of reflex cardiac stimulation caused by dihydropyridines due to their cardiac depressant and vasodilator actions.

L-type VDCC inhibitors are calcium entry blocking drugs whose main pharmacological effect is to prevent or slow entry of calcium into cells via L-type voltage-gated calcium channels. Examples of L-type calcium channel inhibitors include but are not limited to: dihydropyridine L-type blockers such as nisoldipine, nicardipine and nifedipine, AHF (such as 4aR,9aS)-(+)-4a-Amino-1,2,3,4,4a,9a-hexahydro-4a14-fluorene, HCl), isradipine (such as 4-(4- Benzofurazanyl)-1,-4-dihydro-2,6-dimethyl-3,5-pyridinedicarboxylic acid methyl 1- methhylethyl ester), calciseptine (such as isolated from (Dendroaspis polylepis ploylepis), H-Arg-Ile-Cys-Tyr-Ile-His-Lys-Ala-Ser-Leu-Pro-Arg-Ala-Thr-Lys-Thr-Cys-Val-Glu-Asn-Thr-Cys-Tyr-Lys-Met-Phe-Ile-Arg-Thr-Gln-Arg-Glu-Tyr-Ile-Ser-Glu-Arg-Gly-Cys-Gly-Cys-Pro-Thr-Ala-Met-Trp-Pro-Tyr-G1-n-Thr-Glu-Cys-Cys-Lys-Gly-Asp-Arg-Cys-Asn-Lys-OH, Calcicludine (such as isolated from Dendroaspis angusticeps (eastern green mamba)),(H-Trp-Gln-Pro-Pro-Trp-Tyr-Cys-Lys-Glu-Pro-Val-Arg-Ile-Gly-Ser-Cys-Lys-Lys-Gln-Phe-Ser-Ser-Phe-Tyr-Phe-Lys-Trp-Thr-Ala-Lys-Lys-Cys-Leu-Pro-Phe-Leu-Phe-Ser-Gly-Cys-Gly-Gly-Asn-Ala-Asn-Arg-Phe-Gln-Thr-Ile-Gly-Glu-Cys-Arg-Lys-Lys-Cys-Leu-Gly-Lys-OH, Cilnidipine (such as also FRP-8653, a dihydropyridine-type inhibitor), Dilantizem (such as (2S,3S)-(+)-cis-3-Acetoxy-5-(2-dimethylaminoethyl)-2,3-dihydro-2-(4-methoxyphenyl)-1,5-benzothiazepin-4(5H)-one hydrochloride), diltiazem (such as benzothiazepin-4(5H)-one, 3-(acetyloxy)-5-[2-(dimethylamino)ethyl]-2,3-dihydro-2-(4-methoxyphenyl)--,(+)- cis-, monohydrochloride), Felodipine (such as 4-(2,3-Dichlorophenyl)-1,4-dihydro-2,6-dimethyl- 3,5-pyridinecarboxylic acid ethyl methyl ester), FS-2 (such as an isolate from Dendroaspis polylepis polylepis venom), FTX-3.3 (such as an isolate from Agelenopsis aperta), Neomycin sulfate (such as $C_{23}H_{46}N_6O_{13} \cdot 3H2SO4$), Nicardipine (such as 1,4- Dihydro-2,6-dimethyl-4-(3-nitrophenypmethyl-2-[methyl(phenylmethypamino]-3,5- pyridinedicarboxylic acid ethyl ester hydrochloride, also YC-93, Nifedipine (such as 1,4- Dihydro-2,6-dimethyl-4-(2-nitrophenyl)-3,5-pyridinedicarboxylic acid dimethyl ester), Nimodipine (such as 4-Dihydro-2,6-dimethyl-4-(3-nitrophenyl)-3,5-pyridinedicarboxylic acid 2-methoxyethyl 1-methylethyl ester) or (Isopropyl 2-methoxyethyl 1,4-dihydro-2,6-dimethyl-4-(m-nitrophenyl)-3,5-pyridinedicarboxylate), Nitrendipine (such as 1,4-Dihydro-2,6-dimethyl-4-(3-nitrophenyl)-3,5-pyridinedicarboxylic acid ethyl methyl ester), S-Petasin (such as (3 S,4aR,5R,6R)-[2,3,4,4a,5,6,7,8-Octahydro-3-(2-propenyl)-4a,5-dimethyl-2-o-xo-6-naphthyl]Z-3'-methylthio-1'-propenoate), Phlorctin (such as 2',4',6'-Trihydroxy-3 -(4-hydroxyphenyl)propiophenone, also 3-(4-Hydroxyphenyl)-1-(2,4,6-trihydroxyphenyl)-1-propanone, also b-(4-Hydroxyphenyl)-2,4,6-trihydroxypropiophenone), Protopine (such as $C_{20}H_{19}NO_5Cl$), SKF-96365 (such as 1-[b-[3-(4-Methoxyphenyl)propoxy]-4-methoxyphenethyl]-1H-imidazole, HCl), Tetrandine (such as 6,6',7,12-Tetramethoxy-2,2'-dimethylberbaman), (.+-.)-Methoxyverapamil or (+)-Verapamil (such as 54N-(3,4-Dimethoxyphenylethyl)methylamino]-2-(3,4-dimethoxyphenyl)-2-iso- propylvaleronitrile hydrochloride), and (R)-(+)-Bay K8644 (such as R-(+)-1,4-Dihydro-2,6-dimethyl-5-nitro-442- (trifluoromethyl)phenyl]-3-py-ridinecarboxylic acid methyl ester). The foregoing examples may be specific to L-type voltage-gated calcium channels or may inhibit a broader range of voltage- gated calcium channels, e.g. N, P/Q, R, and T-type.

## ENDOTHELINS

Endothelins are vasoconstricting peptides produced primarily in the endothelium that increase blood pressure and vascular tone. This family of peptides includes endothelin-1 (ET-1), endothelin-2 (ET-2) and endothelin-3 (ET-3). These small peptides (21 amino acids) have an important role in vascular homeostasis. ET-1 is secreted mostly by vascular endothelial cells. The predominant ET-1 isoform is expressed in vasculature and is the most potent vasoconstrictor. ET-1 also has inotropic, chemotactic and mitogenic properties. It stimulates the sympathetic nervous system, and influences salt and water homeostasis through its effects on the renin-angiotensin-aldosterone system (RAAS), vasopressin and atrial natriuretic peptide. Endothelins are among the strongest vasoconstrictors known and have been implicated in vascular diseases of several organ systems, including the heart, general circulation and brain.

There are two key endothelin receptor types, ETA and ETB. ETA and ETB have distinct pharmacological characteristics. The ETA-receptor affinity is much higher for ET-1 than for ET-3. ETA-receptors are located in the vascular smooth muscle cells, but not in endothelial cells. The binding of endothelin to ETA increases vasoconstriction and the retention of sodium, leading to increased blood pressure. ETB receptors primarily are located on the endothelial cells that line the interior of the blood vessels. Endothelin binding to ETB receptors lowers blood pressure by increasing natriuresis and diuresis, and releasing nitric oxide. ET-1 and ET-3 activate the ETB-receptor equally, which in turn leads to vasodilation via production of NO and prostaglandins. Endothelin-1 (ET-1) also has been demonstrated to cause vascular smooth muscle constriction via ETA-receptor stimulation and to induce NO production in endothelial cells via ETB-receptors. Some ETB-receptors are located in vascular smooth muscle, where they may mediate vasoconstriction. A number of endothelin receptors are regulated by various factors. Angiotensin II and phorbol esters down-regulate endothelin receptors whereas ischemia and cyclosporin increase the number of endothelin receptors.

A number of peptide and nonpeptide ET antagonists have been studied. ETA-receptor antagonists may include, but are not limited to, A-127722 (non-peptide), ABT-627 (non-peptide), BMS 182874 (non-peptide), BQ-123 (peptide), BQ-153 (peptide), BQ-162 (peptide), BQ-485 (peptide), BQ-518 (peptide), BQ-610 (peptide), EMD-122946 (non-peptide), FR 139317 (peptide), IPI-725 (peptide), L-744453 (non-peptide), LU 127043 (non-peptide), LU 135252 (non-peptide), PABSA (non-peptide), PD 147953 (peptide), PD 151242 (peptide), PD 155080 (non-peptide), PD 156707 (non-peptide), RO 611790 (non-peptide), SB-247083 (non-peptide), clazosentan (non-peptide), atrasentan (non-peptide), sitaxsentan sodium (non-peptide), TA-0201 (non-peptide), TBC 11251 (non-peptide), TTA-386 (peptide), WS-7338B (peptide), ZD-1611 (non-peptide), and aspirin (non-peptide). ETA/B-receptor antagonists may include, but are not limited to, A-182086 (non-peptide), CGS 27830 (non-peptide), CP 170687 (non-peptide), J-104132 (non-peptide), L-751281 (non-peptide), L-754142 (non-peptide), LU 224332 (non- peptide), LU 302872 (non-peptide), PD 142893 (peptide), PD 145065 (peptide), PD 160672 (non-peptide), RO-470203 (bosentan, non-peptide), RO 462005 (non-peptide), RO 470203 (non- peptide), SB 209670 (non-peptide), SB 217242 (non-peptide), and TAK-044 (peptide). ETB¬receptor antagonists may include, but are not limited to, A-192621 (non-peptide), A-308165 (non-peptide), BQ-788 (peptide), BQ-017 (peptide), IRL 1038 (peptide), IRL 2500 (peptide), PD-161721 (non-peptide), RES 701-1 (peptide), and RO 468443 (peptide).

ET-1 is translated initially to a 212 amino-acid peptide (pre-proendothelin-1). It is further converted to proendothelin-1 after removal of the secretory sequence. Proendothelin-1 then is cleaved by furin to generate the biologically-inactive precursor big endothelin-1. Mature ET-1 is formed upon cleavage of big endothelin-1 by one of several endothelin-converting enzymes (ECEs). There are two splice variants of ECE-1; these are ECE-la and ECE-lb. Each has functionally distinct roles and tissue distribution. ECE-la is expressed in the Golgi network of endothelin-producing cells and cleaves big endothelin-1 to form ET-1. ECE-lb is localized at the plasma membrane and cleaves extracellular big endothelin-1. Both ECE-la and ECE-lb are inhibited by metalloprotease inhibitor phosphoramidon. ECEs also are located on $\alpha$-actin filaments in smooth muscle cells. ECE inhibition by phosphoramidon completely blocks vasoconstriction to big endothelin-1. ECE inhibitors may include, but are not limited to, B-90063 (non-peptide), CGS 26393 (non-peptide), CGS 26303 (non-peptide), CGS 35066 (non peptide), phosphoramidon (peptide), PP-36 (peptide), SM-19712 (non-peptide), and TMC-66 (non-peptide).

In a healthy individual, a delicate balance between vasoconstriction and vasodilation is maintained by endothelin and other vasoconstrictors on the one hand and nitric oxide, prostacyclin and other vasodilators on the other. Endothelin antagonists may have a role in the treatment of cardiac, vascular and renal diseases associated with regional or systemic vasoconstriction and cell proliferation, such as essential hypertension, pulmonary hypertension, chronic heart failure and chronic renal failure.

## TRANSIENT RECEPTOR POTENTIAL CHANNELS

The transient receptor potential (TRP) channel family is a member of the calcium channel group. These channels include transient receptor potential protein and homologues thereof, the vanilloid receptor subtype I, stretch-inhibitable non-selective cation channel, olfactory, mechanosensitive channel, insulin-like growth factor I-regulated calcium channel, and vitamin D-responsive apical, epithelial calcium channel (ECaC). Each of these molecules is at least 700 amino acids in length, and shares certain conserved structural features. Predominant among these structural features are six transmembrane domains, with an additional hydrophobic loop present between the fifth and sixth transmembrane domains. It is believed that this loop is integral to the activity of the pore of the channel formed upon membrane insertion. TRP channel proteins also include one or more ankyrin domains and frequently display a proline-rich region at the N-terrminus.

Transient receptor potential (TRP) cation channels are present in vascular smooth muscle and are involved in the smooth muscle depolarizing response to stimuli such as membrane stretch. Uridine triphosphate (UTP) invokes membrane depolarization and constriction of vascular smooth muscle by activating a cation current that exhibits inward rectification, is not rapidly desensitized, and is blocked by $Gd3^+$. Canonical transient receptor potential (TRPC) proteins form $Ca^{2+}$ permeable, non-selective cation channels in a variety of mammalian tissues. Suppression of one member of this family of channels, TRPC6, has been reported to prevent an alpha-adenoreceptor-activated cation current in cultured rabbit portal vein myocytes. However, suppression of TRPC6 channels in cerebral vascular smooth muscle does not attentuate the UTP-induced membrane depolarization and vasoconstriction. In contrast, TRPC3, unlike TRPC6, has been found to mediate the agonist induced depolarization, as observed in rat cerebral artery, following UTP activation of the P2Y receptor. Thus, TRPC3 channels in vascular smooth muscle mediate agonist-induced depolarization which contributes to vasoconstriction in resistance-sized cerebral arteries.

The TRP1 channel family comprises a large group of channels mediating an array of signal and sensory transduction pathways. The proteins of the mammalian TRPC subfamily are the products of at least seven genes coding for cation channels that appear to be activated in response to phospholipase C (PLC)-coupled receptors. The putative ion channel subunits TRPC3, TRPC6, and TRPC7 comprise a structurally related subgroup of the family of mammalian TRPC channels. The ion channels formed by these proteins appear to be activated downstream of phospholipase C (PLC). PLC-dependent activation of TRPC6 and TRPC7 has been shown to involve diacylglycerol and is independent of G proteins or inositol 1,4,5-triphosphate (IP3).

TRPC channels are widely expressed among cell types and may play important roles in receptor-mediated $Ca^{2-}$ signaling. The TRPC3 channel is known to be a $Ca^{2+}$-conducting channel activated in response to PLC-coupled receptors. TRPC3 channels have been shown to interact directly with intracellular inositol 1,4,5-trisphosphate receptors (InsP3Rs), i.e., channel activation is mediated through coupling to InsP3Rs.

Agents useful for increasing arterial blood flow, inhibiting vasoconstriction or inducing vasodilation are agents that inhibit TRP channels. These inhibitors embrace compounds that are TRP channel antagonists. Such inhibitors are referred to as activity inhibitors or TRP channel activity inhibitors. As used herein, the term "activity inhibitor" refers to an agent that interferes with or prevents the activity of a TRP channel. An activity inhibitor may interfere with the ability of the TRP channel to bind an agonist such as UTP. An activity inhibitor may be an agent that competes with a naturally occurring activator of TRP channel for interaction with the activation binding site on the TRP channel Alternatively, an activity inhibitor may bind to the TRP channel at a site distinct from the activation binding site, but in doing so, it may, for example, cause a conformational change in the TRP channel, which is transduced to the activation binding site, thereby precluding binding of the natural activator. Alternatively, an activity inhibitor may interfere with a component upstream or downstream of the TRP channel but which interferes with the activity of the TRP channel. This latter type of activity inhibitor is referred to as a functional antagonist. Non-limiting examples of a TRP channel inhibitor that is an activity inhibitor are gadolinium chloride, lanthanum chloride, SKF 96365 and LOE-908.

Current treatments to prevent or reduce cerebral vasospasm consist of measures to prevent or minimize secondary brain injury, use calcium channel blockers, hemodynamic management and endovascular therapies. Therapy often is initiated prophylactically in patients and may include: (in stage 1) hemodynamic stabilization including maintaining normovolemia, managing blood pressure, and orally-administered L-type voltage-gated calcium channel antagonists; and (in stage 2) further hemodynamic manipulation or infusion of vasodilator drugs into vasospastic arteries or dilating them with balloons. However, the aforementioned treatments are expensive, time consuming and only partially effective.

For over 35 years, physicians have been trying to prevent or reduce the incidence of adverse consequences of SAH, including vasospasm, and have had limited effect due to side effects of current agents or lack of efficacy. There currently are no FDA approved agents for the prevention of vasospasm or the reduction of delayed ischemic neurologic deficits also known as delayed cerebral ischemia (DCI). Current methods to prevent vasospasm have failed due to lack of efficacy or to safety issues, primarily hypotension and cerebral edema. Currently, the only FDA-approved available agent is nimodipinc, which docs not reduce vasospasm, although it improved outcome in SAH patients.

Voltage-gated calcium channel antagonists may be effective in preventing and reversing vasospasm to a certain extent, however, prior art treatments administer doses too low to exert a maximal pharmacologic effect. Endothelin-receptor antagonists also may be effective at preventing and reversing vasospasm to a certain extent, but this reversal or prevention of vasospasm does not translate into as marked an improvement in outcome as would be anticipated by the reduction in vasospasm. Without being limited by theory, it is postulated that the systemic delivery of the voltage-gated calcium channel blockers may cause side effects that mitigate the beneficial effects on vasospasm, such as, for example, systemic hypotension and pulmonary vasodilation with pulmonary edema, which prevent the administration of higher systemic doses. Dilation of blood vessels in the lungs also may cause lung edema and lung injury.

While conventional therapies have been focusing on treating cerebral vasospasms following subarachnoid hemorrhage, accumulating evidence suggests that there are additional complications derived from subarachnoid hemorrhage, which need to be targeted for treatment interventions in order to improve prognosis following subarachnoid hemorrhage treatment. The described invention offers such an approach.

**SUMMARY**

According to one aspect, the described invention provides a method for treating a delayed complication associated with a brain injury in a mammal in need thereof, wherein the brain injury includes interruption of at least one cerebral artery, the method comprising: (a) providing a pharmaceutical composition comprising (i) a microparticulate formulation of a voltage-gated calcium channel blocker; and optionally (ii) a pharmaceutically acceptable carrier; and (b) administering a therapeutic amount of the pharmaceutical composition by a means for administration of the therapeutic amount of the pharmaceutical composition at a site of administration, wherein the therapeutic amount is effective in reducing signs or symptoms of the at least one delayed complication associated with the brain injury, and wherein the at least one delayed complication is at least one of delayed cerebral ischemia (DCI), a plurality of microthromboemboli a cortical spreading ischemia (CSI), and an angiographic vasospasm.

According to one embodiment of the method, in step (b), the means for administration is a surgical injection apparatus and the site of administration is in close proximity to at least one cerebral artery affected by the brain injury. According to another embodiment, in step (b), the means for administration is a surgical injection apparatus and the site of administration is a cerebral ventricle. According to another embodiment, the surgical injection apparatus is a needle, a cannula, a catheter, or a combination thereof. According to another embodiment, According to another embodiment, the microparticulate formulation is carried by cerebrospinal fluid to contact the cerebral artery affected by the brain injury. According to another embodiment, the therapeutic amount is effective to increase the internal diameter of the cerebral artery affected by the brain injury, as compared to a control. According to another embodiment, the brain injury is a result of an aneurysm, sudden traumatic head injury, subarachnoid hemorrhage (SAH), or a combination thereof. According to another embodiment, the brain injury is a result of subarachnoid hemorrhage. According to another embodiment, the at least one delayed complication associated with the brain injury further comprises at least one of an intracerebral hematoma, an intraventricular hemorrhage, a fever, a behavioral deficit, a neurological deficit, a cerebral infarction, and neuronal cell death. According to another embodiment, the behavioral deficit is improved such that the improved behavioral deficit comprises an increase in appetite. According to another embodiment, the neurological deficit is improved such that the improved neurological deficit comprises an improvement of ataxia or paresis. According to another embodiment, the pharmaceutical composition exerts a predominantly localized pharmacologic effect in treating the at least one delayed complication associated with brain injury. According to another embodiment, the pharmaceutical composition exerts a diffuse pharmacologic effect throughout the brain in treating the at least one delayed complication associated with brain injury. According to another embodiment, the microparticulate formulation comprises a plurality of microparticles, wherein the microparticles are of uniform size distribution, and wherein each microparticle comprises a matrix. According to another embodiment, the microparticulate formulation further comprises a plurality of microparticles impregnated with the voltage-gated calcium channel blocker. According to another embodiment, the microparticle formulation comprises a liquid suspension of microparticles. According to another embodiment, the cerebral ventricle is a lateral ventricle, a third ventricle, a fourth ventricle, or a combination thereof. According to another embodiment, the microparticulate formulation of the voltage-gated calcium channel blocker comprises a slow-release compound. According to another embodiment, the slow release compound is a polymer. According to another embodiment, the voltage-gated calcium channel blocker is disposed on or in the slow-release polymer. According to another embodiment, the microparticulate formulation of the voltage-gated calcium channel blocker comprises poly (D, L-lactide-co-glycolide). According to another embodiment, the microparticulate formulation of a voltage-gated calcium channel blocker comprises poly(orthoester). According to another embodiment, the microparticulate formulation of the voltage-gated calcium channel blocker comprises polyanhydride. According to another embodiment, the voltage-gated calcium channel blocker is selected from the group consisting of an L-type voltage-gated calcium channel blocker, an N-type voltage-gated calcium channel blocker, a P/Q-type voltage-gated calcium channel blocker, or a combination thereof. According to another embodiment, the voltage-gated calcium channel blocker is a dihydropyridine calcium channel blocker. According to another embodiment, the dihydropyridine calcium channel blocker is nimodipine. According to another embodiment, the pharmaceutically acceptable carrier comprises a gel compound. According to another embodiment, the gel compound is a biodegradable hydrogel. According to another embodiment, the pharmaceutical composition does not comprise the pharmaceutically acceptable carrier. According to another embodiment, the pharmaceutically acceptable carrier does not comprise hyaluronic acid. According to another embodiment, the pharmaceutically acceptable carrier comprises hyaluronic acid. According to another embodiment, the pharmaceutically acceptable carrier ranges between 0 % to 5 % hyaluronic acid. According to another embodiment, the pharmaceutically acceptable carrier comprises less than 2.3 % hyaluronic acid. According to another embodiment, the pharmaceutically acceptable carrier comprises less than 5 % hyaluronic acid.

According to another aspect, the described invention provides a semisolid multiparticulate delivery system for treating a delayed complication associated with a brain injury in a mammal in need thereof, wherein the brain injury includes interruption of at least one cerebral artery, the system comprising: (a) a pharmaceutical composition comprising (i) a microparticulatc formulation of a voltage-gated calcium channel blocker; and optionally (ii) a pharmaceutically acceptable carrier; and (b) a means for administering a therapeutic amount of the pharmaceutical composition at a site for administration , wherein the therapeutic amount is effective in reducing signs or symptoms of the at least one delayed complication associated with the brain injury.

According to one embodiment, the at least one delayed complication is at least one of delayed cerebral ischemia (DCI), a plurality of microthromboemboli a cortical spreading ischemia (CSI), and an angiographic vasospasm. The system according to claim 31, wherein the means for administration is a surgical injection apparatus and the site of administration is in close proximity to at least one cerebral artery affected by the brain injury. According to another embodiment, the means for administration is a surgical injection apparatus and the site of administration is a cerebral ventricle. According to another embodiment, the surgical injection apparatus is a needle, a cannula, a catheter, or a combination thereof. According to another embodiment, the microparticulate formulation is capable of being carried by cerebrospinal fluid to contact the cerebral artery affected by the brain injury. According to another embodiment,= therapeutic amount is effective to increase the internal diameter of the cerebral artery affected by the brain injury,as compared to a control. According to another embodiment, the brain injury is a result of an aneurysm, sudden traumatic head injury, subarachnoid hemorrhage (SAH), or a combination thereof. According to another embodiment, the brain injury is a result of subarachnoid hemorrhage. According to another embodiment, the at least one delayed complication associated with the brain injury further comprises at least one of an intracerebral hematoma, an intraventricular hemorrhage, a fever, , a behavioral deficit, a neurological deficit, a cerebral infarction, and neuronal cell death. According to another embodiment, the behavioral deficit is improved such that the improved behavioral deficit comprises an increase in appetite. According to another embodiment, the neurological deficit is improved such that the improved neurological deficit comprises an improvement of ataxia or paresis. According to another embodiment, the pharmaceutical composition exerts a predominantly localized pharmacologic effect in treating the at least one delayed complication associated with brain injury. According to another embodiment, the pharmaceutical composition exerts a diffuse pharmacologic effect throughout the brain in treating the at least one delayed complication associated with brain injury. According to another embodiment, the microparticulate formulation comprises a plurality of microparticles, wherein the microparticles are of uniform size distribution, and wherein each microparticle comprises a matrix. According to another embodiment, the microparticulate formulation further comprises a plurality of microparticles impregnated with the voltage-gated calcium channel blocker. According to another embodiment, the microparticle formulation comprises a liquid suspension of microparticles. According to another embodiment, the cerebral ventricle is a lateral ventricle, a third ventricle, a fourth ventricle, or a combination thereof. According to another embodiment, the microparticulate formulation of the voltage-gated calcium channel blocker comprises a slow-release compound. According to another embodiment, the slow release compound is a polymer. According to another embodiment, the voltage-gated calcium channel blocker is disposed on or in the slow-release polymer. According to another embodiment, the microparticulate formulation of a voltage-gated calcium channel blocker comprises poly (D, L-lactide-co-glycolide). According to another embodiment, the microparticulate formulation of a voltage-gated calcium channel blocker comprises poly(orthoester). According to another embodiment, the microparticulate formulation of a voltage-gated calcium channel blocker comprises polyanhydride. According to another embodiment, the voltage-gated calcium channel blocker is selected from the group consisting of an L-type voltage-gated calcium channel blocker, an N-type voltage-gated calcium channel blocker, a P/Q-type voltage-gated calcium channel blocker, or a combination thereof. According to another embodiment, the voltage-gated calcium channel blocker is a dihydropyridine calcium channel blocker. According to another embodiment, the dihydropyridine calcium channel blocker is nimodipine. According to another embodiment, the pharmaceutically acceptable carrier comprises a gel compound. According to another embodiment, the gel compound is a biodegradable hydrogel. According to another embodiment, the pharmaceutical composition does not comprise the pharmaceutically acceptable carrier. According to another embodiment, the pharmaceutically acceptable carrier does not comprise hyaluronic acid. According to another embodiment, the pharmaceutically acceptable carrier is hyaluronic acid. According to another embodiment, the pharmaceutically acceptable carrier ranges between 0 % to 5 % hyaluronic acid. According to another embodiment, the pharmaceutically acceptable carrier comprises less than 2.3 % hyaluronic acid. According to another embodiment, the pharmaceutically acceptable carrier comprises less than 5 % hyaluronic acid.

**BRIEF DESCRIPTION OF THE DRAWINGS**

**Figure 1** shows an illustrative view of the cerebral arteries.

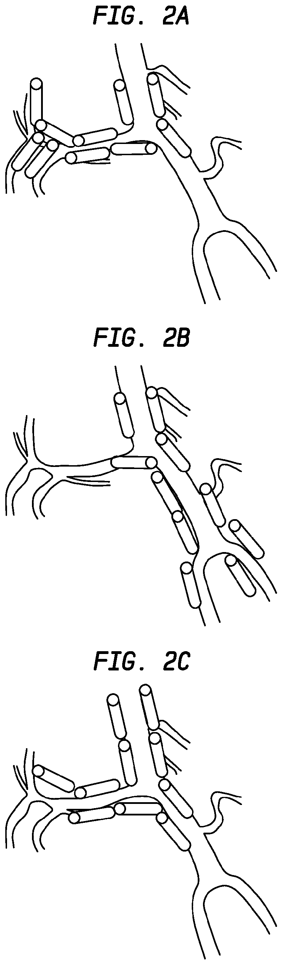

**Figure 2A** shows an exemplary view of the application of a calcium channel blocker, endothelin receptor antagonist, or putative transient receptor potential protein blocker gel, slow-release solid or semisolid compound to the anterior communicating artery according to one embodiment of the present invention.

**Figure 2B** shows a view of one embodiment of the application of calcium channel blocker, endothelin receptor antagonist, or putative transient receptor potential protein blocker gel, slow-release solid or semisolid compound to the middle cerebral artery.

**Figure 2C** shows a view of one embodiment of the application of calcium channel blocker, endothelin antagonist, or putative transient receptor potential protein blocker gel, slow-release solid or semisolid compound to the internal carotid artery.

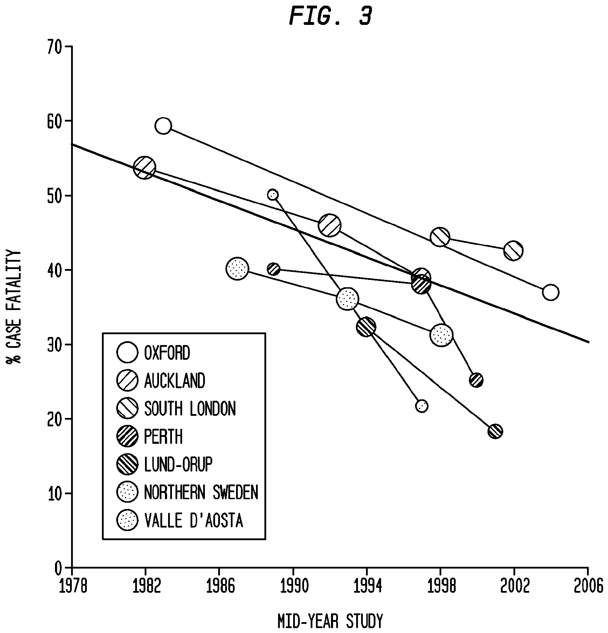

**Figure 3** shows time trends in outcome of subarachnoid hemorrhage in seven population-based studies of subarachnoid hemorrhage (SAH), which shows 50% decrease in mortality over 20 years.

**Figure 4A** shows a simple flow diagram for prognosis following subarachnoid hemorrhage.

**Figure 4B** shows a flow diagram of pathways proposed to be involved in delayed complications after subarachnoid hemorrhage.

**Figure 5** shows an illustrative view of the cerebral arteries. (from Netter FH. The CIBA Collection of Medical Illustrations: Volumes 1, Nervous System. Vol. 1. Part I. CIBA: USA. 1986. pp. 256).

**Figure 6** shows an illustrative view of the cerebral ventricles (page 192, Ross LM, Lamperti ED, Taub E (eds), Schuenke M, Schulte E, Schumacher U. Thieme Atlas of Anatomy. Georg Thieme Verlag: Stuttgart. 2006. pp. 541).

**Figure 7** shows an illustrative view of the CSF flow from the ventricles to the subarachnoid space (page 194, Ross LM, Lamperti ED, Taub E (eds), Schuenke M, Schulte E, Schumacher U. Thieme Atlas of Anatomy. Georg Thieme Verlag: Stuttgart. 2006. pp. 541).

**Figure 8** shows an exemplary view of the application of calcium channel blocker, endothelin receptor antagonist, or putative TRP protein blocker microparticle suspension to the cerebral ventricles through an intraventricular catheter (Figure from Mccomb JG: Techniques of CSF diversion. In: Scott RM (ed). Hydrocephalus. Vol. 3. Williams & Wilkins: Baltimore. 1990. page 48, pp. 128).

**Figure 9** shows a schematic depicting application of a composition comprising at least one of a calcium channel blocker, endothelin receptor antagonist, or putative TRP protein blocker in or on microparticles being carried by CSF flow to each of the arteries of the subarachnoid space (Pollay M: Cerebrospinal fluid. In: Tindall GT, Cooper PR, Barrow DL (eds). The Practice of Neurosurgery. Vol. 1. Williams & Wilkins: Baltimore. 1996. page 36, pp. 1381).

**Figure 10** is a bar graph showing percent (%) changes in mean basilar arterial diameters from baseline following local treatment by intracisternal administration with a low dose (10 mg) microparticulate nimodipine formulation plus saline vehicle ("low dose"), a high dose (30 mg) microparticulate nimodipine formulation plus saline vehicle ("high dose"), and a microparticulate placebo formulation plus saline vehicle ("placebo") in Study 1.

**Figure 11** shows a plot of behavioral scores over time (days) obtained with dogs subjected to subarachnoid hemorrhage, which are treated with a microparticulate placebo formulation plus saline vehicle ("placebo"), a low dose (10 mg) microparticulate nimodipine formulation plus saline vehicle ("low dose"), or a high dose (30 mg) microparticulate nimodipine formulation plus saline vehicle ("high dose") in Study 1.

**Figure 12** shows a plot of serum drug concentrations (ng/mL) over time (days) in dogs subjected to subarachnoid hemorrhage, which are treated with a microparticulate placebo formulation plus saline vehicle ("placebo"), a low dose (10 mg) microparticulate nimodipine formulation plus saline vehicle ("low dose") or a high dose (30 mg) of microparticulate nimodipine formulation plus saline vehicle ("high dose") in Study 1. Values are means +/- standard deviations (n=2 per group).

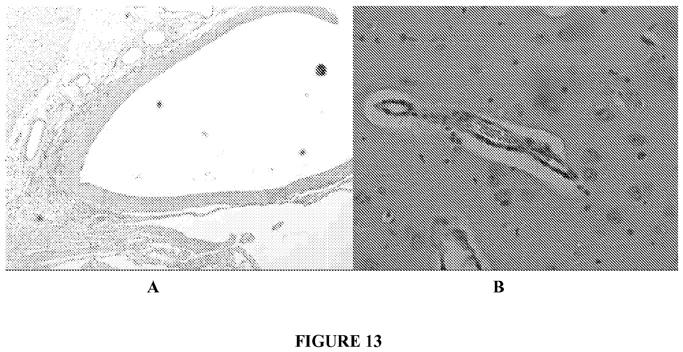

**Figure 13** shows histopathology of a dog subjected to subarachnoid hemorrhage, which is treated with a microparticulate placebo formulation plus saline vehicle (A) or with low dose (10 mg) of microparticulate nimodipine formulation plus saline vehicle (B). The subarachnoid space of a dog treated with placebo microparticles exhibited mild granulomatous inflammation (A). Microthromboemboli were observed in small vessels of dogs with subarachnoid hemorrhage treated with placebo, low or high dose nimodipine microparticles. There were qualitatively more microthromboemboli in the brains of placebo-treated dogs. The figure (B) shows an example of microthromboemboli in the small vessels of a dog with subarachnoid hemorrhage treated with low dose of nimodipine microparticles.

**Figure 14** shows sectional planes used in the canine model experiments.

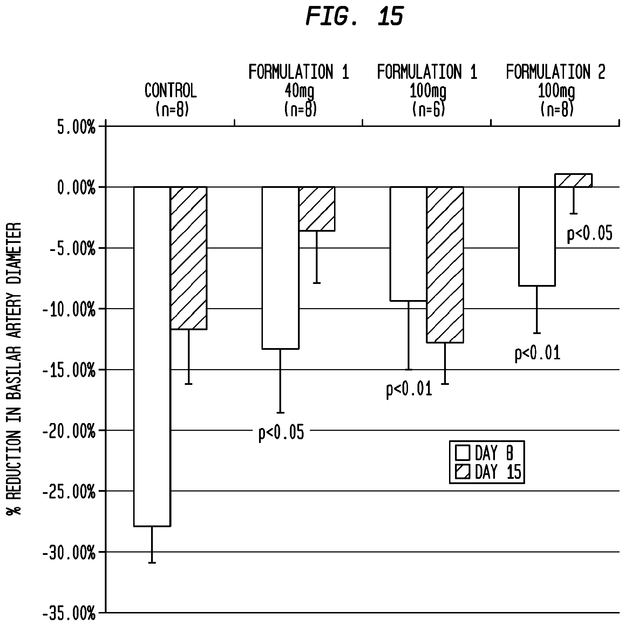

**Figure 15** is a bar graph showing percent (%) change in mean basilar arterial diameters from baseline following treatment with microparticulate nimodipine formulation plus hyaluronic acid (HA) vehicle ("Formulation 1") at dose 1 (40 mg) by intracisternal administration; microparticulate nimodipine formulation plus hyaluronic acid (HA) vehicle ("Formulation 1") at dose 2 (100 mg) by intracisternal administration; microparticulate nimodipine formulation without hyaluronic acid (HA) ("Formulation 2") at dose 2 (100 mg) by intraventricular administration; and a control (microparticulate placebo formulation plus hyaluronic acid (HA) vehicle by intracisternal administration followed by oral nimodipine) in Study 2.

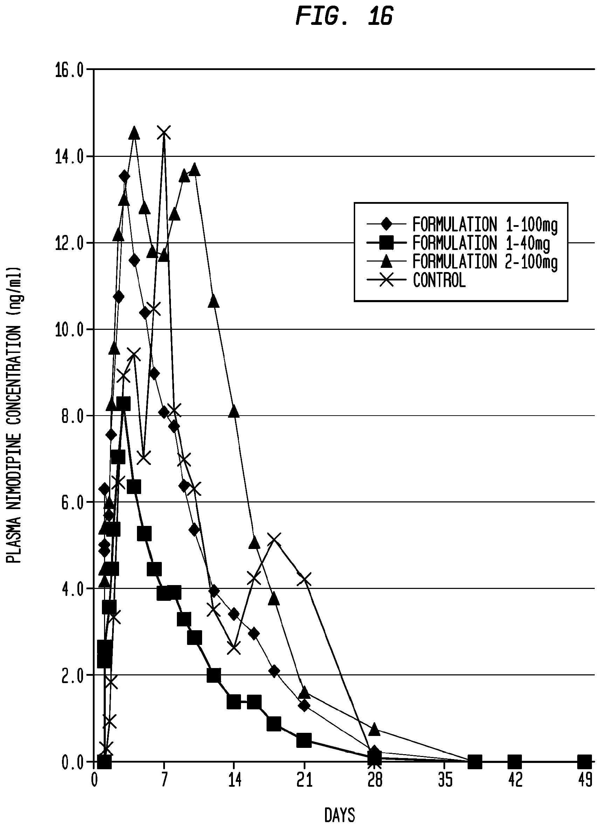

**Figure 16** shows a plot of serum drug concentrations (ng/mL) over time (days) in dogs subjected to subarachnoid hemorrhage, which are treated with a microparticulate nimodipine formulation plus hyaluronic acid (HA) vehicle ("Formulation 1") at dose 1 (40 mg) by intracisternal administration; microparticulate nimodipine formulation plus hyaluronic acid (HA) vehicle ("Formulation 1") at dose 2 (100 mg) by intracisternal administration; microparticulate nimodipine formulation without hyaluronic acid (HA) ("Formulation 2") at dose 2 (100 mg) by intraventricular administration; and a control (microparticulate placebo formulation plus hyaluronic acid (HA) vehicle by intracisternal administration followed by oral nimodipine) in Study 2.

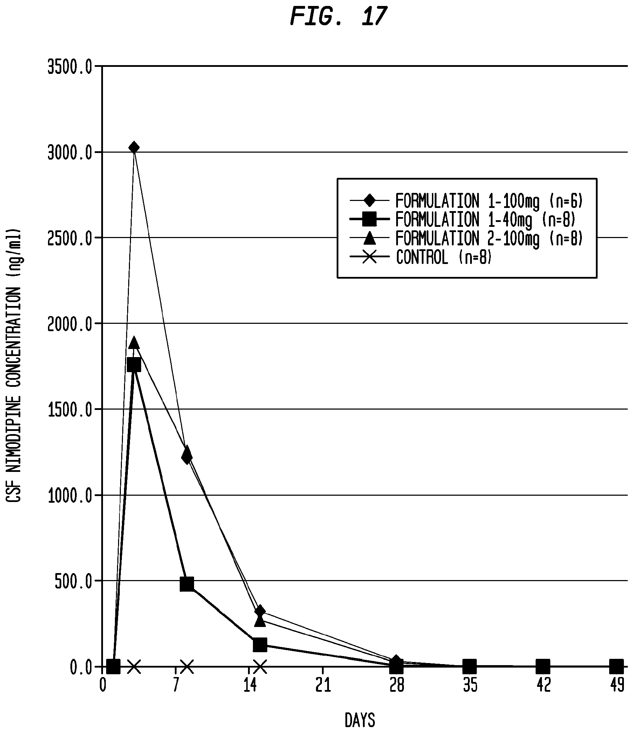

**Figure 17** shows a plot of cerebrospinal fluid (CSF) nimodipine concentrations (ng/mL) over time (days) in dogs subjected to subarachnoid hemorrhage treated with a microparticulate nimodipine formulation plus hyaluronic acid (HA) vehicle ("Formulation 1") at dose 1 (40 mg) by intracisternal administration; microparticulate nimodipine formulation plus hyaluronic acid (HA) vehicle ("Formulation 1") at dose 2 (100 mg) by intracisternal administration; microparticulate nimodipine formulation without hyaluronic acid (HA) ("Formulation 2") at dose 2 (100 mg) by intraventricular administration; and a control (microparticulate placebo formulation plus hyaluronic acid (HA) vehicle by intracisternal administration followed by oral nimodipine) in Study 2.

## DETAILED DESCRIPTION OF THE INVENTION

### GLOSSARY

The term "active" as used herein refers to the ingredient, component or constituent of the compositions of the present invention responsible for the intended therapeutic effect.

The term "antagonist" as used herein refers to a substance that counteracts the effects of another substance.

The term "administering" as used herein includes in vivo administration, as well as administration directly to tissue ex vivo. Generally, compositions may be administered systemically either orally, buccally, parenterally, topically, by inhalation or insufflation (i.e., through the mouth or through the nose), or rectally in dosage unit formulations containing conventional nontoxic pharmaceutically acceptable carriers, adjuvants, and vehicles as desired, or may be locally administered by means such as, but not limited to, injection, implantation, grafting, topical application, or parenterally.

The term "agonist" as used herein refers to a chemical substance capable of activating a receptor to induce a full or partial pharmacological response. Receptors can be activated or inactivated by either endogenous or exogenous agonists and antagonists, resulting in stimulating or inhibiting a biological response. A physiological agonist is a substance that creates the same bodily responses, but does not bind to the same receptor. An endogenous agonist for a particular receptor is a compound naturally produced by the body which binds to and activates that receptor. A superagonist is a compound that is capable of producing a greater maximal response than the endogenous agonist for the target receptor, and thus an efficiency greater than 100%. This does not necessarily mean that it is more potent than the endogenous agonist, but is rather a comparison of the maximum possible response that can be produced inside a cell following receptor binding. Full agonists bind and activate a receptor, displaying full efficacy at that receptor. Partial agonists also bind and activate a given receptor, but have only partial efficacy at the receptor relative to a full agonist. An inverse agonist is an agent which binds to the same receptor binding-site as an agonist for that receptor and reverses constitutive activity of receptors. Inverse agonists exert the opposite pharmacological effect of a receptor agonist. An irreversible agonist is a type of agonist that binds permanently to a receptor in such a manner that the receptor is permanently activated. It is distinct from a mere agonist in that the association of an agonist to a receptor is reversible, whereas the binding of an irreversible agonist to a receptor is believed to be irreversible. This causes the compound to produce a brief burst of agonist activity, followed by desensitization and internalization of the receptor, which with long-term treatment produces an effect more like an antagonist. A selective agonist is specific for one certain type of receptor.

The terms "anastomosis" and "anastomoses" as are used interchangeably to refer to interconnections between blood vessels. These interconnections protect the brain when part of its vascular supply is compromised. At the circle of Willis, the two anterior cerebral arteries are connected by the anterior communicating artery and the posterior cerebral arteries are connected to the internal carotid arteries by the posterior communicating arteries. Other important anastomoses include connections between the ophthalmic artery and branches of the external carotid artery through the orbit, and connections at the brain surface between branches of the middle, anterior, and posterior cerebral arteries (Principles of Neural Sciences, 2d Ed., Eric R. Kandel and James H. Schwartz, Elsevier Science Publishing Co., Inc., New York, pp. 854-56 (1985)).

The term "angiographic vasospasm" as used herein refers to the reduction of vessel size that can be detected on angiographic exams, occurring in approximately 50% of patients following subarachnoid hemorrhage. On the other hand, the term "clinical vasospasm" as used herein refers to the syndrome of confusion and decreased level of consciousness associated with reduced blood flow to the brain parenchyma, occurring in approximatedly 30% of patients.

The term "antagonist" as used herein refers to a substance that counteracts the effects of another substance.

The term "ataxia" as used herein refers to an inability to coordinate muscle activity during voluntary movement.

The term "biocompatible" as used herein refers to causing no clinically relevant tissue irritation, injury, toxic reaction, or immunological reaction to living tissue.

The term "biodegradable" as used herein refers to material that will break down actively or passively over time by simple chemical processes, by action of body enzymes or by other similar biological activity mechanisms.

The term "blocker" as used herein refers to a substance that inhibits the physiological action of another substance.

The term "carrier" as used herein describes a material that does not cause significant irritation to an organism and does not abrogate the biological activity and properties of the active compound of the composition of the described invention. Carriers must be of sufficiently high purity and of sufficiently low toxicity to render them suitable for administration to the mammal being treated. The carrier can be inert, or it can possess pharmaceutical benefits, cosmetic benefits or both. The terms "excipient", "carrier", or "vehicle" are used interchangeably to refer to carrier materials suitable for formulation and administration of pharmaceutically acceptable compositions described herein. Carriers and vehicles useful herein include any such materials know in the art which are nontoxic and do not interact with other components.

As shown in FIG. 1, the term "cerebral arteries" refers to at least one of the anterior communication artery, middle cerebral artery, internal carotid artery, anterior cerebral artery, ophthalmic artery, anterior choroidal artery, posterior communicating artery, and basilar artery, and vertebral artery among others.

The term "cerebral vasospasm" as used herein refers to the delayed occurrence of narrowing of large capacitance arteries at the base of the brain after subarachnoid hemorrhage, often associated with diminished perfusion in the territory distal to the affected vessel. Cerebral vasospasm may occur any time after rupture of an aneurysm but most commonly peaks at seven days following the hemorrhage and often resolves within 14 days when the blood has been absorbed by the body.

The phrase "in close proximity" as used herein refers to in the subarachnoid space within less than 10 mm, less than 9.9 mm, less than 9.8 mm, less than 9.7 mm, less than 9.6 mm, less than 9.5 mm, less than 9.4 mm, less than 9.3 mm, less than 9.2 mm, less than 9.1 mm, less than 9.0 mm, less than 8.9 mm, less than 8.8 mm, less than 8.7 mm, less than 8.6 mm, less than 8.5 mm, less than 8.4 mm, less than 8.3 mm, less than 8.2 mm, less than 8.1 mm, less than 8.0 mm, less than 7.9 mm, less than 7.8 mm, less than 7.7 mm, less than 7.6 mm, less than 7.5 mm, less than 7.4 mm, less than 7.3 mm, less than 7.2 mm, less than 7.1 mm, less than 7.0 mm, less than 6.9 mm, less than 6.8 mm, less than 6.7 mm, less than 6.6 mm, less than 6.5 mm, less than 6.4 mm, less than 6.3 mm, less than 6.2 mm, less than 6.1 mm, less than 6.0 mm, less than 5.9 mm, less than 5.8 mm, less than 5.7 mm, less than 5.6 mm, less than 5.5 mm, less than 5.4 mm, less than 5.3 mm, less than 5.2 mm, less than 5.1 mm, less than 5.0 mm, less than 4.9 mm, less than 4.8 mm, less than 4.7 mm, less than 4.6 mm, less than 4.5 mm, less than 4.4 mm, less than 4.3 mm, less than 4.2 mm, less than 4.1 mm, less than 4.0 mm, less than 3.9 mm, less than 3.8 mm, less than 3.7 mm, less than 3.6 mm, less than 3.5 mm, less than 3.4 mm, less than 3.3 mm, less than 3.2 mm, less than 3.1 mm, less than 3.0 mm, less than 2.9 mm, less than 2.8 mm, less than 2.7 mm, less than 2.6 mm, less than 2.5 mm, less than 2.4 mm, less than 2.3 mm, less than 2.2 mm, less than 2.1 mm, less than 2.0 mm, less than 1.9 mm, less than 1.8 mm, less than 1.7 mm, less than 1.6 mm, less than 1.5 mm, less than 1.4 mm, less than 1.3 mm, less than 1.2 mm, less than 1.1 mm, less than 1.0 mm, less than 0.9 mm, less than 0.8 mm, less than 0.7 mm, less than 0.6 mm, less than 0.5 mm, less than 0.4 mm, less than 0.3 mm, less than 0.2 mm, less than 0.1 mm, less than 0.09 mm, less than 0.08 mm, less than 0.07 mm, less than 0.06 mm, less than 0.05 mm, less than 0.04 mm, less than 0.03 mm, less than 0.02 mm, less than 0.01 mm, less than 0.009 mm, less than 0.008 mm, less than 0.007 mm, less than 0.006 mm, less than 0.005 mm, less than 0.004 mm, less than 0.003 mm, less than 0.002 mm, less than 0.001 mm of a site of brain injury or into a blood vessel in close proximity to the site of brain injury.

The term "complication" as used herein refers to a pathological process or event that develops during the treatment of a preexisting disorder. Complications associated with subarachnoid hemorrhage include, but are not limited to, angiographic vasospasm, microthromboemboli, and cortical spreading ischemia.

The term "condition", as used herein, refers to a variety of health states and is meant to include disorders or diseases caused by any underlying mechanism or disorder, injury, and the promotion of healthy tissues and organs.

The term "contact" and all its grammatical forms as used herein refers to a state or condition of touching or of immediate or local proximity.

The term "controlled release" is intended to refer to any drug-containing formulation in which the manner and profile of drug release from the formulation are regulated. This refers to immediate as well as non-immediate release formulations, with non-immediate release formulations including, but not limited to, sustained release and delayed release formulations.

The term "cortical spreading depolarization" or "CSD" as used herein refers to a wave of near-complete neuronal depolarization and neuronal swelling in the brain that is ignited when passive cation influx across the cellular membrane exceeds ATP-dependent sodium and calcium pump activity. The cation influx is followed by water influx and shrinkage of the extracellular space by about 70%. If normal ion homoeostasis is not restored through additional recruitment of sodium and calcium pump activity, the cell swelling is maintained-a process then termed "cytotoxic edema," since it potentially leads to cell death through a protracted intracellular calcium surge and mitochondrial depolarization. CSD induces dilation of resistance vessels in healthy tissue; hence regional cerebral blood flow increases during the neuronal depolarization phase. (Dreier, J.P. et al., Brain 132: 1866-81 (2009).

The term "cortical spreading ischemia" or "CSI," or "inverse hemodynamic response" refers to a severe microvascular spasm that is coupled to the neuronal depolarization phase. The resulting spreading perfusion deficit prolongs neuronal depolarization [as reflected by a prolonged negative shift of the extracellular direct current (DC) potential] and the intracellular sodium and calcium surge. The hypoperfusion is significant enough to produce a mismatch between neuronal energy demand and supply. (Id.).

The term "delayed cerebral ischemia" or "DCI" as used herein refers to the occurrence of focal neurological impairment (such as hemiparesis, aphasia, apraxia, hemianopia, or neglect), or a decrease in the Glasgow coma scale (either on the total score or on one of its individual components [eye, motor on either side, verbal]).This may or may not last for at least one hour, is not apparent immediately after aneurysm occlusion and cannot be attributed to other causes by means of clinical assessment, CT or magnetic resonance imaging (MRI) scanning of the brain, and appropriate laboratory studies. Angiographic cerebral vasospasm is a description of a radiological test (either CT angiography [CTA], MR angiography [MRA] MRA or catheter angiography [CA]), and may be a cause of DCI.

The term "delayed release" is used herein in its conventional sense to refer to a drug formulation in which there is a time delay between administration of the formulation and the release of the drug there from. "Delayed release" may or may not involve gradual release of drug over an extended period of time, and thus may or may not be "sustained release."

The term "diffuse pharmacologic effect", as used herein, refers to a pharmacologic effect that spreads, disperses or scatters widely over a space or surface.

The term "disease" or "disorder", as used herein, refers to an impairment of health or a condition of abnormal functioning.

The term "disposed", as used herein, refers to being placed, arranged or distributed in a particular fashion.

The term "drug" as used herein refers to a therapeutic agent or any substance, other than food, used in the prevention, diagnosis, alleviation, treatment, or cure of disease.

The term "effective amount" refers to the amount necessary or sufficient to realize a desired biologic effect.

The term "emulsion" as used herein refers to a two-phase system prepared by combining two immiscible liquid carriers, one of which is disbursed uniformly throughout the other and consists of globules that have diameters equal to or greater than those of the largest colloidal particles. The globule size is critical and must be such that the system achieves maximum stability. Usually, separation of the two phases will occur unless a third substance, an emulsifying agent, is incorporated. Thus, a basic emulsion contains at least three components, the two immiscible liquid carriers and the emulsifying agent, as well as the active ingredient. Most emulsions incorporate an aqueous phase into a non-aqueous phase (or vice versa). However, it is possible to prepare emulsions that are basically non-aqueous, for example, anionic and cationic surfactants of the non-aqueous immiscible system glycerin and olive oil.

The term "flowable", as used herein, refers to that which is capable of movement in or as if in a stream by continuous change of relative position.

The term "granulomatous inflammation" as used herein refers to an inflammation reaction characterized by a predominance of regular to epithelioid macrophages with or without multinucleated giant cells and connective tissue.

The term "hydrogel" as used herein refers to a substance resulting in a solid, semisolid, pseudoplastic, or plastic structure containing a necessary aqueous component to produce a gelatinous or jelly-like mass.

The term "hypertension" as used herein refers to high systemic blood pressure; transitory or sustained elevation of systemic blood pressure to a level likely to induce cardiovascular damage or other adverse consequences.

The term "hypotension" as used herein refers to subnormal systemic arterial blood pressure; reduced pressure or tension of any kind.

The term "implanting" as used herein refers to grafting, embedding or inserting a substance, composition, or device into a pre-determined location within a tissue.

The term "impregnate", as used herein in its various grammatical forms refers to causing to be infused or permeated throughout; to fill interstices with a substance.

The term "infarction" as used herein refers to a sudden insufficiency of arterial or venous blood supply due to emboli, thrombi, mechanical factors, or pressure that produces a macroscopic area of necrosis. The term "cerebral infarction" as used herein refers to loss of brain tissue subsequent to the transient or permanent loss of circulation and/or oxygen delivery to the cerebrum region of the brain.

The term "inflammation" as used herein refers to the physiologic process by which vascularized tissues respond to injury. See, e.g., FUNDAMENTAL IMMUNOLOGY, 4th Ed., William E. Paul, ed. Lippincott-Raven Publishers, Philadelphia (1999) at 1051-1053, incorporated herein by reference. During the inflammatory process, cells involved in detoxification and repair are mobilized to the compromised site by inflammatory mediators. Inflammation is often characterized by a strong infiltration of leukocytes at the site of inflammation, particularly neutrophils (polymorphonuclear cells). These cells promote tissue damage by releasing toxic substances at the vascular wall or in uninjured tissue. Traditionally, inflammation has been divided into acute and chronic responses.

The term "injury," as used herein, refers to damage or harm to a structure or function of the body caused by an outside agent or force, which may be physical or chemical.

The term "ischemia" as used herein refers to a lack of blood supply and oxygen that occurs when reduced perfusion pressure distal to an abnormal narrowing (stenosis) of a blood vessel is not compensated by autoregulatory dilation of the resistance vessels.

The term "isolated molecule" as used herein refers to a molecule that is substantially pure and is free of other substances with which it is ordinarily found in nature or in vivo systems to an extent practical and appropriate for its intended use.

The terms "in the body", "void volume", "resection pocket", "excavation", "injection site", "deposition site" or "implant site" or "site of delivery"as used herein are meant to include all tissues of the body without limit, and may refer to spaces formed therein from injections, surgical incisions, tumor or tissue removal, tissue injuries, abscess formation, or any other similar cavity, space, or pocket formed thus by action of clinical assessment, treatment or physiologic response to disease or pathology as non-limiting examples thereof.

The phrase "localized administration", as used herein, refers to administration of a therapeutic agent in a particular location in the body that may result in a localized pharmacologic effect (i.e., a pharmacologic effect limited to a certain location) or a diffuse pharmacologic effect (i.e., a pharmacologic effect that spreads, disperses or scatters widely over a aspace or a surface).

The phrase "localized pharmacologic effect", as used herein, refers to a pharmacologic effect limited to a certain location, i.e. in close proximity to a certain location, place, area or site.

The term "long-term" release, as used herein, means that the implant is constructed and arranged to deliver therapeutic levels of the active ingredient for at least 7 days, and potentially up to about 30 to about 60 days.

The term "microthromboembolus" (or plural "microthromboemboli") as used here refers to a small fragment of blood clot that causes obstruction or occlusion of a blood vessel.

The term "modulate" as used herein means to regulate, alter, adapt, or adjust to a certain measure or proportion.

The term "optionally", as used herein, means that the pharmacuetical composition of the described invention may or may not contain a pharmaceutically acceptable carrier, and includes a pharmaceutical composition containing both a microparticulate formulation of a voltage-gated calcium channel blocker and a pharmaceutically acceptable carrier.

The term "parenteral" as used herein refers to introduction into the body by way of an injection (i.e., administration by injection) outside the gastrointestinal tract, including, for example, subcutaneously (i.e., an injection beneath the skin), intramuscularly (i.e., an injection into a muscle); intravenously (i.e., an injection into a vein), intrathecally (i.e., an injection into the space around the spinal cord or under the arachnoid membrane of the brain), intrasternal injection, or infusion techniques. A parenterally administered composition is delivered using a needle, e.g., a surgical needle. The term "surgical needle" as used herein, refers to any needle adapted for delivery of fluid (i.e., capable of flow) compositions into a selected anatomical structure. Injectable preparations, such as sterile injectable aqueous or oleaginous suspensions, may be formulated according to the known art using suitable dispersing or wetting agents and suspending agents.

The term "paresis" as used herein refers to partial or incomplete paralysis.

The terms "particles" or "microparticles", as used herein, refer to extremely small constituents, e.g., nanoparticles or microparticles) that may contain in whole or in part at least one therapeutic agent as described herein. The particles may contain therapeutic agent(s) in a core surrounded by a coating. Therapeutic agent(s) also may be dispersed throughout the particles. Therapeutic agent(s) also may be adsorbed into the particles. The particles may be of any order release kinetics, including zero order release, first order release, second order release, delayed release, sustained release, immediate release, etc., and any combination thereof The particle may include, in addition to therapeutic agent(s), any of those materials routinely used in the art of pharmacy and medicine, including, but not limited to, erodible, nonerodible, biodegradable, or nonbiodegradable material or combinations thereof. The particles may be microcapsules that contain the voltage-gated calcium channel blocker in a solution or in a semi-solid state. The particles may be of virtually any shape.

The term "pharmaceutically acceptable carrier" as used herein refers to one or more compatible solid or liquid filler, diluents or encapsulating substances which are suitable for administration to a human or other vertebrate animal. The term "carrier" as used herein refers to an organic or inorganic ingredient, natural or synthetic, with which the active ingredient is combined to facilitate the application. The components of the pharmaceutical compositions also are capable of being commingled in a manner such that there is no interaction which would substantially impair the desired pharmaceutical efficiency.

The term "pharmaceutical composition" is used herein to refer to a composition that is employed to prevent, reduce in intensity, cure or otherwise treat a target condition or disease.

The term "pharmacologic effect", as used herein, refers to a result or consequence of exposure to an active agent.

The phrase "predominantly localized pharmacologic effect", as used herein, refers to a pharmacologic effect of a drug limited to a certain location by at least 1 to 3 orders of magnitude achieved with a localized administration as compared to a systemic administration.

The term "prognosis" as used herein refers to an expected future cause and outcome of a disease or disorder, based on medical knowledge.

The term "reduce" or "reducing" as used herein refers to a diminution, a decrease, an attenuation, limitation or abatement of the degree, intensity, extent, size, amount, density, number or occurrence of the disorder in individuals at risk of developing the disorder.

The term "subacute inflammation" as used herein refers to a tissue reaction typically seen subsequent to the early inflammatory process characterized by a mixture of neutrophils, lymphocytes, and occasionally macrophages and/or plasma cells.

The term "subarachnoid hemorrhage" or "SAH" is used herein to refer to a condition in which blood collects beneath the arachnoid mater. This area, called the subarachnoid space, normally contains cerebrospinal fluid. The accumulation of blood in the subarachnoid space may lead to stroke, seizures, and other complications. Additionally, SAH may cause permanent brain damage and a number of harmful biochemical events in the brain. Causes of SAH include bleeding from a cerebral aneurysm, vascular anomaly, trauma and extension into the subarachnoid space from a primary intracerebral hemorrhage. Symptoms of SAH include, for example, sudden and severe headache, nausea and/or vomiting, symptoms of meningeal irritation (e.g., neck stiffness, low back pain, bilateral leg pain), photophobia and visual changes, and/or loss of consciousness. SAH is often secondary to a head injury or a blood vessel defect known as an aneurysm. In some instances, SAH can induce cerebral vasospasm that may in turn lead to an ischemic stroke. A common manifestation of a SAH is the presence of blood in the CSF. Subjects having a SAH may be identified by a number of symptoms. For example, a subject having a subarachnoid hemorrhage will present with blood in the subarachnoid, usually in a large amount. Subjects having a subarachnoid hemorrhage can also be identified by an intracranial pressure that approximates mean arterial pressure, by a fall in cerebral perfusion pressure, or by the sudden transient loss of consciousness (sometimes preceded by a painful headache). In about half of cases, subjects present with a severe headache which may be associated with physical exertion. Other symptoms associated with subarachnoid hemorrhage include nausea, vomiting, memory loss, hemiparesis and aphasia. Subjects having a SAH also may be identified by the presence of creatine kinase-BB isoenzyme activity in their CSF. This enzyme is enriched in the brain but normally is not present in the CSF. Thus, its presence in the CSF is indicative of "leak" from the brain into the subarachnoid space. Assay of creatine-kinase BB isoenzyme activity in the CSF is described by Coplin et al. (Coplin et al 1999 Arch Neurol 56, 1348-1352) Additionally, a spinal tap or lumbar puncture may be used to demonstrate whether blood is present in the CSF, a strong indication of a subarachnoid hemorrhage. A cranial CT scan or an MRI also may be used to identify blood in the subarachnoid region. Angiography also may be used to determine not only whether a hemorrhage has occurred, but also the location of the hemorrhage. Subarachnoid hemorrhage commonly results from rupture of an intracranial saccular aneurysm or from malformation of the arteriovenous system in, and leading to, the brain. Accordingly, a subject at risk of having a subarachnoid hemorrhage includes a subject having a saccular aneurysm as well as a subject having a malformation of the arteriovenous system. Common sites of saccular aneurysms are the top of the basilar artery and the junction of the basilar artery with the superior cerebellar or the anterior inferior cerebellar artery. Subjects having a subarachnoid hemorrhage may be identified by an eye examination, whereby slowed eye movement may indicate brain damage. A subject with a saccular aneurysm may be identified through routine medical imaging techniques, such as CT and MRI. A saccular or cerebral aneurysm forms a mushroom-like or berry-like shape (sometimes referred to as "a dome with a neck" shape).

The terms "subject" or "individual" or "patient" are used interchangeably to refer to a member of an animal species of mammalian origin, including humans.

The phrase "a subject having vasospasm" as used herein refers to a subject who presents with diagnostic markers and symptoms associated with vasospasm. Diagnostic markers include, but are not limited to, the presence of blood in the CSF and/or a recent history of a subarachnoid hemorrhage. Vasospasm associated symptoms include, but are not limited to, paralysis on one side of the body, inability to vocalize the words or to understand spoken or written words, and inability to perform tasks requiring spatial analysis. Such symptoms may develop over a few days, or they may fluctuate in their appearance, or they may present abruptly.

The phrase "a subject having angiographic vasospasm" as used herein refers to a subject who presents with diagnostic markers associated with vasospasm. Diagnostic markers include, but are not limited to, the presence of blood in the CSF, a recent history of a SAH and/or reduction in the lumen diameter of cerebral arteries observed on a catheter, computed tomographic or magnetic resonance angiogram one to 14 days after an SAH or TBI. Vasospasm associated symptoms include, but are not limited to, paralysis on one side of the body, inability to vocalize the words or to understand spoken or written words, and inability to perform tasks requiring spatial analysis. Such symptoms may develop over a few days, or they may fluctuate in their appearance, or they may present abruptly. Transcranial Doppler ultrasound also may be used to diagnose and monitor the progression of, for example, a angiographic vasospasm. Presence of blood in the CSF may be detected using CT scans. However, in some instances where the amount of blood is so small as to not be detected by CT, a lumbar puncture is warranted.

The phrase "a subject having cerebral vasospasm" as used herein refers to one who has symptoms of or has been diagnosed with cerebral vasospasm. A subject at risk of cerebral vasospasm is one who has one or more predisposing factors to the development of cerebral vasospasms. A predisposing factor includes, but is not limited to, existence of a subarachnoid hemorrhage. A subject who has experienced a recent SAH is at significantly higher risk of developing cerebral vasospasm than a subject who has not had a recent SAH. MR angiography, CT angiography and catheter angiography can be used to diagnose cerebral vasospasm Angiography is a technique in which a contrast agent is introduced into the blood stream in order to view blood flow and/or arteries. A contrast agent is required because blood flow and/or arteries sometimes are only weakly apparent in a regular MR scan, CT scan or radiographic film for catheter angiography. Appropriate contrast agents will vary depending upon the imaging technique used. For example, gadolinium is commonly used as a contrast agent used in MR scans. Other MR appropriate contrast agents are known in the art.

The phrase "a subject having "delayed cerebral ischemia" or "DCI" as used herein refers to a subject who presents with diagnostic markers associated with DCI. Diagnostic markers include, but are not limited to, the presence of blood in the CSF and/or a recent history of a SAH and/or development of neurological deterioration one to 14 days after SAH when the neurological deterioration is not due to another cause that can be diagnosed, including but not limited to seizures, hydrocephalus, increased intracranial pressure, infection, intracranial hemorrhage or other systemic factors. DCI-associated symptoms include, but are not limited to, paralysis on one side of the body, inability to vocalize the words or to understand spoken or written words, and inability to perform tasks requiring spatial analysis. Such symptoms may develop over a few days, or they may fluctuate in their appearance, or they may present abruptly.

The phrase "a subject having microthromboemboli" as used herein refers to a subject who presents with diagnostic markers associated with microthromboemboli. Diagnostic markers include, but are not limited to, the presence of blood in the CSF and/or a recent history of a SAH and/or development of neurological deterioration one to 14 days after SAH when the neurological deterioration is not due to another cause that can be diagnosed, including but not limited to seizures, hydrocephalus, increased intracranial pressure, infection, intracranial hemorrhage or other systemic factors. Another diagnostic marker may be embolic signals detected on transcranial Doppler ultrasound of large conducting cerebral arteries. Microthromboemboli-associated symptoms include, but are not limited to, paralysis on one side of the body, inability to vocalize the words or to understand spoken or written words, and inability to perform tasks requiring spatial analysis. Such symptoms may develop over a few days, or they may fluctuate in their appearance, or they may present abruptly.

The phrase "a subject having cortical spreading ischemia" as used herein means refers to a subject who presents with diagnostic markers associated with cortical spreading ischemia. Diagnostic markers include, but are not limited to, the presence of blood in the CSF and/or a recent history of a SAH and/or development of neurological deterioration one to 14 days after SAH when the neurological deterioration is not due to another cause that can be diagnosed, including but not limited to seizures, hydrocephalus, increased intracranial pressure, infection, intracranial hemorrhage or other systemic factors. Another diagnostic marker may be detection of propagating waves of depolarization with vasoconstriction detected by electrocorticography. Cortical spreading ischemia-associated symptoms include, but are not limited to, paralysis on one side of the body, inability to vocalize the words or to understand spoken or written words, and inability to perform tasks requiring spatial analysis. Such symptoms may develop over a few days, or they may fluctuate in their appearance, or they may present abruptly.

A subject at risk of DCI, microthromboemboli, cortical spreading ischemia, or angiographic vasospasm is one who has one or more predisposing factors to the development of these conditions. A predisposing factor includes, but is not limited to, existence of a SAH. A subject who has experienced a recent SAH is at significantly higher risk of developing angiographic vasospasm and DCI than a subject who has not had a recent SAH. MR angiography, CT angiography and catheter angiography can be used to diagnose at least one of DCI, microthromboemboli, cortical spreading ischemia or angiographic vasospasm. Angiography is a technique in which a contrast agent is introduced into the blood stream in order to view blood flow and/or arteries. A contrast agent is required because blood flow and/or arteries sometimes are only weakly apparent in a regular MR scan, CT scan or radiographic film for catheter angiography.

Appropriate contrast agents will vary depending upon the imaging technique used. For example, gadolinium is commonly used as a contrast agent used in MR scans. Other MR appropriate contrast agents are known in the art.

The phrase "substantially pure" as used herein refers to a condition of a therapeutic agent such that it has been substantially separated from the substances with which it may be associated in living systems or during synthesis. According to some embodiments, a substantially pure therapeutic agent is at least 70% pure, at least 75% pure, at least 80% pure, at least 85% pure, at least 90% pure, at least 95% pure, at least 96% pure, at least 97% pure, at least 98% pure, or at least 99% pure.

The term "sustained release" (also referred to as "extended release") is used herein in its conventional sense to refer to a drug formulation that provides for gradual release of a drug over an extended period of time, and that preferably, although not necessarily, results in substantially constant blood levels of a drug over an extended time period. Alternatively, delayed absorption of a parenterally administered drug Form Is accomplished by dissolving or suspending the drug in an oil vehicle. Nonlimiting examples of sustained release biodegradable polymers include polyesters, polyester polyethylene glycol copolymers, polyamino-derived biopolymers, polyanhydrides, polyorthoesters, polyphosphazenes, SAIB, photopolymerizable biopolymers, protein polymers, collagen, polysaccharides, chitosans, and alginates.

The term "syndrome," as used herein, refers to a pattern of symptoms indicative of some disease or condition.

The phrase "systemic administration", as used herein, refers to administration of a therapeutic agent with a pharmacologic effect on the entire body. Systemic administration includes enteral administration (e.g. oral) through the gastrointestinal tract and parenteral administration (e.g. intravenous, intramuscular, etc.) outside the gastrointestinal tract.

The term "therapeutic amount" or an "amount effective" of one or more of the active agents is an amount that is sufficient to provide the intended benefit of treatment. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen may be planned which does not cause substantial toxicity and yet is effective to treat the particular subject. A therapeutically effective amount of the active agents that can be employed ranges from generally 0.1 mg/kg body weight and about 50 mg/kg body weight. Therapeutically effective amount for any particular application may vary depending on such factors as the disease or condition being treated, the particular voltage-gated calcium channel blocker being administered, the size of the subject, or the severity of the disease or condition. One of ordinary skill in the art may determine empirically the effective amount of a particular inhibitor and/or other therapeutic agent without necessitating undue experimentation. It is preferred generally that a maximum dose be used, that is, the highest safe dose according to some medical judgment. However, dosage levels are based on a variety of factors, including the type of injury, the age, weight, sex, medical condition of the patient, the severity of the condition, the route of administration, and the particular active agent employed. Thus the dosage regimen may vary widely, but can be determined routinely by a surgeon using standard methods. "Dose" and "dosage" are used interchangeably herein.

The term "therapeutic agent" as used herein refers to a drug, molecule, nucleic acid, protein, composition or other substance that provides a therapeutic effect. The terms "therapeutic agent" and "active agent" are used interchangeably. The active agent may be a calcium channel inhibitor, a calcium channel antagonist, a calcium channel blocker, a transient receptor potential protein blocker, or an endothelin anatagonist.

Therapeutic agent(s), including the calcium channel inhibitor, the calcium channel antagonist, the calcium channel blocker, a transient receptor potential protein blocker, and/or an endothelin antagonist may be provided in particles.

The term "therapeutic component" as used herein refers to a therapeutically effective dosage (i.e., dose and frequency of administration) that eliminates, reduces, or prevents the progression of a particular disease manifestation in a percentage of a population. An example of a commonly used therapeutic component is the ED50 which describes the dose in a particular dosage that is therapeutically effective for a particular disease manifestation in 50% of a population.

The term "therapeutic effect" as used herein refers to a consequence of treatment, the results of which are judged to be desirable and beneficial. A therapeutic effect may include, directly or indirectly, the arrest, reduction, or elimination of a disease manifestation. A therapeutic effect may also include, directly or indirectly, the arrest reduction or elimination of the progression of a disease manifestation.

The term "topical" refers to administration of a composition at, or immediately beneath, the point of application. The phrase "topically applying" describes application onto one or more surfaces(s) including epithelial surfaces. Topical administration, in contrast to transdermal administration, generally provides a local rather than a systemic effect.

The term "transient receptor potential protein blocker" as used herein refers to a protein that is structurally distinct from other calcium channel blockers and that blocks intracellular calcium increases in cells due to receptor-mediated calcium influx.

The term "transient receptor potential protein antagonist" as used herein refers to a protein that is structurally distinct from other calcium channel antagonists and that antagonizes intracellular calcium increases in cells due to receptor-mediated calcium influx. Transient receptor potential protein blockers and antagonists include, but are not limited to, SK&F 96365 (1-(beta-[3-(4- methoxy-phenyl)propoxy]-4-methoxyphenethyl)-1H- imidazole hydrochloride) and LOE 908 (RS)-(3,4-dihydro-6,7-dimethoxyisoquinoline-1-gamma 1)-2-phenyl-N,N-dit2-(2,3,4- trimethoxyphenypethyl]acetamide).

The term "treat" or "treating" includes abrogating, substantially inhibiting, slowing or reversing the progression of a disease, condition or disorder, substantially ameliorating clinical or esthetical symptoms of a condition, substantially preventing the appearance of clinical or esthetical symptoms of a disease, condition, or disorder, and protecting from harmful or annoying symptoms. Treating further refers to accomplishing one or more of the following: (a) reducing the severity of the disorder; (b) limiting development of symptoms characteristic of the disorder(s) being treated; (c) limiting worsening of symptoms characteristic of the disorder(s) being treated; (d) limiting recurrence of the disorder(s) in patients that have previously had the disorder(s); and (e) limiting recurrence of symptoms in patients that were previously asymptomatic for the disorder(s).

The term "vasoconstriction" as used herein refers to the narrowing of the blood vessels resulting from contracting of the muscular wall of the vessels. When blood vessels constrict, the flow of blood is restricted or slowed.

The term "vasodilation" which is the opposite of vasoconstriction as used herein refers to the widening of blood vessels. The terms "vasoconstrictors," "vasopressors," or "pressors" as used herein refer to factors causing vasoconstriction.

The term "vasospasm" as used herein refers to a decrease in the internal diameter of a cerebral artery that results from contraction of smooth muscle within the wall of the artery which causes a decrease in blood flow, but generally without an increase in systemic vascular resistance. Vasospasm results in decreased cerebral blood flow and increased cerebral vascular resistance. Without being limited by theory, it generally is believed that vasospasm is caused by local injury to vessels, such as that which results from atherosclerosis and other structural injury including traumatic head injury, aneurismal subarachnoid hemorrhage and other causes of subarachnoid hemorrhage. Cerebral vasospasm is a naturally occurring vasoconstriction that also may be triggered by the presence of blood in the CSF, a common occurrence after rupture of an ancurysm or following traumatic head injury. Cerebral vasospasm ultimately can lead to brain cell damage, in the form of cerebral ischemia and infarction, due to interrupted blood supply. The term "cerebral vasospasm" as used herein further refers to the delayed occurrence of narrowing of large capacitance arteries at the base of the brain after subarachnoid hemorrhage, often associated with diminished perfusion in the territory distal to the affected vessel. Cerebral vasospasm may occur any time after rupture of an aneurysm but most commonly peaks at seven days following the hemorrhage and often resolves within 14 days when the blood has been absorbed by the body. Angiographic vasospasm is a consequence of SAH, but also can occur after any condition that deposits blood in the subarachnoid space. More specifically, the term "angiographic cerebral vasospasm" refers to the narrowing of the large capacitance arteries at the base of the brain (i.e., cerebral arteries) following hemorrhage into the subarachnoid space, and leads to reduced perfusion of distal brain regions.

**I. Compositions**

In one aspect, the described invention provides a pharmaceutical composition comprising (i) a microparticulate formulation of a voltage-gated calcium channel blocker; and optionally (ii) a pharmaceutically acceptable carrier.

According to some embodiments, the pharmaceutical composition can prevent or reduce the incidence or severity of at least one delayed complication associated with a brain injury in a mammal in need thereof, wherein the brain injury includes interruption of at least one cerebral artery. According to some such embodiments, the at least one delayed complication is selected from the group consisting of a delayed cerebral ischemia (DCI), an intracerebral hematoma, an intraventricular hemorrhage, a fever, an angiographic vasospasm, a microthromboembolus, a cortical spreading ischemia (CSI), a behavioral deficit, a neurological deficit, a cerebral infarction, neuronal cell death, or a combination thereof. According to one embodiment, the at least one delayed complication is a delayed cerebral ischemia (DCI). According to another embodiment, the at least one delayed complication is an intracerebral hematoma. According to another embodiment, the at least one delayed complication is an intraventricular hemorrhage. According to another embodiment, the at least one delayed complication is a fever. According to another embodiment, the at least one delayed complication is an angiographic vasospasm. According to another embodiment, the at least one delayed complication is a cortical spreading ischemia (CSI). According to another embodiment, the at least one delayed complication is a microthrombocmbolism. According to another embodiment, the at least one delayed complication is a behavioral deficit. According to another embodiment, the at least one delayed complication is a neurological deficit. According to another embodiment, the at least one delayed complication is a cerebral infarction. According to another embodiment, the at least one delayed complication is neuronal cell death.

According to some embodiments, the brain injury is a result of an underlying condition. Exemplary underlying conditions include, but are not limited to, aneurysm, sudden traumatic head injury, subarachnoid hemorrhage (SAH), or a combination thereof. According to one embodiment, the underlying condition is an aneurysm. According to another embodiment, the underlying condition is a sudden traumatic head injury. According to another embodiment, the underlying condition is a subarachnoid hemorrhage (SAH). According to another embodiment, the underlying condition is a combination of an aneurysm, a sudden traumatic head injury, and a subarachnoid hemorrhage (SAH).

According to some embodiments, the pharmaceutical composition, when administered in a therapeutic amount at a site of delivery in the mammal, is effective in preventing or reducing the incidence or severity of at least one delayed complication associated with a brain injury in a mammal in need thereof, wherein the brain injury includes interruption of at least one cerebral artery. According to one embodiment, the site of delivery is a cerebral ventricle. According to some embodiments, the cerebral ventricle is selected from the group consisting of a lateral ventricle, a third ventricle, a fourth ventricle, or a combination thereof. According to one embodiment, the site of delivery is in subarachnoid space. According to one embodiment, the site of delivery is in close proximity to the brain injury. According to another embodiment, the site of delivery is in close proximity to a blood vessel that is affected by the brain injury. According to one embodiment, the blood vessel is at least one cerebral artery. According to another embodiment, the blood vessel is the at least one cerebral artery affected by the brain injury.

According to some embodiments, the site of delivery is within 10 mm, less than 10 mm, less than 9.9 mm, less than 9.8 mm, less than 9.7 mm, less than 9.6 mm, less than 9.5 mm, less than 9.4 mm, less than 9.3 mm, less than 9.2 mm, less than 9.1 mm, less than 9.0 mm, less than 8.9 mm, less than 8.8 mm, less than 8.7 mm, less than 8.6 mm, less than 8.5 mm, less than 8.4 mm, less than 8.3 mm, less than 8.2 mm, less than 8.1 mm, less than 8.0 mm, less than 7.9 mm, less than 7.8 mm, less than 7.7 mm, less than 7.6 mm, less than 7.5 mm, less than 7.4 mm, less than 7.3 mm, less than 7.2 mm, less than 7.1 mm, less than 7.0 mm, less than 6.9 mm, less than 6.8 mm, less than 6.7 mm, less than 6.6 mm, less than 6.5 mm, less than 6.4 mm, less than 6.3 mm, less than 6.2 mm, less than 6.1 mm, less than 6.0 mm, less than 5.9 mm, less than 5.8 mm, less than 5.7 mm, less than 5.6 mm, less than 5.5 mm, less than 5.4 mm, less than 5.3 mm, less than 5.2 mm, less than 5.1 mm, less than 5.0 mm, less than 4.9 mm, less than 4.8 mm, less than 4.7 mm, less than 4.6 mm, less than 4.5 mm, less than 4.4 mm, less than 4.3 mm, less than 4.2 mm, less than 4.1 mm, less than 4.0 mm, less than 3.9 mm, less than 3.8 mm, less than 3.7 mm, less than 3.6 mm, less than 3.5 mm, less than 3.4 mm, less than 3.3 mm, less than 3.2 mm, less than 3.1 mm, less than 3.0 mm, less than 2.9 mm, less than 2.8 mm, less than 2.7 mm, less than 2.6 mm, less than 2.5 mm, less than 2.4 mm, less than 2.3 mm, less than 2.2 mm, less than 2.1 mm, less than 2.0 mm, less than 1.9 mm, less than 1.8 mm, less than 1.7 mm, less than 1.6 mm, less than 1.5 mm, less than 1.4 mm, less than 1.3 mm, less than 1.2 mm, less than 1.1 mm, less than 1.0 mm, less than 0.9 mm, less than 0.8 mm, less than 0.7 mm, less than 0.6 mm, less than 0.5 mm, less than 0.4 mm, less than 0.3 mm, less than 0.2 mm, less than 0.1 mm, less than 0.09 mm, less than 0.08 mm, less than 0.07 mm, less than 0.06 mm, less than 0.05 mm, less than 0.04 mm, less than 0.03 mm, less than 0.02 mm, less than 0.01 mm, less than 0.009 mm, less than 0.008 mm, less than 0.007 mm, less than 0.006 mm, less than 0.005 mm, less than 0.004 mm, less than 0.003 mm, less than 0.002 mm, less than 0.001 mm of the brain injury.

According to some embodiments, the site of delivery is within 10 mm, less than 10 mm, less than 9.9 mm, less than 9.8 mm, less than 9.7 mm, less than 9.6 mm, less than 9.5 mm, less than 9.4 mm, less than 9.3 mm, less than 9.2 mm, less than 9.1 mm, less than 9.0 mm, less than 8.9 mm, less than 8.8 mm, less than 8.7 mm, less than 8.6 mm, less than 8.5 mm, less than 8.4 mm, less than 8.3 mm, less than 8.2 mm, less than 8.1 mm, less than 8.0 mm, less than 7.9 mm, less than 7.8 mm, less than 7.7 mm, less than 7.6 mm, less than 7.5 mm, less than 7.4 mm, less than 7.3 mm, less than 7.2 mm, less than 7.1 mm, less than 7.0 mm, less than 6.9 mm, less than 6.8 mm, less than 6.7 mm, less than 6.6 mm, less than 6.5 mm, less than 6.4 mm, less than 6.3 mm, less than 6.2 mm, less than 6.1 mm, less than 6.0 mm, less than 5.9 mm, less than 5.8 mm, less than 5.7 mm, less than 5.6 mm, less than 5.5 mm, less than 5.4 mm, less than 5.3 mm, less than 5.2 mm, less than 5.1 mm, less than 5.0 mm, less than 4.9 mm, less than 4.8 mm, less than 4.7 mm, less than 4.6 mm, less than 4.5 mm, less than 4.4 mm, less than 4.3 mm, less than 4.2 mm, less than 4.1 mm, less than 4.0 mm, less than 3.9 mm, less than 3.8 mm, less than 3.7 mm, less than 3.6 mm, less than 3.5 mm, less than 3.4 mm, less than 3.3 mm, less than 3.2 mm, less than 3.1 mm, less than 3.0 mm, less than 2.9 mm, less than 2.8 mm, less than 2.7 mm, less than 2.6 mm, less than 2.5 mm, less than 2.4 mm, less than 2.3 mm, less than 2.2 mm, less than 2.1 mm, less than 2.0 mm, less than 1.9 mm, less than 1.8 mm, less than 1.7 mm, less than 1.6 mm, less than 1.5 mm, less than 1.4 mm, less than 1.3 mm, less than 1.2 mm, less than 1.1 mm, less than 1.0 mm, less than 0.9 mm, less than 0.8 mm, less than 0.7 mm, less than 0.6 mm, less than 0.5 mm, less than 0.4 mm, less than 0.3 mm, less than 0.2 mm, less than 0.1 mm, less than 0.09 mm, less than 0.08 mm, less than 0.07 mm, less than 0.06 mm, less than 0.05 mm, less than 0.04 mm, less than 0.03 mm, less than 0.02 mm, less than 0.01 mm, less than 0.009 mm, less than 0.008 mm, less than 0.007 mm, less than 0.006 mm, less than 0.005 mm, less than 0.004 mm, less than 0.003 mm, less than 0.002 mm, less than 0.001 mm of the at least one cerebral artery affected by the brain injury.

According to some embodiments, the pharmaceutical composition is capable of releasing the voltage gated calcium channel blocker at the site of delivery within a half-life ranging from 1 day to 30 days. According to one embodiment, the pharmaceutical composition is capable of releasing the voltage gated calcium channel blocker at the site of delivery within a half-life of 1 day. According to another embodiment, the pharmaceutical composition is capable of releasing the voltage gated calcium channel blocker at the site of delivery within a half-life of 2 days. According to another embodiment, the pharmaceutical composition is capable of releasing the voltage gated calcium channel blocker at the site of delivery within a half-life of 3 days. According to another embodiment, the pharmaceutical composition is capable of releasing the voltage gated calcium channel blocker within a half-life of 4 days. According to another embodiment, the pharmaceutical composition is capable of releasing the voltage gated calcium channel blocker at the site of delivery within a half-life of 5 days. According to another embodiment, the pharmaceutical composition is capable of releasing the voltage gated calcium channel blocker at the site of delivery within a half-life of 6 days. According to another embodiment, the pharmaceutical composition is capable of releasing the voltage gated calcium channel blocker at the site of delivery within a half-life of 7 days. According to another embodiment, the pharmaceutical composition is capable of releasing the voltage gated calcium channel blocker at the site of delivery within a half-life of 8 days. According to another embodiment, the pharmaceutical composition is capable of releasing the voltage gated calcium channel blocker at the site of delivery within a half-life of 9 days. According to another embodiment, the pharmaceutical composition is capable of releasing the voltage gated calcium channel blocker at the site of delivery within a half-life of 10 days. According to another embodiment, the pharmaceutical composition is capable of releasing the voltage gated calcium channel blocker at the site of delivery within a half-life of 15 days. According to another embodiment, the pharmaceutical composition is capable of releasing the voltage gated calcium channel blocker at the site of delivery within a half-life of 30 days.

According to another embodiment, the release of the voltage-gated calcium channel blocker at the site of delivery can produce a predominantly localized pharmacologic effect over a desired amount of time. According to one embodiment, the release of the voltage-gated calcium channel blocker at the site of delivery produces a predominantly localized pharmacologic effect for 1 day. According to one embodiment, the release of the voltage-gated calcium channel blocker at the site of delivery produces a predominantly localized pharmacologic effect for 2 days. According to one embodiment, the release of the voltage-gated calcium channel blocker at the site of delivery produces a predominantly localized pharmacologic effect for 3 days. According to one embodiment, the release of the voltage-gated calcium channel blocker at the site of delivery produces a predominantly localized pharmacologic effect for 4 days. According to one embodiment, the release of the voltage-gated calcium channel blocker at the site of delivery produces a predominantly localized pharmacologic effect for 5 days. According to one embodiment, the release of the voltage-gated calcium channel blocker at the site of delivery produces a predominantly localized pharmacologic effect for 6 days. According to one embodiment, the release of the voltage-gated calcium channel blocker at the site of delivery produces a predominantly localized pharmacologic effect for 7 days. According to one embodiment, the release of the voltage-gated calcium channel blocker at the site of delivery produces a predominantly localized pharmacologic effect for 8 days. According to one embodiment, the release of the voltage-gated calcium channel blocker at the site of delivery produces a predominantly localized pharmacologic effect for 15 days. According to one embodiment, the release of the voltage-gated calcium channel blocker at the site of delivery produces a predominantly localized pharmacologic effect for 30 days.

According to another embodiment, the release of the voltage-gated calcium channel blocker at the site of delivery produces a diffuse pharmacologic effect throughout the central nervous system (CNS) over a desired amount of time. According to another embodiment, the release of the voltage-gated calcium channel blocker can produce a diffuse pharmacologic effect throughout the central nervous system (CNS) for 1 day. According to another embodiment, the release of the voltage-gated calcium channel blocker can produce a diffuse pharmacologic effect throughout the central nervous system (CNS) for 2 days. According to another embodiment, the release of the voltage-gated calcium channel blocker can produce a diffuse pharmacologic effect throughout the central nervous system (CNS) for 3 days. According to another embodiment, the release of the voltage-gated calcium channel blocker can produce a diffuse pharmacologic effect throughout the central nervous system (CNS) for 4 days. According to another embodiment, the release of the voltage-gated calcium channel blocker can produce a diffuse pharmacologic effect throughout the central nervous system (CNS) for 5 days. According to another embodiment, the release of the voltage-gated calcium channel blocker can produce a diffuse pharmacologic effect throughout the central nervous system (CNS) for 6 days. According to another embodiment, the release of the voltage-gated calcium channel blocker can produce a diffuse pharmacologic effect throughout the central nervous system (CNS) for 7 days. According to another embodiment, the release of the voltage-gated calcium channel blocker can produce a diffuse pharmacologic effect for throughout the central nervous system (CNS) 8 days. According to another embodiment, the release of the voltage-gated calcium channel blocker can produce a diffuse pharmacologic effect throughout the central nervous system (CNS) for 15 days. According to another embodiment, the release of the voltage-gated calcium channel blocker can produce a diffuse pharmacologic effect for throughout the central nervous system (CNS) 30 days.

According to one embodiment, the localized pharmacologic effect at the site of delivery is a reduction of vasospasm such that internal diameter of the at least one cerebral artery affected by the brain injury is increased compared to a control. According to one embodiment, the pharmaceutical composition is effective to increase the internal diameter of the cerebral artery affected by the brain injury as compared to a control.

According to one embodiment, the diffuse pharmacologic effect is a reduction of vasospasm such that internal diameter of a blood vessel that is at least 10 mm, at least 9.9 mm, at least 9.8 mm, at least 9.7 mm, at least 9.6 mm, at least 9.5 mm, at least 9.4 mm, at least 9.3 mm, at least 9.2 mm, at least 9.1 mm, at least 9.0 mm, at least 8.9 mm, at least 8.8 mm, at least 8.7 mm, at least 8.6 mm, at least 8.5 mm, at least 8.4 mm, at least 8.3 mm, at least 8.2 mm, at least 8.1 mm, at least 8.0 mm, at least 7.9 mm, at least 7.8 mm, at least 7.7 mm, at least 7.6 mm, at least 7.5 mm, at least 7.4 mm, at least 7.3 mm, at least 7.2 mm, at least 7.1 mm, at least 7.0 mm, at least 6.9 mm, at least 6.8 mm, at least 6.7 mm, at least 6.6 mm, at least 6.5 mm, at least 6.4 mm, at least 6.3 mm, at least 6.2 mm, at least 6.1 mm, at least 6.0 mm, at least 5.9 mm, at least 5.8 mm, at least 5.7 mm, at least 5.6 mm, at least 5.5 mm, at least 5.4 mm, at least 5.3 mm, at least 5.2 mm, at least 5.1 mm, at least 5.0 mm from the site of delivery is increased as compared to a control.

### Voltage-gated Calcium Channel Blocker

According to some embodiments, the voltage-gated channel blocker is selected from the group consisting of L-type voltage-gated calcium channel blocker, N-type voltage-gated calcium channel blocker, P/Q-type voltage-gated calcium channel blocker, or a combination thereof.

Non-limiting examples of the voltage-gated calcium channel blocker that can be formulated into the composition include, but are not limited to, L-type voltage-gated calcium channel blocker, N-type voltage-gated calcium channel blocker, P/Q-type voltage-gated calcium channel blocker, or a combination thereof.

For example, L-type voltage-gated calcium channel blockers include, but are not limited to: dihydropyridine L-type blockers such as nisoldipine, nicardipine and nifedipine, AHF (such as 4aR,9aS)-(+)-4a-Amino-1,2,3,4,4a,9a-hexahydro-4aH-fluorene, HCl), isradipine (such as 4-(4-Benzofurazanyl)-1,-4-dihydro-2,6-dimethyl-3,5-pyridinedicarboxylic acid methyl 1-methhylethyl ester), calciseptine (such as isolated from (Dendroaspis polylepis ploylepis), H-Arg-Ile-Cys-Tyr-Ile-His-Lys-Ala-Ser-Leu-Pro-Arg-Ala-Thr-Lys-Thr-Cys-Val-      Glu-Asn-Thr-Cys-Tyr-Lys-Met-Phe-Ile-Arg-Thr-Gln-Arg-Glu-Tyr-Ile-Ser-Glu-Arg-Gly-Cys-Gly-Cys-Pro-Thr-Ala-Met-Trp-Pro-Tyr-Gln-Thr-Glu-Cys-Cys-Lys-Gly-Asp-Arg-Cys-Asn-Lys-OH, Calcicludine (such as isolated from Dendroaspis angusticeps (eastern green mamba)),(H-Trp-Gln-Pro-Pro-Trp-Tyr-Cys-Lys-Glu-Pro-Val-Arg-Ile-Gly-Ser-Cys-Lys-Lys-Gln-Phe-Ser-Ser-Phe-Tyr-     Phe-Lys-Trp-Thr-Ala-Lys-Lys-Cys-Leu-Pro-Phe-Leu-Phe-Ser-Gly-Cys-Gly-Gly-Asn-Ala-Asn-Arg-Phe-Gln-Thr-Ile-Gly-Glu-Cys-Arg-Lys-Lys-Cys-Leu-Gly-Lys-OH, Cilnidipine (such as also FRP-8653, a dihydropyridine-type inhibitor), Dilantizem (such as (2S,3S)-(+)-cis-3-Acetoxy-5-(2-dimethylaminoethyl)-2,3-dihydro-2-(4-methoxyphenyl)-1,5-benzothiazepin-4(5H)-one hydrochloride), diltiazcm (such as benzothiazepin-4(5H)-one, 3-(acctyloxy)-5-[2-(dimethylamino)ethyl]-2,3-dihydro-2-(4-methoxyphenyl)--,(+)- cis-, monohydrochloride), Felodipine (such as 4-(2,3-Dichlorophenyl)-1,4-dihydro-2,6-dimethyl-3,5-pyridinecarboxylic acid ethyl methyl ester), FS-2 (such as an isolate from Dendroaspis polylepis polylepis venom), FTX-3.3 (such as an isolate from Agelenopsis aperta), Neomycin sulfate (such as $C_{23}H_{46}N_6O_{13} \cdot 3H_2SO_4$), Nicardipine (such as 1,4-Dihydro-2,6-dimethyl-4-(3-nitrophenypmethyl-2-[methyl(phenylmethypa-mino]-3,5-pyridinedicarboxylic acid ethyl ester hydrochloride, also YC-93, Nifedipine (such as 1,4-Dihydro-2,6-dimethyl-4-(2-nitrophenyl)-3,5-pyridinedicarboxylic acid dimethyl ester), Nimodipine (such as 4-Dihydro-2,6-dimethyl-4-(3-nitrophenyl)-3,5-pyridinedicarboxylic acid 2-methoxyethyl 1-methylethyl ester) or (Isopropyl 2-methoxyethyl 1,4-dihydro-2,6-dimethyl-4-(m¬nitrophenyl)-3,5-pyridinedicarboxylate), Nitrendipine (such as 1,4-Dihydro-2,6-dimethyl-4-(3- nitrophenyl)-3,5-pyridinedicarboxylic acid ethyl methyl ester), S-Petasin (such as (3S,4aR,5R,6R)-[2,3,4,4a,5,6,7,8-Octahydro-3-(2-propenyl)-4a,5-dimethyl-2-o-xo-6-naphthyl]Z¬3'-methylthio-1'-propenoate), Phloretin (such as 2',4',6'-Trihydroxy-3 -(4-hydroxyphenyl)propiophenone, also 3-(4-Hydroxyphenyl)-1-(2,4,6-trihydroxyphenyl)-1-propanone, also b-(4-Hydroxyphenyl)-2,4,6-trihydroxypro-piophenone), Protopine (such as $C_{20}H_{19}NO_5Cl$), SKF-96365 (such as 1-[b-[3-(4-Methoxyphenyl)propoxy]-4-methoxyphenethyl]-1H-imidazole, HCl), Tetrandine (such as 6,6',7,12-Tetramethoxy-2,2'-dimcthylbcrbaman), (.+-.)-Mcthoxyvcrapamil or (+)-Vcrapamil (such as 54N-(3,4-Dimethoxyphenylethyl)methylamino]-2-(3,4-dimethoxyphenyl)-2-iso-propylvaleronitrile hydrochloride), and (R)-(+)-Bay K8644 (such as R-(+)-1,4-Dihydro-2,6-dimethyl-5-nitro-442-(trifluoromethyl)phenyl]-3-pyridinecarboxylic acid methyl ester). The foregoing examples may be specific to L-type voltage-gated calcium channels or may inhibit a broader range of voltage- gated calcium channels, e.g. N, P/Q, R, and T-type.

According to some embodiments, the voltage-gated calcium channel blocker is a dihydropyridine calcium channel blocker. According to one embodiment, the dihydropyridine calcium channel blocker is nimodipine. According to one embodiment, the nimodipine has a half life of 7-10 days when formulated as described herein, and appropriate lipid solubility.

According to some embodiments, the voltage gated calcium channel blocker is an isolated molecule. The term "isolated molecule" as used herein refers to a molecule that is substantially pure and is free of other substances with which it is ordinarily found in nature or in vivo systems to an extent practical and appropriate for its intended use.

According to some embodiments, the voltage gated calcium channel blocker is admixed with a pharmaceutically-acceptable carrier in a pharmaceutical preparation. According to some such embodiments, the voltage gated calcium channel blocker comprises only a small percentage by weight of the preparation. According to some embodiments, the voltage gated calcium channel blocker is substantially pure.

### Pharmaceutically acceptable carrier

According to some embodiments, the pharmaceutical composition does not comprise the pharmaceutically acceptable carrier. According to some embodiments, the pharmaceutically acceptable carrier does not comprise hyaluronic acid.

According to one embodiment, the pharmaceutically acceptable carrier is a solid carrier or excipient. According to another embodiment, the pharmaceutically acceptable carrier is a gel-phase carrier or excipient. Examples of carriers or excipients include, but are not limited to, calcium carbonate, calcium phosphate, various monomeric and polymeric sugars (including without limitation hyaluronic acid), starches, cellulose derivatives, gelatin, and polymers. An exemplary carrier can also include saline vehicle, e.g. hydroxyl propyl methyl cellulose (HPMC) in phosphate buffered saline (PBS).

According to some embodiments, the pharmaceutically acceptable carrier imparts stickiness to the composition. According to one embodiment, the pharmaceutically acceptable carrier comprises hyaluronic acid. According to some embodiments, the pharmaceutically acceptable carrier comprises 0 % to 5 % hyaluronic acid. According to one embodiment, the pharmaceutically acceptable carrier comprises less than 0.05 % hyaluronic acid. According to another embodiment, the pharmaceutically acceptable carrier comprises less than 0.1 % hyaluronic acid. According to another embodiment, the pharmaceutically acceptable carrier comprises less than 0.2 % hyaluronic acid. According to another embodiment, the pharmaceutically acceptable carrier comprises less than 0.3 % hyaluronic acid. According to another embodiment, the pharmaceutically acceptable carrier comprises less than 0.4 % hyaluronic acid. According to another embodiment, the pharmaceutically acceptable carrier comprises less than 0.5 % hyaluronic acid. According to another embodiment, the pharmaceutically acceptable carrier comprises less than 0.6 % hyaluronic acid. According to another embodiment, the pharmaceutically acceptable carrier comprises less than 0.7 % hyaluronic acid. According to another embodiment, the pharmaceutically acceptable carrier comprises less than 0.8 % hyaluronic acid. According to another embodiment, the pharmaceutically acceptable carrier comprises less than 0.9 % hyaluronic acid. According to another embodiment, the pharmaceutically acceptable carrier comprises less than 1.0 % hyaluronic acid. According to another embodiment, the pharmaceutically acceptable carrier comprises less than 1.1 % hyaluronic acid. According to another embodiment, the pharmaceutically acceptable carrier comprises less than 1.2 % hyaluronic acid. According to another embodiment, the pharmaceutically acceptable carrier comprises less than 1.3 % hyaluronic acid. According to another embodiment, the pharmaceutically acceptable carrier comprises less than 1.4 % hyaluronic acid. According to another embodiment, the pharmaceutically acceptable carrier comprises less than 1.5 % hyaluronic acid. According to another embodiment, the pharmaceutically acceptable carrier comprises less than 1.6 % hyaluronic acid. According to another embodiment, the pharmaceutically acceptable carrier comprises less than 1.7 % hyaluronic acid. According to another embodiment, the pharmaceutically acceptable carrier comprises less than 1.8 % hyaluronic acid. According to another embodiment, the pharmaceutically acceptable carrier comprises less than 1.9 % hyaluronic acid. According to another embodiment, the pharmaceutically acceptable carrier comprises less than 2.0 % hyaluronic acid. According to another embodiment, the pharmaceutically acceptable carrier comprises less than 2.1 % hyaluronic acid. According to another embodiment, the pharmaceutically acceptable carrier comprises less than 2.2 % hyaluronic acid. According to another embodiment, the pharmaceutically acceptable carrier comprises less than 2.3% hyaluronic acid. According to another embodiment, the pharmaceutically acceptable carrier comprises less than 2.4 % hyaluronic acid. According to another embodiment, the pharmaceutically acceptable carrier comprises less than 2.5 % hyaluronic acid. According to another embodiment, the pharmaceutically acceptable carrier comprises less than 2.6 % hyaluronic acid. According to another embodiment, the pharmaceutically acceptable carrier comprises less than 2.7 % hyaluronic acid. According to another embodiment, the pharmaceutically acceptable carrier comprises less than 2.8 % hyaluronic acid. According to another embodiment, the pharmaceutically acceptable carrier comprises less than 2.9 % hyaluronic acid. According to another embodiment, the pharmaceutically acceptable carrier comprises less than 3.0 % hyaluronic acid. According to another embodiment, the pharmaceutically acceptable carrier comprises less than 3.5 % hyaluronic acid. According to another embodiment, the pharmaceutically acceptable carrier comprises less than 4.0 % hyaluronic acid. According to another embodiment, the pharmaceutically acceptable carrier comprises less than 4.5 % hyaluronic acid. According to another embodiment, the pharmaceutically acceptable carrier comprises less than 5.0 % hyaluronic acid.

In some embodiments, the pharmaceutically acceptable carrier includes, but is not limited to, a gel, slow-release solid or semisolid compound, optionally as a sustained release gel. In some such embodiments, the voltage-gated calcium channel blocker is embedded into the pharmaceutically acceptable carrier. In some embodiments, the voltage-gated calcium channel blocker is coated on the pharmaceutically acceptable carrier. The coating can be of any desired material, preferably a polymer or mixture of different polymers. Optionally, the polymer can be utilized during the granulation stage to form a matrix with the active ingredient so as to obtain a desired release pattern of the active ingredient. The gel, slow-release solid or semisolid compound is capable of releasing the active agent over a desired period of time. The gel, slow-release solid or semisolid compound can be implanted in a tissue within human brain, for example, but not limited to, in close proximity to a blood vessel, such as a cerebral artery.

According to another embodiment, the pharmaceutically acceptable carrier comprises a slow-release solid compound. According to one such embodiment, the voltage-gated calcium channel blocker is embedded in the slow-release solid compound or coated on the slow-release solid compound. According to yet another embodiment, the pharmaceutically acceptable carrier comprises a slow-release microparticle containing the voltage-gated calcium channel blocker.

According to another embodiment, the pharmaceutically acceptable carrier is a gel compound, such as a biodegradable hydrogel.

*Microparticle Formulation*

According to some embodiments, the voltage-gated calcium channel blocker is provided in the form of a particle. The term "particle" as used herein refers to nano or microparticles (or in some instances larger) that may contain in whole or in part the calcium channel blocker. According to some embodiments, the microparticulate formulation comprises a plurality of microparticles impregnated with the voltage-gated calcium channel blocker. According to one embodiment, the voltage-gated calcium channel blocker is contained within the core of the particle surrounded by a coating. According to another embodiment, the voltage-gated calcium channel blocker is dispersed throughout the surface of the particle. According to another embodiment, the voltage-gated calcium channel blocker is disposed on or in the microparticle. According to another embodiment, the voltage-gated calcium channel blocker is disposed throughout the surface of the microparticle. According to another embodiment, the voltage-gated calcium channel blocker is adsorbed into the particle.

According to some such embodiments, the microparticles are of uniform size distribution. According to some embodiments, the uniform distribution of microparticle size is achieved by a homogenization process to form a uniform emulsion comprising microparticles. According to some such embodiments, each microparticle comprises a matrix. According to some embodiments, the matrix comprises the voltage gated calcium channel blocker.

According to some embodiments, the pharmaceutical composition is flowable. According to some embodiments, the microparticulate formulation component of the pharmaceutical composition is flowable.

According to some embodiments, the particle is selected from the group consisting of a zero order release, first order release, second order release, delayed release, sustained release, immediate release, and a combination thereof. The particle can include, in addition to therapeutic agent(s), any of those materials routinely used in the art of pharmacy and medicine, including, but not limited to, erodible, nonerodible, biodegradable, or nonbiodegradable material or combinations thereof

According to some embodiments, the particle is a microcapsule that contains the voltage-gated calcium channel blocker in a solution or in a semi-solid state. According to some embodiments, the particle is a microparticle that contains the voltage-gated calcium channel blocker, in whole or in part. According to some embodiments, the particle is a nanoparticle that contains the voltage-gated calcium channel blocker, in whole or in part. According to some embodiments, the particles can be of virtually any shape.

According to some embodiments, the particle size is between about 25 $\mu$m to about 100 $\mu$m. According to some embodiments, the particle size is between about 30 $\mu$m to about 80 $\mu$m. According to one embodiment, the particle size is at least about 25 $\mu$m. According to another embodiment, the particle size is at least about 30 $\mu$m. According to another embodiment, the particle size is at least about 35 $\mu$m. According to another embodiment, the particle size is at least about 40 $\mu$m. According to another embodiment, the particle size is at least about 45 $\mu$m. According to another embodiment, the particle size is at least about 50 $\mu$m. According to another embodiment, the particle size is at least about 55 $\mu$m. According to another embodiment, the particle size is at least about 60 $\mu$m. According to another embodiment, the particle size is at least about 65 $\mu$m. According to another embodiment, the particle size is at least about 70 $\mu$m. According to another embodiment, the particle size is at least about 75 $\mu$m. According to another embodiment, the particle size is at least about 80 $\mu$m. According to another embodiment, the particle size is at least about 85 $\mu$m. According to another embodiment, the particle size is at least about 90 $\mu$m. According to another embodiment, the particle size is at least about 95 $\mu$m. According to another embodiment, the particle size is at least about 100 $\mu$m.

According to another embodiment, the voltage gated calcium channel blocker can be provided in strings. The strings can contain the voltage-gated calcium channel blocker in a core surrounded by a coating, or the voltage-gated calcium channel blocker can be dispersed throughout the string, or therapeutic agent(s) may be absorbed into the string. The string can be of any order release kinetics, including zero order release, first order release, second order release, delayed release, sustained release, immediate release, etc., and any combination thereof. The string can include, in addition to therapeutic agent(s), any of those materials routinely used in the art of pharmacy and medicine, including, but not limited to, erodible, nonerodible, biodegradable, or nonbiodegradable material or combinations thereof.

According to another embodiment, the voltage-gated calcium channel blocker can be provided in at least one sheet. The sheet can contain the voltage-gated calcium channel blocker and at least one additional therapeutic agent in a core surrounded by a coating, or the voltage-gated calcium channel blocker and at least one additional therapeutic agent can be dispersed throughout the sheet, or the voltage-gated calcium channel blocker can be absorbed into the sheet. The sheet can be of any order release kinetics, including zero order release, first order release, second order release, delayed release, sustained release, immediate release, etc., and any combination thereof. The sheet can include, in addition to the voltage-gated calcium channel blocker and at least one additional therapeutic agent, any of those materials routinely used in the art of pharmacy and medicine, including, but not limited to, erodible, nonerodible, biodegradable, or nonbiodegradable material or combinations thereof.

According to some embodiments, the pharmaceutical composition further comprises a preservative agent. According to some such embodiments, the pharmaceutical composition is presented in a unit dosage form. Exemplary unit dosage forms include, but arc not limited to, ampoules or multi-dose containers.

According to some embodiments, the microparticulate formulation comprises a suspension of microparticles. According to some embodiments, the pharmaceutical composition further comprises at least one of a suspending agent, a stabilizing agent and a dispersing agent. According to some such embodiments, the pharmaceutical composition is presented as a suspension. According to some such embodiments, the pharmaceutical composition is presented as a solution. According to some such embodiments, the pharmaceutical composition is presented as an emulsion.

According to some embodiments, a formulation of the pharmaceutical composition comprises an aqueous solution of the voltage-gated calcium channel blocker in water-soluble form. According to some embodiments, the formulation of the pharmaceutical composition comprises an oily suspension of the voltage-gated calcium channel blocker. Oily suspension of the voltage-gated calcium channel blocker can be prepared using suitable lipophilic solvents. Exemplary lipophilic solvents or vehicles include, but are not limited to, fatty oils such as sesame oil, or synthetic fatty acid esters, such as ethyl oleate or triglycerides. According to some embodiments, the formulation of the pharmaceutical composition comprises an aqueous suspension of the volgate-gated calcium channel blocker. Aqueous injection suspensions can contain substances which increase the viscosity of the suspension, such as sodium carboxymethyl cellulose, sorbitol, or dextran. Optionally, the suspension can also contain suitable stabilizers or agents which increase the solubility of the compounds to allow for the preparation of highly concentrated solutions. Alternatively, the volgate-gated calcium channel blocker can be in powder form for constitution with a suitable vehicle, e.g., sterile pyrogen-free water, before use.

Suitable liquid or solid pharmaceutical preparations include, for example, microencapsulated dosage forms, and if appropriate, with one or more excipients, encochleated, coated onto microscopic gold particles, contained in liposomes, pellets for implantation into the tissue, or dried onto an object to be rubbed into the tissue. As used herein, the term "microencapsulation" refers to a process in which very tiny droplets or particles are surrounded or coated with a continuous film of biocompatible, biodegradable, polymeric or non-polymeric material to produce solid structures including, but not limited to, nonpareils, pellets, crystals, agglomerates, microspheres, or nanoparticlcs. Such pharmaceutical compositions also can be in the form of granules, beads, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, or solubilizers are customarily used as described above. The pharmaceutical compositions are suitable for use in a variety of drug delivery systems. For a brief review of methods for drug delivery, see Langer (1990) Science 249, 1527-1533, which is incorporated herein by reference.

## *Microencapsulation Process*

Examples of microencapsulation processes and products; methods for the production of emulsion-based microparticles; emulsion-based microparticles and methods for the production thereof; solvent extraction microencapsulation with tunable extraction rates; microencapsulation process with solvent and salt; a continuous double emulsion process for making microparticles; drying methods for tuning microparticle properties, controlled release systems from polymer blends; polymer mixtures comprising polymers having different non-repeating units and methods for making and using the same; and an emulsion based process for preparing microparticles and workhead assembly for use with same are disclosed and described in U.S. Patent No. 5, 407,609 (entitled Microencapsulation Process and Products Thereof), U.S. Application No. 10/553,003 (entitled Method for the production of emulsion-based microparticles), U.S. Application No. 11/799,700 (entitled Emulsion-based microparticles and methods for the production thereof), U.S. Application No. 12/557,946 (entitled Solvent Extraction Microencapsulation With Tunable Extraction Rates), U.S. Application No. 12/779,138 (entitled Hyaluronic Acid (HA) Injection Vehicle), U.S. Application No. 12/562,455 entitled Microencapsulation Process With Solvent And Salt), U.S. Application No. 12/338,488 (entitled Process For Preparing Microparticles Having A Low Residual Solvent Volume); U.S. Application No. 12/692,027 (entitled Controlled Release Systems From Polymer Blends); U.S. Application No. 12/692,020 (entitled Polymer Mixtures Comprising Polymers Having Different Non-Repeating Units And Methods For Making And Using Same); U.S. Application No. 10/565,401 (entitled "Controlled release compositions"); U.S. Application No. 12/692,029 (entitled "Drying Methods for Tuning Microparticle Properties); U.S. Application No. 12/968,708 (entitled "Emulsion Based Process for Preparing Microparticles and Workhead for Use with Same); and U.S. Application No. 13/074,542 (entitled Composition and Methods for Improved Retention of a Pharmaceutical Composition at a Local Administration Site") The contents of each of these are incorporated herein by reference in its entirety.

According to some embodiments, delivery of the voltage-gated calcium channel blocker using microparticle technology involves bioresorbable, polymeric particles that encapsulate the voltage-gated calcium channel blocker and at least one additional therapeutic agent.

According to one embodiment, the microparticle formulation comprises a polymer matrix, wherein the voltage-gated calcium channel blocker is impregnated in the polymer matrix. According to one embodiment, the polymer is a slow release polymer. According to one embodiment, the polymer is poly (D, L-Lactide-co-glycolide). According to another embodiment, the polymer is poly(orthoester). According to another embodiment, the polymer is poly(anhydride). According to another embodiment, the polymer is polylactide-polyglycolide.

Both non-biodegradable and biodegradable polymeric materials can be used in the manufacture of particles for delivering the voltage-gated calcium channel blocker. Such polymers can be natural or synthetic polymers. The polymer is selected based on the period of time over which release is desired. Bioadhesive polymers of particular interest include, but are not limited to, bioerodible hydrogels as described by Sawhney et al in Macromolecules (1993) 26, 581-587, the teachings of which are incorporated herein. Exemplary bioerodible hydrogels include, but are not limited to, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate). According to one embodiment, the bioadhesive polymer is hyaluronic acid. In some such embodiments, the bioadhesive polymer include less than about 2.3 % of hyaluronic acid.

According to some embodiments, the pharmaceutical composition is formulated for parenteral injection, surgical implantation, or a combination thereof. According to some such embodiments, the pharmaceutical composition is in the form of a pharmaceutically acceptable sterile aqueous or nonaqueous solution, dispersion, suspension or emulsion or a sterile powder for reconstitution into a sterile injectable solution or dispersion. Examples of suitable aqueous and nonaqueous carriers, diluents, solvents or vehicles include, but are not limited to, water, ethanol, dichloromethane, acetonitrile, ethyl acetate, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants. Suspensions can further contain suspending agents, as, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

According to some embodiments, the pharmaceutical composition is formulated in an injectable depot form. Injectable depot forms are made by forming microencapsulated matrices of the voltage gated calcium channel blocker in a biodegradable polymer. Depending upon the ratio of drug to polymer and the nature of the particular polymer employed, the rate of drug release may be controlled. Such long acting formulations can be formulated with suitable polymeric or hydrophobic materials (for example as an emulsion in an acceptable oil) or ion exchange resins, or as sparingly soluble derivatives, for example, as a sparingly soluble salt. Examples of biodegradable polymers include, but are not limited to, polylactide-polyglycolide, poly(orthoesters) and poly(anhydrides). Depot injectable formulations are also prepared by entrapping the drug in liposomes or microemulsions which are compatible with body tissues.

According to some embodiments, the voltage-gated calcium channel blocker is impregnated in or on a polyglycolide (PGA) matrix. PGA is a linear aliphatic polyester developed for use in sutures. Studies have reported PGA copolymers formed with trimethylene carbonate, polylactic acid (PLA), and polycaprolactone. Some of these copolymers may be formulated as microparticles for sustained drug release.

According to some embodiments, the voltage-gated calcium channel blocker is impregnated in or on a polyester-polyethylene glycol matrix. Polyester-polyethylene glycol compounds can be synthesized; these are soft and may be used for drug delivery.

According to some embodiments, the voltage-gated calcium channel blocker is impregnated in or on a poly (amino)-derived biopolymer matrix. Poly (amino)-derived biopolymers can include, but are not limited to, those containing lactic acid and lysine as the aliphatic diamine (see, for example, U.S. Patent 5,399,665), and tyrosine-derived polycarbonates and polyacrylates. Modifications of polycarbonates may alter the length of the alkyl chain of the ester (ethyl to octyl), while modifications of polyarylates may further include altering the length of the alkyl chain of the diacid (for example, succinic to sebasic), which allows for a large permutation of polymers and great flexibility in polymer properties.

According to some embodiments, the voltage-gated calcium channel blocker is impregnated in or on a polyanhydride matrix. Polyanhydrides are prepared by the dehydration of two diacid molecules by melt polymerization (see, for example, U.S. Patent 4,757,128). These polymers degrade by surface erosion (as compared to polyesters that degrade by bulk erosion). The release of the drug can be controlled by the hydrophilicity of the monomers chosen.

According to some embodiments, the voltage-gated calcium channel blocker is impregnated in or on a photopolymerizable biopolymer matrix. Photopolymerizable biopolymers include, but are not limited to, lactic acid/polyethylene glycol/acrylate copolymers.

According to some embodiments, the voltage-gated calcium channel blocker is impregnated in or on a hydrogel matrix. The term "hydrogel" refers to a substance resulting in a solid, semisolid, pseudoplastic or plastic structure containing a necessary aqueous component to produce a gelatinous or jelly-like mass. Hydrogels generally comprise a variety of polymers, including hydrophilic polymers, acrylic acid, acrylamide and 2-hydroxyethylmethacrylate (HEMA).

According to some embodiments, the voltage-gated calcium channel blocker is impregnated in or on a naturally-occurring biopolymer matrix. Naturally-occurring biopolymers include, but are not limited to, protein polymers, collagen, polysaccharides, and photopolymerizable compounds.

According to some embodiments, the voltage-gated calcium channel blocker is impregnated in or on a protein polymer matrix. Protein polymers have been synthesized from self-assembling protein polymers such as, for example, silk fibroin, elastin, collagen, and combinations thereof.

According to some embodiments, the voltage-gated calcium channel blocker is impregnated in or on a naturally-occurring polysaccharide matrix. Naturally-occurring polysaccharides include, but are not limited to, chitin and its derivatives, hyaluronic acid, dextran and cellulosics (which generally are not biodegradable without modification), and sucrose acetate isobutyrate (SAIB).

According to some embodiments, the voltage-gated calcium channel blocker is impregnated in or on a chitin matrix. Chitin is composed predominantly of 2-acetamido-2-deoxy-D-glucose groups and is found in yeasts, fungi and marine invertebrates (shrimp, crustaceous) where it is a principal component of the exoskeleton. Chitin is not water soluble and the deacetylated chitin, chitosan, only is soluble in acidic solutions (such as, for example, acetic acid). Studies have reported chitin derivatives that are water soluble, very high molecular weight (greater than 2 million daltons), viscoelastic, non-toxic, biocompatible and capable of crosslinking with peroxides, gluteraldehyde, glyoxal and other aldehydes and carbodiamides, to form gels.

According to some embodiments, the voltage-gated calcium channel blocker is impregnated in or on a hyaluronic acid (HA) matrix. Hyaluronic acid (HA), which is composed of alternating glucuronidic and glucosaminidic bonds and is found in mammalian vitreous humor, extracellular matrix of the brain, synovial fluid, umbilical cords and rooster combs from which it is isolated and purified, also can be produced by fermentation processes.

According to some embodiments, the pharmaceutical composition further comprises an adjuvant. Exemplary adjuvants include, but are not limited to, preservative agents, wetting agents, emulsifying agents, and dispersing agents. Prevention of the action of microorganisms can be ensured by various antibacterial and antifungal agents, for example, parabens, chlorobutanol, phenol, sorbic acid, and the like. Isotonic agents, for example, sugars, sodium chloride and the like, can also be included. Prolonged absorption of the injectable pharmaceutical form can be brought about by the use of agents delaying absorption, for example, aluminum monostearate and gelatin.

The formulations can be sterilized, for example, by terminal gamma irradiation, filtration through a bacterial-retaining filter or by incorporating sterilizing agents in the form of sterile solid compositions that may be dissolved or dispersed in sterile water or other sterile injectable medium just prior to use. Injectable preparations, for example, sterile injectable aqueous or oleaginous suspensions may be formulated according to the known art using suitable dispersing or wetting agents and suspending agents. The sterile injectable preparation also may be a sterile injectable solution, suspension or emulsion in a nontoxic, parenterally acceptable diluent or solvent such as a solution in 1,3-butanediol, dichloromethane, ethyl acetate, acetonitrile, etc. Among the acceptable vehicles and solvents that may be employed are water, Ringer's solution, U.S.P. and isotonic sodium chloride solution. In addition, sterile, fixed oils conventionally are employed or as a solvent or suspending medium. For this purpose any bland fixed oil may be employed including synthetic mono- or diglycerides. In addition, fatty acids such as oleic acid are used in the preparation of injectables.

Formulations for parenteral (including but not limited to, subcutaneous, intradermal, intramuscular, intravenous, intraarterial, intrathecal, intraventricular and intraarticular) administration include aqueous and non-aqueous sterile injection solutions that can contain anti-oxidants, buffers, bacteriostats and solutes, which render the formulation isotonic with the blood of the intended recipient; and aqueous and non-aqueous sterile suspensions, which can include suspending agents and thickening agents.

According to another embodiment, the pharmaceutical composition is formulated by conjugating the voltage-gated calcium channel blocker to a polymer that enhances aqueous solubility. Examples of suitable polymers include but are not limited to polyethylene glycol, poly-(d-glutamic acid), poly-(1-glutamic acid), poly-(1-glutamic acid), poly-(d-aspartic acid), poly-(1-aspartic acid), poly-(1-aspartic acid) and copolymers thereof. Polyglutamic acids having molecular weights between about 5,000 to about 100,000, with molecular weights between about 20,000 and about 80,000 may be used and with molecular weights between about 30,000 and about 60,000 may also be used. The polymer is conjugated via an ester linkage to one or more hydroxyls of an inventive epothilone using a protocol as essentially described by U.S. Pat. No. 5,977,163 which is incorporated herein by reference. Particular conjugation sites include the hydroxyl off carbon-21 in the case of 21-hydroxy-derivatives of the present invention. Other conjugation sites include, but are not limited, to the hydroxyl off carbon 3 and/or the hydroxyl off carbon 7.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

**II. Delivery Systems**

In another aspect, the present invention provides a semisolid (meaning having a somewhat firm consistency) microparticulatc delivery system for treating a delayed complication associated with a brain injury in a mammal in need thereof, wherein the brain injury includes interruption of at least one cerebral artery, the system comprising (a) a pharmaceutical composition comprising (i) a microparticulate formulation of a voltage-gated calcium channel blocker; and optionally (ii) a pharmaceutically acceptable carrier; and (b) a means for administering a therapeutic amount of the pharmaceutical composition, wherein the therapeutic amount is effective in reducing signs or symptoms of the at least one delayed complication associated with the brain injury.

According to some embodiments, the semisolid multiparticulate delivery system can prevent or reduce the incidence or severity of at least one delayed complication associated with a brain injury in a mammal in need thereof, wherein the brain injury includes interruption of at least one cerebral artery. According to some such embodiments, the at least one delayed complication is selected from the group consisting of a delayed cerebral ischemia (DCI), an intracerebral hematoma, an intraventricular hemorrhage, a fever, an angiographic vasospasm, a microthromboembolus, a cortical spreading ischemia (CSI), a behavioral deficit, a neurological deficit, a cerebral infarction, neuronal cell death, or a combination thereof. According to one embodiment, the at least one delayed complication is a delayed cerebral ischemia (DCI). According to another embodiment, the at least one delayed complication is an intracerebral hematoma. According to another embodiment, the at least one delayed complication is an intraventricular hemorrhage. According to another embodiment, the at least one delayed complication is a fever. According to another embodiment, the at least one delayed complication is an angiographic vasospasm. According to another embodiment, the at least one delayed complication is a cortical spreading ischemia (CSI). According to another embodiment, the at least one delayed complication is a plurality of microthromboemboli. According to another embodiment, the at least one delayed complication is a behavioral deficit. According to another embodiment, the at least one delayed complication is a neurological deficit. According to another embodiment, the at least one delayed complication is a cerebral infarction. According to another embodiment, the at least one delayed complication is neuronal cell death.

According to some embodiments, the brain injury is a result of an underlying condition. Exemplary underlying conditions include, but are not limited to, aneurysm, sudden traumatic head injury, subarachnoid hemorrhage (SAH), etc. According to some such embodiments, the underlying condition is selected from the group consisting of aneurysm, sudden traumatic head injury, subarachnoid hemorrhage (SAH), or a combination thereof. According to one embodiment, the underlying condition is an aneurysm. According to another embodiment, the underlying condition is a sudden traumatic head injury. According to another embodiment, the underlying condition is a subarachnoid hemorrhage (SAH).

According to some embodiments, the pharmaceutical composition is administered by parenteral injection or surgical implantation.

According to some embodiments, the semisolid multiparticulate delivery system comprises a cannula or catheter through which the pharmaceutical composition is delivered, wherein the catheter is inserted into a site of delivery in the mammal. According to one embodiment, the site of delivery is in a cerebral ventricle. According to some embodiments, the cerebral ventricle is selected from the group consisting of a lateral ventricle, a third ventricle, a fourth ventricle, or a combination thereof. According to one embodiment, the site of delivery is in subarachnoid space. According to one embodiment, the site of delivery is in close proximity to the brain injury. According to another embodiment, the site of delivery is in close proximity to a blood vessel that is affected by the brain injury. According to one embodiment, the blood vessel is at least one cerebral artery. According to another embodiment, the at least one cerebral artery is affected by the brain injury.

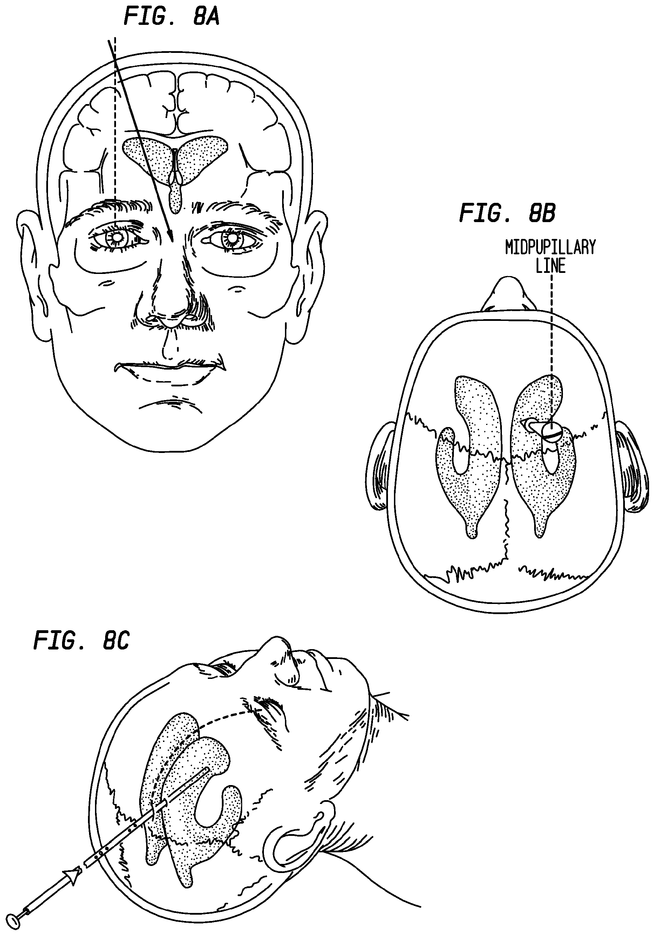

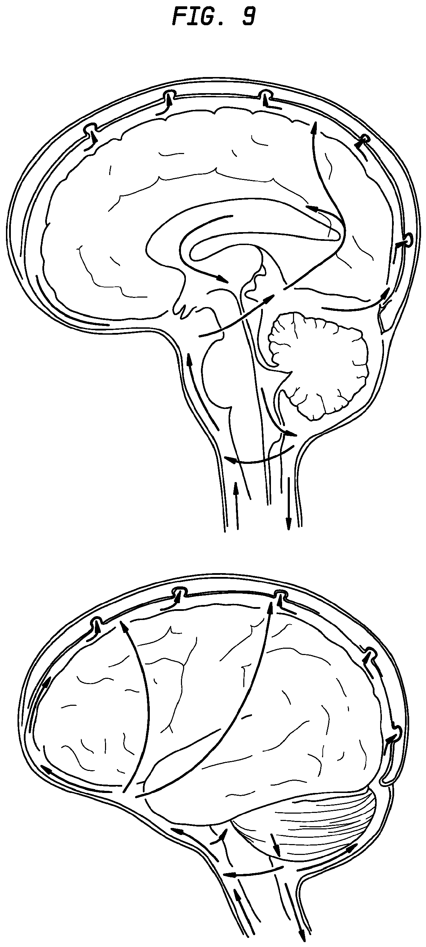

The cerebral ventricles may be cannulated or catheterized as is well-known in the art and as described in various neurosurgical textbooks. This is called insertion of a ventricular catheter or drain or ventriculostomy. A hole of varying size can be drilled in the skull and the outer dura mater covering the brain incised. The pia mater is incised and a catheter (a hollow tube generally made of silicone elastomer of some other biocompatible, nonabsorbable compound) is inserted through the brain into the ventricle of choice. This usually is the lateral ventricle but any ventricle could be catheterized. The catheter can be used to monitor the pressure inside the head, to drain CSF or to administer substances into the CSF. Figure 8 shows an exemplary view of delivery of the pharmaceutical composition comprising the voltage-gated calcium channel blocker microparticle suspension to the cerebral ventricles through an intraventricular catheter. Figure 9 shows a schematic depicting application of the pharmaceutical composition comprising a voltage-gated calcium channel blocker in or on microparticles being carried by CSF flow to each of the arteries of the subarachnoid space.

According to some embodiments, the site of delivery is within 10 mm, less than 10 mm, less than 9.9 mm, less than 9.8 mm, less than 9.7 mm, less than 9.6 mm, less than 9.5 mm, less than 9.4 mm, less than 9.3 mm, less than 9.2 mm, less than 9.1 mm, less than 9.0 mm, less than 8.9 mm, less than 8.8 mm, less than 8.7 mm, less than 8.6 mm, less than 8.5 mm, less than 8.4 mm, less than 8.3 mm, less than 8.2 mm, less than 8.1 mm, less than 8.0 mm, less than 7.9 mm, less than 7.8 mm, less than 7.7 mm, less than 7.6 mm, less than 7.5 mm, less than 7.4 mm, less than 7.3 mm, less than 7.2 mm, less than 7.1 mm, less than 7.0 mm, less than 6.9 mm, less than 6.8 mm, less than 6.7 mm, less than 6.6 mm, less than 6.5 mm, less than 6.4 mm, less than 6.3 mm, less than 6.2 mm, less than 6.1 mm, less than 6.0 mm, less than 5.9 mm, less than 5.8 mm, less than 5.7 mm, less than 5.6 mm, less than 5.5 mm, less than 5.4 mm, less than 5.3 mm, less than 5.2 mm, less than 5.1 mm, less than 5.0 mm, less than 4.9 mm, less than 4.8 mm, less than 4.7 mm, less than 4.6 mm, less than 4.5 mm, less than 4.4 mm, less than 4.3 mm, less than 4.2 mm, less than 4.1 mm, less than 4.0 mm, less than 3.9 mm, less than 3.8 mm, less than 3.7 mm, less than 3.6 mm, less than 3.5 mm, less than 3.4 mm, less than 3.3 mm, less than 3.2 mm, less than 3.1 mm, less than 3.0 mm, less than 2.9 mm, less than 2.8 mm, less than 2.7 mm, less than 2.6 mm, less than 2.5 mm, less than 2.4 mm, less than 2.3 mm, less than 2.2 mm, less than 2.1 mm, less than 2.0 mm, less than 1.9 mm, less than 1.8 mm, less than 1.7 mm, less than 1.6 mm, less than 1.5 mm, less than 1.4 mm, less than 1.3 mm, less than 1.2 mm, less than 1.1 mm, less than 1.0 mm, less than 0.9 mm, less than 0.8 mm, less than 0.7 mm, less than 0.6 mm, less than 0.5 mm, less than 0.4 mm, less than 0.3 mm, less than 0.2 mm, less than 0.1 mm, less than 0.09 mm, less than 0.08 mm, less than 0.07 mm, less than 0.06 mm, less than 0.05 mm, less than 0.04 mm, less than 0.03 mm, less than 0.02 mm, less than 0.01 mm, less than 0.009 mm, less than 0.008 mm, less than 0.007 mm, less than 0.006 mm, less than 0.005 mm, less than 0.004 mm, less than 0.003 mm, less than 0.002 mm, less than 0.001 mm of the brain injury.

According to some embodiments, the site of delivery is within 10 mm, less than 10 mm, less than 9.9 mm, less than 9.8 mm, less than 9.7 mm, less than 9.6 mm, less than 9.5 mm, less than 9.4 mm, less than 9.3 mm, less than 9.2 mm, less than 9.1 mm, less than 9.0 mm, less than 8.9 mm, less than 8.8 mm, less than 8.7 mm, less than 8.6 mm, less than 8.5 mm, less than 8.4 mm, less than 8.3 mm, less than 8.2 mm, less than 8.1 mm, less than 8.0 mm, less than 7.9 mm, less than 7.8 mm, less than 7.7 mm, less than 7.6 mm, less than 7.5 mm, less than 7.4 mm, less than 7.3 mm, less than 7.2 mm, less than 7.1 mm, less than 7.0 mm, less than 6.9 mm, less than 6.8 mm, less than 6.7 mm, less than 6.6 mm, less than 6.5 mm, less than 6.4 mm, less than 6.3 mm, less than 6.2 mm, less than 6.1 mm, less than 6.0 mm, less than 5.9 mm, less than 5.8 mm, less than 5.7 mm, less than 5.6 mm, less than 5.5 mm, less than 5.4 mm, less than 5.3 mm, less than 5.2 mm, less than 5.1 mm, less than 5.0 mm, less than 4.9 mm, less than 4.8 mm, less than 4.7 mm, less than 4.6 mm, less than 4.5 mm, less than 4.4 mm, less than 4.3 mm, less than 4.2 mm, less than 4.1 mm, less than 4.0 mm, less than 3.9 mm, less than 3.8 mm, less than 3.7 mm, less than 3.6 mm, less than 3.5 mm, less than 3.4 mm, less than 3.3 mm, less than 3.2 mm, less than 3.1 mm, less than 3.0 mm, less than 2.9 mm, less than 2.8 mm, less than 2.7 mm, less than 2.6 mm, less than 2.5 mm, less than 2.4 mm, less than 2.3 mm, less than 2.2 mm, less than 2.1 mm, less than 2.0 mm, less than 1.9 mm, less than 1.8 mm, less than 1.7 mm, less than 1.6 mm, less than 1.5 mm, less than 1.4 mm, less than 1.3 mm, less than 1.2 mm, less than 1.1 mm, less than 1.0 mm, less than 0.9 mm, less than 0.8 mm, less than 0.7 mm, less than 0.6 mm, less than 0.5 mm, less than 0.4 mm, less than 0.3 mm, less than 0.2 mm, less than 0.1 mm, less than 0.09 mm, less than 0.08 mm, less than 0.07 mm, less than 0.06 mm, less than 0.05 mm, less than 0.04 mm, less than 0.03 mm, less than 0.02 mm, less than 0.01 mm, less than 0.009 mm, less than 0.008 mm, less than 0.007 mm, less than 0.006 mm, less than 0.005 mm, less than 0.004 mm, less than 0.003 mm, less than 0.002 mm, less than 0.001 mm of the at least one cerebral artery affected by the brain injury.

According to some embodiments, the pharmaceutical composition comprising the voltage-gated calcium channel blocker can be delivered to the cerebral ventricles and then be carried by the flow of cerebrospinal fluid (CSF) to at least one cerebral artery of the subarachnoid space to effectuate a localized release of a therapeutically effective amount of the voltage-gated calcium channel blocker, thereby treating or reducing the incidence or severity of the at least one delayed complication caused by an underlying disease, disorder, condition or a sudden brain injury, and improving prognosis. According to one embodiment, the site of delivery is into at least one cerebral ventricle. Because the voltage-gated calcium channel blocker is delivered locally to the site of brain injury, the dosage required to treat or reduce the severity of the at least one delayed complication is lower circumventing unwanted side effects associated with systemic delivery of higher doses such as hypotension.

According to one embodiment, the pharmaceutical composition comprising the voltage-gated calcium channel blocker can be delivered by inserting a catheter into the ventricle and injecting the pharmaceutical composition through the catheter such that the pharmaceutical composition emanates from the end of the catheter locally into the ventricle.

According to another embodiment, the pharmaceutical composition is given as a single bolus injection. According to another embodiment, the injection is repeated after a pre-determined time period. According to some such embodiments, the pre-determined time period could range from 1 minute or more to 10 days or more. For example, a repeat injection can be given if monitoring of the patient showed that the patient still had evidence of angiographic vasospasm and/or DCI. The CSF circulation could carry the pharmaceutical composition out of the ventricles into the subarachnoid space. The circulation of CSF is often slowed after SAH and the subarachnoid space contains blood clots. Thus, the pharmaceutical composition can become trapped in the blood clots and thereby, there would be localized release of the pharmacological agent(s) from the composition where it/they would exert a pharmacological effect in the adjacent arteries and brain.

More generally, the semisolid multiparticulate delivery system of the present invention provides a number of advantages over systemic administration either orally or by infusion. As an example, nimodipine now must be administered as a continuous intravenous infusion or orally as pills given every 2 to 4 hours. The concentration of the voltage-gated calcium channel blocker in the CSF locally where they exert their effect is higher and the plasma concentration is lower than when the e voltage-gated calcium channel blocker is administered orally or intravenously. This results in a localized pharmacological effect in the brain arteries and/or brain with less effect in the body. Side effects in the body, such as hypotension, are less likely to occur. Some pharmacological agents do not cross the blood-brain barrier well after systemic administration. Administration directly into a cerebral ventricle overcomes this problem. The total dose of the voltage-gated calcium channel blockers administered is much lower than that administered systemically so the risk of other and unknown side effects is lower.

According to some embodiments, the pharmaceutical composition comprising the voltage-gated calcium channel blocker is contained in a controlled release system. In order to prolong the effect of a drug, it often is desirable to slow the absorption of the drug. This can be accomplished by the use of a liquid suspension of crystalline or amorphous material with poor water solubility. The rate of absorption of the drug then depends upon its rate of dissolution which, in turn, may depend upon crystal size and crystalline form. For example, according to some embodiments, a SABER™ Delivery System comprising a high-viscosity base component, such as sucrose acetate isobutyrate (SAIB), is used to provide controlled release of the voltage-gated calcium channel blocker. (See U.S. Pat. No. 5,747,058 and U.S. Pat. No. 5,968,542, incorporated herein by reference). When the high viscosity SAIB is formulated with drug, biocompatible excipients and other additives, the resulting formulation is liquid enough to inject easily with standard syringes and needles. After injection of a SABER™ formulation, the excipients diffuse away, leaving a viscous depot.

According to some embodiments, the controlled release system is capable of releasing the voltage gated calcium channel blocker at the site of delivery within a half-life ranging from 1 day to 30 days. According to one embodiment, the controlled release system is capable of releasing the voltage gated calcium channel blocker at the site of delivery within a half-life of 1 day. According to another embodiment, the controlled release system is capable of releasing the voltage gated calcium channel blocker at the site of delivery within a half-life of 2 days. According to another embodiment, the controlled release system is capable of releasing the voltage gated calcium channel blocker at the site of delivery within a half-life of 3 days. According to another embodiment, the controlled release system is capable of releasing the voltage gated calcium channel blocker within a half-life of 4 days. According to another embodiment, the controlled release system is capable of releasing the voltage gated calcium channel blocker at the site of delivery within a half-life of 5 days. According to another embodiment, the controlled release system is capable of releasing the voltage gated calcium channel blocker at the site of delivery within a half-life of 6 days. According to another embodiment, the controlled release system is capable of releasing the voltage gated calcium channel blocker at the site of delivery within a half-life of 7 days. According to another embodiment, the controlled release system is capable of releasing the voltage gated calcium channel blocker at the site of delivery within a half-life of 8 days. According to another embodiment, the controlled release system is capable of releasing the voltage gated calcium channel blocker at the site of delivery within a half-life of 9 days. According to another embodiment, the controlled release system is capable of releasing the voltage gated calcium channel blocker at the site of delivery within a half-life of 10 days. According to another embodiment, the controlled release system is capable of releasing the voltage gated calcium channel blocker at the site of delivery within a half-life of 15 days. According to another embodiment, the controlled release system is capable of releasing the voltage gated calcium channel blocker at the site of delivery within a half-life of 30 days.

According to some embodiments, the controlled release system comprises a long term sustained release implant that can be particularly suitable for treatment of chronic conditions. The term "long-term" release, as used herein, means that the implant is constructed and arranged to deliver therapeutic levels of the active ingredient for at least 7 days, and preferably about 30 to about 60 days. Long-term sustained release implants are well-known to those of ordinary skill in the art and include some of the release systems described above.

According to another embodiment, the release of the voltage-gated calcium channel blocker at the site of delivery can produce a predominantly localized pharmacologic effect over a desired amount of time. According to one embodiment, the release of the voltage-gated calcium channel blocker at the site of delivery produces a predominantly localized pharmacologic effect for 1 day. According to one embodiment, the release of the voltage-gated calcium channel blocker at the site of delivery produces a predominantly localized pharmacologic effect for 2 days. According to one embodiment, the release of the voltage-gated calcium channel blocker at the site of delivery produces a predominantly localized pharmacologic effect for 3 days. According to one embodiment, the release of the voltage-gated calcium channel blocker at the site of delivery produces a predominantly localized pharmacologic effect for 4 days. According to one embodiment, the release of the voltage-gated calcium channel blocker at the site of delivery produces a predominantly localized pharmacologic effect for 5 days. According to one embodiment, the release of the voltage-gated calcium channel blocker at the site of delivery produces a predominantly localized pharmacologic effect for 6 days. According to one embodiment, the release of the voltage-gated calcium channel blocker at the site of delivery produces a predominantly localized pharmacologic effect for 7 days. According to one embodiment, the release of the voltage-gated calcium channel blocker at the site of delivery produces a predominantly localized pharmacologic effect for 8 days. According to one embodiment, the release of the voltage-gated calcium channel blocker at the site of delivery produces a predominantly localized pharmacologic effect for 15 days. According to one embodiment, the release of the voltage-gated calcium channel blocker at the site of delivery produces a predominantly localized pharmacologic effect for 30 days.

According to another embodiment, the release of the voltage-gated calcium channel blocker at the site of delivery produces a diffuse pharmacologic effect throughout the central nervous system (CNS) over a desired amount of time. According to another embodiment, the release of the voltage-gated calcium channel blocker can produce a diffuse pharmacologic effect throughout the central nervous system (CNS) for 1 day. According to another embodiment, the release of the voltage-gated calcium channel blocker can produce a diffuse pharmacologic effect throughout the central nervous system (CNS) for 2 days. According to another embodiment, the release of the voltage-gated calcium channel blocker can produce a diffuse pharmacologic effect throughout the central nervous system (CNS) for 3 days. According to another embodiment, the release of the voltage-gated calcium channel blocker can produce a diffuse pharmacologic effect throughout the central nervous system (CNS) for 4 days. According to another embodiment, the release of the voltage-gated calcium channel blocker can produce a diffuse pharmacologic effect throughout the central nervous system (CNS) for 5 days. According to another embodiment, the release of the voltage-gated calcium channel blocker can produce a diffuse pharmacologic effect throughout the central nervous system (CNS) for 6 days. According to another embodiment, the release of the voltage-gated calcium channel blocker can produce a diffuse pharmacologic effect throughout the central nervous system (CNS) for 7 days. According to another embodiment, the release of the voltage-gated calcium channel blocker can produce a diffuse pharmacologic effect for throughout the central nervous system (CNS) 8 days. According to another embodiment, the release of the voltage-gated calcium channel blocker can produce a diffuse pharmacologic effect throughout the central nervous system (CNS) for 15 days. According to another embodiment, the release of the voltage-gated calcium channel blocker can produce a diffuse pharmacologic effect for throughout the central nervous system (CNS) 30 days.

According to one embodiment, the localized pharmacologic effect at the site of delivery is a reduction of vasospasm such that internal diameter of the at least one cerebral artery affected by the brain injury is increased compared to a control. According to one embodiment, the pharmaceutical composition is effective to increase the internal diameter of the cerebral artery affected by the brain injury as compared to a control.

According to one embodiment, the diffuse pharmacologic effect is a reduction of vasospasm such that internal diameter of a blood vessel that is within at least 10 mm, at least 9.9 mm, at least 9.8 mm, at least 9.7 mm, at least 9.6 mm, at least 9.5 mm, at least 9.4 mm, at least 9.3 mm, at least 9.2 mm, at least 9.1 mm, at least 9.0 mm, at least 8.9 mm, at least 8.8 mm, at least 8.7 mm, at least 8.6 mm, at least 8.5 mm, at least 8.4 mm, at least 8.3 mm, at least 8.2 mm, at least 8.1 mm, at least 8.0 mm, at least 7.9 mm, at least 7.8 mm, at least 7.7 mm, at least 7.6 mm, at least 7.5 mm, at least 7.4 mm, at least 7.3 mm, at least 7.2 mm, at least 7.1 mm, at least 7.0 mm, at least 6.9 mm, at least 6.8 mm, at least 6.7 mm, at least 6.6 mm, at least 6.5 mm, at least 6.4 mm, at least 6.3 mm, at least 6.2 mm, at least 6.1 mm, at least 6.0 mm, at least 5.9 mm, at least 5.8 mm, at least 5.7 mm, at least 5.6 mm, at least 5.5 mm, at least 5.4 mm, at least 5.3 mm, at least 5.2 mm, at least 5.1 mm, at least 5.0 mm of the site of delivery is increased as compared to a control.

## MEANS OF ADMINISTRATION

According to one embodiment, the means for administering is (1) via surgical injection so that the formulation is placed at a site in close proximity to the cerebral artery affected by the brain injury, such that a therapeutic amount of the pharmaceutical composition is delivered in subarachnoid space.

According to another embodiment, the means for administering is (2) via a catheter locally into a cerebral ventricle so that the formulation is carried by CSF circulation to contact the cerebral artery affected by the brain injury, such that a therapeutic amount of the pharmaceutical composition is delivered in subarachnoid space.

## PHARMACEUTICAL COMPOSITION

### *Voltage-gated Calcium Channel Blocker*

According to some embodiments, the voltage-gated channel blocker is selected from the group consisting of L-type voltage-gated calcium channel blocker, N-type voltage-gated calcium channel blocker, P/Q-type voltage-gated calcium channel blocker, or a combination thereof.

Non-limiting examples of the voltage-gated calcium channel blocker that can be formulated into the composition include, but are not limited to, L-type voltage-gated calcium channel blocker, N-type voltage-gated calcium channel blocker, P/Q-type voltage-gated calcium channel blocker, or a combination thereof.

For example, L-type voltage-gated calcium channel blockers include, but are not limited to: dihydropyridine L-type blockers such as nisoldipine, nicardipine and nifedipine, AHF (such as 4aR,9aS)-(+)-4a-Amino-1,2,3,4,4a,9a-hexahydro-4aH-fluorene, HCl), isradipine (such as 4-(4-Benzofurazanyl)-1,-4-dihydro-2,6-dimethyl-3,5-pyridinedicarboxylic acid methyl 1-methhylethyl ester), calciseptine (such as isolated from (Dendroaspis polylepis ploylepis), H-Arg-Ile-Cys-Tyr-Ile-His-Lys-Ala-Ser-Leu-Pro-Arg-Ala-Thr-Lys-Thr-Cys-Val- Glu-Asn-Thr-Cys-Tyr-Lys-Met-Phe-Ile-Arg-Thr-Gln-Arg-Glu-Tyr-Ile-Ser-Glu-Arg-Gly-Cys-Gly-Cys-Pro-Thr-Ala-Met-Trp-Pro-Tyr-Gln-Thr-Glu-Cys-Cys-Lys-Gly-Asp-Arg-Cys-Asn-Lys-OH, Calcicludine (such as isolated from Dendroaspis angusticeps (eastern green mamba)),(H-Trp-Gln-Pro-Pro- Trp-Tyr-Cys-Lys-Glu-Pro-Val-Arg-Ile-Gly-Ser-Cys-Lys-Lys-Gln-Phe-Ser-Ser-Phe-Tyr- Phe-Lys-Trp-Thr-Ala-Lys-Lys-Cys-Leu-Pro-Phe-Leu-Phe-Ser-Gly¬Cys-Gly-Gly-Asn-Ala-Asn-Arg-Phe-Gln-Thr-Ile-Gly-Glu-Cys-Arg-Lys-Lys-Cys-Leu-Gly-Lys-OH, Cilnidipine (such as also FRP-8653, a dihydropyridine-type inhibitor), Dilantizem (such as (2S,3S)-(+)-cis-3-Acetoxy-5-(2-dimethylaminoethyl)-2,3-dihydro-2-(4-methoxyphenyl)-1,5-benzothiazepin-4(5H)-one hydrochloride), diltiazem (such as benzothiazepin-4(5H)-one, 3-(acetyloxy)-5-[2-(dimethylamino)ethyl]-2,3-dihydro-2-(4-methoxyphenyl)--,(+)- cis-, monohydrochloridc), Felodipine (such as 4-(2,3-Dichlorophenyl)-1,4-dihydro-2,6-dimethyl-3,5-pyridinecarboxylic acid ethyl methyl ester), FS-2 (such as an isolate from Dendroaspis polylepis polylepis venom), FTX-3.3 (such as an isolate from Agelenopsis aperta), Neomycin sulfate (such as $C_{23}H_{46}N_6O_{13} \cdot 3H_2SO_4$), Nicardipine (such as 1,4- Dihydro-2,6-dimethyl-4-(3-nitrophenypmethyl-2-[methyl(phenylmethypa-mino]-3,5-pyridinedicarboxylic acid ethyl ester hydrochloride, also YC-93, Nifedipine (such as 1,4-Dihydro-2,6-dimethyl-4-(2-nitrophenyl)-3,5-pyridinedicarboxylic acid dimethyl ester), Nimodipine (such as 4-Dihydro-2,6-dimethyl-4-(3-nitrophenyl)-3,5-pyridinedicarboxylic acid 2-methoxyethyl 1-methylethyl ester) or (Isopropyl 2-methoxyethyl 1,4-dihydro-2,6-dimethyl-4-(m¬nitrophenyl)-3,5-pyridinedicarboxylate), Nitrendipine (such as 1,4-Dihydro-2,6-dimethyl-4-(3- nitrophenyl)-3,5-pyridinedicarboxylic acid ethyl methyl ester), S-Petasin (such as (3S,4aR,5R,6R)-[2,3,4,4a,5,6,7,8-Octahydro-3-(2-propenyl)-4a,5-dimethyl-2-o-xo-6-naphthyl]Z¬3'-methylthio-1'-propenoate), Phloretin (such as 2',4',6'-Trihydroxy-3 -(4-hydroxyphenyl)propiophenone, also 3-(4-Hydroxyphenyl)-1-(2,4,6-trihydroxyphenyl)-1-propanone, also b-(4-Hydroxyphenyl)-2,4,6-trihy-droxypropiophenone), Protopine (such as $C_{20}H_{19}NO_5Cl$), SKF-96365 (such as 1-[b-[3-(4-Methoxyphenyl)propoxy]-4-methoxyphenethyl]-1H-imidazole, HCl), Tetrandine (such as 6,6',7,12-Tetramethoxy-2,2'-dimethylberbaman), (.+-.)-Methoxyverapamil or (+)-Verapamil (such as 54N-(3,4-Dimethoxyphenylethyl)methylamino]-2-(3,4-dimethoxyphenyl)-2-iso- propylvalcronitrilc hydrochloride), and (R)-(+)-Bay K8644 (such as R-(+)-1,4-Dihydro-2,6-dimethyl-5-nitro-442-(trifluoromethyl)phenyl]-3-pyridinecarboxylic acid methyl ester). The foregoing examples may be specific to L-type voltage-gated calcium channels or may inhibit a broader range of voltage- gated calcium channels, e.g. N, P/Q, R, and T-type.

According to some embodiments, the voltage-gated calcium channel blocker is a dihydropyridine calcium channel blocker. According to one embodiment, the dihydropyridine calcium channel blocker is nimodipine. According to one embodiment, the nimodipine has a half life of 7-10 days when formulated as described herein, and appropriate lipid solubility.

According to some embodiments, the voltage gated calcium channel blocker is an isolated molecule. The term "isolated molecule" as used herein refers to a molecule that is substantially pure and is free of other substances with which it is ordinarily found in nature or in vivo systems to an extent practical and appropriate for its intended use.

According to some embodiments, the voltage gated calcium channel blocker is admixed with a pharmaceutically-acceptable carrier in a pharmaceutical preparation. According to some such embodiments, the voltage gated calcium channel blocker comprises only a small percentage by weight of the preparation. According to some embodiments, the voltage gated calcium channel blocker is substantially pure.

***Pharmaceutically acceptable carrier***

According to some embodiments, the pharmaceutical composition does not comprise the pharmaceutically acceptable carrier. According to some embodiments, the pharmaceutically acceptable carrier does not comprise hyaluronic acid.

According to one embodiment, the pharmaceutically acceptable carrier is a solid carrier or excipient. According to another embodiment, the pharmaceutically acceptable carrier is a gel-phase carrier or excipient. Examples of carriers or excipients include, but are not limited to, calcium carbonate, calcium phosphate, various monomeric and polymeric sugars (including without limitation hyaluronic acid), starches, cellulose derivatives, gelatin, and polymers. An exemplary carrier can also include saline vehicle, e.g. hydroxyl propyl methyl cellulose (HPMC) in phosphate buffered saline (PBS).

According to some embodiments, the pharmaceutically acceptable carrier imparts stickiness. According to one embodiment, the pharmaceutically acceptable carrier comprises hyaluronic acid. According to some embodiments, the pharmaceutically acceptable carrier comprises 0 % to 5 % hyaluronic acid. According to one embodiment, the pharmaceutically acceptable carrier comprises less than 0.05 % hyaluronic acid. According to another embodiment, the pharmaceutically acceptable carrier comprises less than 0.1 % hyaluronic acid. According to another embodiment, the pharmaceutically acceptable carrier comprises less than 0.2 % hyaluronic acid. According to another embodiment, the pharmaceutically acceptable carrier comprises less than 0.3 % hyaluronic acid. According to another embodiment, the pharmaceutically acceptable carrier comprises less than 0.4 % hyaluronic acid. According to another embodiment, the pharmaceutically acceptable carrier comprises less than 0.5 % hyaluronic acid. According to another embodiment, the pharmaceutically acceptable carrier comprises less than 0.6 % hyaluronic acid. According to another embodiment, the pharmaceutically acceptable carrier comprises less than 0.7 % hyaluronic acid. According to another embodiment, the pharmaceutically acceptable carrier comprises less than 0.8 % hyaluronic acid. According to another embodiment, the pharmaceutically acceptable carrier comprises less than 0.9 % hyaluronic acid. According to another embodiment, the pharmaceutically acceptable carrier comprises less than 1.0 % hyaluronic acid. According to another embodiment, the pharmaceutically acceptable carrier comprises less than 1.1 % hyaluronic acid. According to another embodiment, the pharmaceutically acceptable carrier comprises less than 1.2 % hyaluronic acid. According to another embodiment, the pharmaceutically acceptable carrier comprises less than 1.3 % hyaluronic acid. According to another embodiment, the pharmaceutically acceptable carrier comprises less than 1.4 % hyaluronic acid. According to another embodiment, the pharmaceutically acceptable carrier comprises less than 1.5 % hyaluronic acid. According to another embodiment, the pharmaceutically acceptable carrier comprises less than 1.6 % hyaluronic acid. According to another embodiment, the pharmaceutically acceptable carrier comprises less than 1.7 % hyaluronic acid. According to another embodiment, the pharmaceutically acceptable carrier comprises less than 1.8 % hyaluronic acid. According to another embodiment, the pharmaceutically acceptable carrier comprises less than 1.9 % hyaluronic acid. According to another embodiment, the pharmaceutically acceptable carrier comprises less than 2.0 % hyaluronic acid. According to another embodiment, the pharmaceutically acceptable carrier comprises less than 2.1 % hyaluronic acid. According to another embodiment, the pharmaceutically acceptable carrier comprises less than 2.2 % hyaluronic acid. According to another embodiment, the pharmaceutically acceptable carrier comprises less than 2.3% hyaluronic acid. According to another embodiment, the pharmaceutically acceptable carrier comprises less than 2.4 % hyaluronic acid. According to another embodiment, the pharmaceutically acceptable carrier comprises less than 2.5 % hyaluronic acid. According to another embodiment, the pharmaceutically acceptable carrier comprises less than 2.6 % hyaluronic acid. According to another embodiment, the pharmaceutically acceptable carrier comprises less than 2.7 % hyaluronic acid. According to another embodiment, the pharmaceutically acceptable carrier comprises less than 2.8 % hyaluronic acid. According to another embodiment, the pharmaceutically acceptable carrier comprises less than 2.9 % hyaluronic acid. According to another embodiment, the pharmaceutically acceptable carrier comprises less than 3.0 % hyaluronic acid. According to another embodiment, the pharmaceutically acceptable carrier comprises less than 3.5 % hyaluronic acid. According to another embodiment, the pharmaceutically acceptable carrier comprises less than 4.0 % hyaluronic acid. According to another embodiment, the pharmaceutically acceptable carrier comprises less than 4.5 % hyaluronic acid. According to another embodiment, the pharmaceutically acceptable carrier comprises less than 5.0 % hyaluronic acid.

In some embodiments, the pharmaceutically acceptable carrier includes, but is not limited to, a gel, slow-release solid or semisolid compound, optionally as a sustained release gel. In some such embodiments, the voltage-gated calcium channel blocker is embedded into the pharmaceutically acceptable carrier. In some embodiments, the voltage-gated calcium channel blocker is coated on the pharmaceutically acceptable carrier. The coating can be of any desired material, preferably a polymer or mixture of different polymers. Optionally, the polymer can be utilized during the granulation stage to form a matrix with the active ingredient so as to obtain a desired release pattern of the active ingredient. The gel, slow-release solid or semisolid compound is capable of releasing the active agent over a desired period of time. The gel, slow-release solid or semisolid compound can be implanted in a tissue within human brain, for example, but not limited to, in close proximity to a blood vessel, such as a cerebral artery.

According to another embodiment, the pharmaceutically acceptable carrier comprises a slow-release solid compound. According to one such embodiment, the voltage-gated calcium channel blocker is embedded in the slow-release solid compound or coated on the slow-release solid compound. According to yet another embodiment, the pharmaceutically acceptable carrier comprises a slow-release microparticle containing the voltage-gated calcium channel blocker.

According to another embodiment, the pharmaceutically acceptable carrier is a gel compound, such as a biodegradable hydrogel.

### Microparticle Formulation

According to some embodiments, the voltage-gated calcium channel blocker is provided in the form of a particle. The term "particle" as used herein refers to nano or microparticles (or in some instances larger) that may contain in whole or in part the calcium channel blocker. According to some embodiments, the microparticulate formulation comprises a plurality of microparticles impregnated with the voltage-gated calcium channel blocker. According to one embodiment, the voltage-gated calcium channel blocker is contained within the core of the particle surrounded by a coating. According to another embodiment, the voltage-gated calcium channel blocker is dispersed throughout the surface of the particle. According to another embodiment, the voltage-gated calcium channel blocker is disposed on or in the microparticle. According to another embodiment, the voltage-gated calcium channel blocker is disposed throughout the surface of the microparticle. According to another embodiment, the voltage-gated calcium channel blocker is adsorbed into the particle.

According to some such embodiments, the microparticles are of uniform size distribution. According to some embodiments, the uniform distribution of microparticle size is achieved by a homogenization process to form a uniform emulsion comprising microparticles. According to some such embodiments, each microparticle comprises a matrix. According to some embodiments, the matrix comprises the voltage gated calcium channel blocker.

According to some embodiments, the pharmaceutical composition is flowable. According to some embodiments, the microparticulate formulation component of the pharmaceutical composition is flowable.

According to some embodiments, the particle is selected from the group consisting of a zero order release, first order release, second order release, delayed release, sustained release, immediate release, and a combination thereof. The particle can include, in addition to therapeutic agent(s), any of those materials routinely used in the art of pharmacy and medicine, including, but not limited to, erodible, nonerodible, biodegradable, or nonbiodegradable material or combinations thereof

According to some embodiments, the particle is a microcapsule that contains the voltage-gated calcium channel blocker in a solution or in a semi-solid state. According to some embodiments, the particle is a microparticle that contains the voltage-gated calcium channel blocker, in whole or in part. According to some embodiments, the particle is a nanoparticle that contains the voltage-gated calcium channel blocker, in whole or in part. According to some embodiments, the particles can be of virtually any shape.

According to some embodiments, the particle size is between about 25 $\mu$m to about 100 $\mu$m. According to some embodiments, the particle size is between about 30 $\mu$m to about 80 $\mu$m. According to one embodiment, the particle size is at least about 25 $\mu$m. According to another embodiment, the particle size is at least about 30 $\mu$m. According to another embodiment, the particle size is at least about 35 $\mu$m. According to another embodiment, the particle size is at least about 40 $\mu$m. According to another embodiment, the particle size is at least about 45 $\mu$m. According to another embodiment, the particle size is at least about 50 $\mu$m. According to another embodiment, the particle size is at least about 55 $\mu$m. According to another embodiment, the particle size is at least about 60 $\mu$m. According to another embodiment, the particle size is at least about 65 $\mu$m. According to another embodiment, the particle size is at least about 70 $\mu$m. According to another embodiment, the particle size is at least about 75 $\mu$m. According to another embodiment, the particle size is at least about 80 $\mu$m. According to another embodiment, the particle size is at least about 85 $\mu$m. According to another embodiment, the particle size is at least about 90 $\mu$m. According to another embodiment, the particle size is at least about 95 $\mu$m. According to another embodiment, the particle size is at least about 100 $\mu$m.

According to another embodiment, the voltage gated calcium channel blocker can be provided in strings. The strings can contain the voltage-gated calcium channel blocker in a core surrounded by a coating, or the voltage-gated calcium channel blocker can be dispersed throughout the string, or therapeutic agent(s) may be absorbed into the string. The string can be of any order release kinetics, including zero order release, first order release, second order release, delayed release, sustained release, immediate release, etc., and any combination thereof. The string can include, in addition to therapeutic agent(s), any of those materials routinely used in the art of pharmacy and medicine, including, but not limited to, erodible, nonerodible, biodegradable, or nonbiodegradable material or combinations thereof.

According to another embodiment, the voltage-gated calcium channel blocker can be provided in at least one sheet. The sheet can contain the voltage-gated calcium channel blocker and at least one additional therapeutic agent in a core surrounded by a coating, or the voltage-gated calcium channel blocker and at least one additional therapeutic agent can be dispersed throughout the sheet, or the voltage-gated calcium channel blocker can be absorbed into the sheet. The sheet can be of any order release kinetics, including zero order release, first order release, second order release, delayed release, sustained release, immediate release, etc., and any combination thereof. The sheet can include, in addition to the voltage-gated calcium channel blocker and at least one additional therapeutic agent, any of those materials routinely used in the art of pharmacy and medicine, including, but not limited to, erodible, nonerodible, biodegradable, or nonbiodegradable material or combinations thereof.

According to some embodiments, the pharmaceutical composition further comprises a preservative agent. According to some such embodiments, the pharmaceutical composition is presented in a unit dosage form. Exemplary unit dosage forms include, but arc not limited to, ampoules or multi-dose containers.

According to some embodiments, the microparticulate formulation comprises a suspension of microparticles. According to some embodiments, the pharmaceutical composition further comprises at least one of a suspending agent, a stabilizing agent and a dispersing agent. According to some such embodiments, the pharmaceutical composition is presented as a suspension. According to some such embodiments, the pharmaceutical composition is presented as a solution. According to some such embodiments, the pharmaceutical composition is presented as an emulsion.

According to some embodiments, a formulation of the pharmaceutical composition comprises an aqueous solution of the voltage-gated calcium channel blocker in water-soluble form. According to some embodiments, the formulation of the pharmaceutical composition comprises an oily suspension of the voltage-gated calcium channel blocker. Oily suspension of the voltage-gated calcium channel blocker can be prepared using suitable lipophilic solvents. Exemplary lipophilic solvents or vehicles include, but are not limited to, fatty oils such as sesame oil, or synthetic fatty acid esters, such as ethyl oleate or triglycerides. According to some embodiments, the formulation of the pharmaceutical composition comprises an aqueous suspension of the volgate-gated calcium channel blocker. Aqueous injection suspensions can contain substances which increase the viscosity of the suspension, such as sodium carboxymethyl cellulose, sorbitol, or dextran. Optionally, the suspension can also contain suitable stabilizers or agents which increase the solubility of the compounds to allow for the preparation of highly concentrated solutions. Alternatively, the volgate-gated calcium channel blocker can be in powder form for constitution with a suitable vehicle, e.g., sterile pyrogen-free water, before use.

Suitable liquid or solid pharmaceutical preparations include, for example, microencapsulated dosage forms, and if appropriate, with one or more excipients, encochleated, coated onto microscopic gold particles, contained in liposomes, pellets for implantation into the tissue, or dried onto an object to be rubbed into the tissue. As used herein, the term "microencapsulation" refers to a process in which very tiny droplets or particles are surrounded or coated with a continuous film of biocompatible, biodegradable, polymeric or non-polymeric material to produce solid structures including, but not limited to, nonpareils, pellets, crystals, agglomerates, microspheres, or nanoparticlcs. Such pharmaceutical compositions also can be in the form of granules, beads, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, or solubilizers are customarily used as described above. The pharmaceutical compositions are suitable for use in a variety of drug delivery systems. For a brief review of methods for drug delivery, see Langer (1990) Science 249, 1527-1533, which is incorporated herein by reference.

*Microencapsulation Process*

Examples of microencapsulation processes and products; methods for the production of emulsion-based microparticles; emulsion-based microparticles and methods for the production thereof; solvent extraction microencapsulation with tunable extraction rates; microencapsulation process with solvent and salt; a continuous double emulsion process for making microparticles; drying methods for tuning microparticle properties, controlled release systems from polymer blends; polymer mixtures comprising polymers having different non-repeating units and methods for making and using the same; and an emulsion based process for preparing microparticles and workhead assembly for use with same are disclosed and described in U.S. Patent No. 5, 407,609 (entitled Microencapsulation Process and Products Thereof), U.S. Application No. 10/553,003 (entitled Method for the production of emulsion-based microparticles), U.S. Application No. 11/799,700 (entitled Emulsion-based microparticles and methods for the production thereof), U.S. Application No. 12/557,946 (entitled Solvent Extraction Microencapsulation With Tunable Extraction Rates), U.S. Application No. 12/779,138 (entitled Hyaluronic Acid (HA) Injection Vehicle), U.S. Application No. 12/562,455 entitled Microencapsulation Process With Solvent And Salt), U.S. Application No. 12/338,488 (entitled Process For Preparing Microparticles Having A Low Residual Solvent Volume); U.S. Application No. 12/692,027 (entitled Controlled Release Systems From Polymer Blends); U.S. Application No. 12/692,020 (entitled Polymer Mixtures Comprising Polymers Having Different Non-Repeating Units And Methods For Making And Using Same); U.S. Application No. 10/565,401 (entitled "Controlled release compositions"); U.S. Application No. 12/692,029 (entitled "Drying Methods for Tuning Microparticle Properties); U.S. Application No. 12/968,708 (entitled "Emulsion Based Process for Preparing Microparticles and Workhead for Use with Same); and U.S. Application No. 13/074,542 (entitled Composition and Methods for Improved Retention of a Pharmaceutical Composition at a Local Administration Site") The contents of each of these are incorporated herein by reference in its entirety.

According to some embodiments, delivery of the voltage-gated calcium channel blocker using microparticle technology involves bioresorbable, polymeric particles that encapsulate the voltage-gated calcium channel blocker and at least one additional therapeutic agent.

According to one embodiment, the microparticle formulation comprises a polymer matrix, wherein the voltage-gated calcium channel blocker is impregnated in the polymer matrix. According to one embodiment, the polymer is a slow release polymer. According to one embodiment, the polymer is poly (D, L-Lactide-co-glycolide). According to another embodiment, the polymer is poly(orthoester). According to another embodiment, the polymer is poly(anhydride). According to another embodiment, the polymer is polylactide-polyglycolide.

Both non-biodegradable and biodegradable polymeric materials can be used in the manufacture of particles for delivering the voltage-gated calcium channel blocker. Such polymers can be natural or synthetic polymers. The polymer is selected based on the period of time over which release is desired. Bioadhesive polymers of particular interest include, but are not limited to, bioerodible hydrogels as described by Sawhney et al in Macromolecules (1993) 26, 581-587, the teachings of which are incorporated herein. Exemplary bioerodible hydrogels include, but are not limited to, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate). According to one embodiment, the bioadhesive polymer is hyaluronic acid. In some such embodiments, the bioadhesive polymer include less than about 2.3 % of hyaluronic acid.

According to some embodiments, the pharmaceutical composition is formulated for parenteral injection, surgical implantation, or a combination thereof. According to some such embodiments, the pharmaceutical composition is in the form of a pharmaceutically acceptable sterile aqueous or nonaqueous solution, dispersion, suspension or emulsion or a sterile powder for reconstitution into a sterile injectable solution or dispersion. Examples of suitable aqueous and nonaqueous carriers, diluents, solvents or vehicles include, but are not limited to, water, ethanol, dichloromethane, acetonitrile, ethyl acetate, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants. Suspensions can further contain suspending agents, as, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

According to some embodiments, the pharmaceutical composition is formulated in an injectable depot form. Injectable depot forms are made by forming microencapsulated matrices of the voltage gated calcium channel blocker in a biodegradable polymer. Depending upon the ratio of drug to polymer and the nature of the particular polymer employed, the rate of drug release may be controlled. Such long acting formulations can be formulated with suitable polymeric or hydrophobic materials (for example as an emulsion in an acceptable oil) or ion exchange resins, or as sparingly soluble derivatives, for example, as a sparingly soluble salt. Examples of biodegradable polymers include, but are not limited to, polylactide-polyglycolide, poly(orthoesters) and poly(anhydrides). Depot injectable formulations are also prepared by entrapping the drug in liposomes or microemulsions which are compatible with body tissues.

According to some embodiments, the voltage-gated calcium channel blocker is impregnated in or on a polyglycolide (PGA) matrix. PGA is a linear aliphatic polyester developed for use in sutures. Studies have reported PGA copolymers formed with trimethylene carbonate, polylactic acid (PLA), and polycaprolactone. Some of these copolymers may be formulated as microparticles for sustained drug release.

According to some embodiments, the voltage-gated calcium channel blocker is impregnated in or on a polyester-polyethylene glycol matrix. Polyester-polyethylene glycol compounds can be synthesized; these are soft and may be used for drug delivery.

According to some embodiments, the voltage-gated calcium channel blocker is impregnated in or on a poly (amino)-derived biopolymer matrix. Poly (amino)-derived biopolymers can include, but are not limited to, those containing lactic acid and lysine as the aliphatic diamine (see, for example, U.S. Patent 5,399,665), and tyrosine-derived polycarbonates and polyacrylates. Modifications of polycarbonates may alter the length of the alkyl chain of the ester (ethyl to octyl), while modifications of polyarylates may further include altering the length of the alkyl chain of the diacid (for example, succinic to sebasic), which allows for a large permutation of polymers and great flexibility in polymer properties.

According to some embodiments, the voltage-gated calcium channel blocker is impregnated in or on a polyanhydride matrix. Polyanhydrides are prepared by the dehydration of two diacid molecules by melt polymerization (see, for example, U.S. Patent 4,757,128). These polymers degrade by surface erosion (as compared to polyesters that degrade by bulk erosion). The release of the drug can be controlled by the hydrophilicity of the monomers chosen.

According to some embodiments, the voltage-gated calcium channel blocker is impregnated in or on a photopolymerizable biopolymer matrix. Photopolymerizable biopolymers include, but are not limited to, lactic acid/polyethylene glycol/acrylate copolymers.

According to some embodiments, the voltage-gated calcium channel blocker is impregnated in or on a hydrogel matrix. The term "hydrogel" refers to a substance resulting in a solid, semisolid, pseudoplastic or plastic structure containing a necessary aqueous component to produce a gelatinous or jelly-like mass. Hydrogels generally comprise a variety of polymers, including hydrophilic polymers, acrylic acid, acrylamide and 2-hydroxyethylmethacrylate (HEMA).

According to some embodiments, the voltage-gated calcium channel blocker is impregnated in or on a naturally-occurring biopolymer matrix. Naturally-occurring biopolymers include, but are not limited to, protein polymers, collagen, polysaccharides, and photopolymerizable compounds.

According to some embodiments, the voltage-gated calcium channel blocker is impregnated in or on a protein polymer matrix. Protein polymers have been synthesized from self-assembling protein polymers such as, for example, silk fibroin, elastin, collagen, and combinations thereof.

According to some embodiments, the voltage-gated calcium channel blocker is impregnated in or on a naturally-occurring polysaccharide matrix. Naturally-occurring polysaccharides include, but are not limited to, chitin and its derivatives, hyaluronic acid, dextran and cellulosics (which generally are not biodegradable without modification), and sucrose acetate isobutyrate (SAIB).

According to some embodiments, the voltage-gated calcium channel blocker is impregnated in or on a chitin matrix. Chitin is composed predominantly of 2-acetamido-2-deoxy-D-glucose groups and is found in yeasts, fungi and marine invertebrates (shrimp, crustaceous) where it is a principal component of the exoskeleton. Chitin is not water soluble and the deacetylated chitin, chitosan, only is soluble in acidic solutions (such as, for example, acetic acid). Studies have reported chitin derivatives that are water soluble, very high molecular weight (greater than 2 million daltons), viscoelastic, non-toxic, biocompatible and capable of crosslinking with peroxides, gluteraldehyde, glyoxal and other aldehydes and carbodiamides, to form gels.

According to some embodiments, the voltage-gated calcium channel blocker is impregnated in or on a hyaluronic acid (HA) matrix. Hyaluronic acid (HA), which is composed of alternating glucuronidic and glucosaminidic bonds and is found in mammalian vitreous humor, extracellular matrix of the brain, synovial fluid, umbilical cords and rooster combs from which it is isolated and purified, also can be produced by fermentation processes.

According to some embodiments, the pharmaceutical composition further comprises an adjuvant. Exemplary adjuvants include, but are not limited to, preservative agents, wetting agents, emulsifying agents, and dispersing agents. Prevention of the action of microorganisms can be ensured by various antibacterial and antifungal agents, for example, parabens, chlorobutanol, phenol, sorbic acid, and the like. Isotonic agents, for example, sugars, sodium chloride and the like, can also be included. Prolonged absorption of the injectable pharmaceutical form can be brought about by the use of agents delaying absorption, for example, aluminum monostearate and gelatin.

The formulations can be sterilized, for example, by terminal gamma irradiation, filtration through a bacterial-retaining filter or by incorporating sterilizing agents in the form of sterile solid compositions that may be dissolved or dispersed in sterile water or other sterile injectable medium just prior to use. Injectable preparations, for example, sterile injectable aqueous or oleaginous suspensions may be formulated according to the known art using suitable dispersing or wetting agents and suspending agents. The sterile injectable preparation also may be a sterile injectable solution, suspension or emulsion in a nontoxic, parenterally acceptable diluent or solvent such as a solution in 1,3-butanediol, dichloromethane, ethyl acetate, acetonitrile, etc. Among the acceptable vehicles and solvents that may be employed are water, Ringer's solution, U.S.P. and isotonic sodium chloride solution. In addition, sterile, fixed oils conventionally are employed or as a solvent or suspending medium. For this purpose any bland fixed oil may be employed including synthetic mono- or diglycerides. In addition, fatty acids such as oleic acid are used in the preparation of injectables.

Formulations for parenteral (including but not limited to, subcutaneous, intradermal, intramuscular, intravenous, intraarterial, intrathecal, intraventricular and intraarticular) administration include aqueous and non-aqueous sterile injection solutions that can contain anti-oxidants, buffers, bacteriostats and solutes, which render the formulation isotonic with the blood of the intended recipient; and aqueous and non-aqueous sterile suspensions, which can include suspending agents and thickening agents.

According to another embodiment, the pharmaceutical composition is formulated by conjugating the voltage-gated calcium channel blocker to a polymer that enhances aqueous solubility. Examples of suitable polymers include but are not limited to polyethylene glycol, poly-(d-glutamic acid), poly-(1-glutamic acid), poly-(1-glutamic acid), poly-(d-aspartic acid), poly-(1-aspartic acid), poly-(1-aspartic acid) and copolymers thereof. Polyglutamic acids having molecular weights between about 5,000 to about 100,000, with molecular weights between about 20,000 and about 80,000 may be used and with molecular weights between about 30,000 and about 60,000 may also be used. The polymer is conjugated via an ester linkage to one or more hydroxyls of an inventive epothilone using a protocol as essentially described by U.S. Pat. No. 5,977,163 which is incorporated herein by reference. Particular conjugation sites include the hydroxyl off carbon-21 in the case of 21-hydroxy-derivatives of the present invention. Other conjugation sites include, but are not limited, to the hydroxyl off carbon 3 and/or the hydroxyl off carbon 7.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

According to another embodiment, the semisolid multiparticulate delivery system comprises a partially or in whole biocompatible, biodegradable, viscous semisolid wherein the semisolid comprises a hydrogel, wherein the hydrogel comprises the voltage-gated calcium channel blocker. The term "hydrogel" as used herein refers to a substance resulting in a solid, semisolid, pseudoplastic, or plastic structure containing a necessary aqueous component to produce a gelatinous or jelly-like mass. The hydrogel incorporates and retains significant amounts of $H_2O$, which eventually will reach an equilibrium content in the presence of an aqueous environment. In one embodiment, glyceryl monooleate, hereinafter referred to as GMO, is the intended semisolid delivery system or hydrogel. However, many hydrogels, polymers, hydrocarbon compositions and fatty acid derivatives having similar physical/chemical properties with respect to viscosity/rigidity may function as a semisolid delivery system.

In one embodiment, the gel system is produced by heating GMO above its melting point (40-50°C) and by adding a warm aqueous-based buffer or electrolyte solution, such as, for example, phosphate buffer or normal saline, which thus produces a three-dimensional structure. The aqueous-based buffer may be comprised of other aqueous solutions or combinations containing semi-polar solvents.

GMO provides a predominantly lipid-based hydrogel, which has the ability to incorporate lipophilic materials. The term "lipophilic" as used herein refers to preferring or possessing an affinity for a non-polar environment compared to a polar or aqueous environment. GMO further provides internal aqueous channels that incorporate and deliver hydrophilic compounds. The term "hydrophilic" as used herein refers to a material or substance having an affinity for polar substances, such as water. It is recognized that at room temperature (-25°C), the gel system may exhibit differing phases which comprise a broad range of viscosity measures.

In one embodiment, two gel system phases are utilized due to their properties at room temperature and physiologic temperature (about 37°C) and pH (about 7.4). Within the two gel system phases, the first phase is a lamellar phase of approximately 5% to approximately 15% $H_2O$ content and approximately 95% to approximately 85% GMO content. The lamellar phase is a moderately viscous fluid, that may be easily manipulated, poured and injected. The second phase is a cubic phase consisting of approximately 15% to approximately 40% $H_2O$ content and approximately 85%-60% GMO content. It has an equilibrium water content of approximately 35% to approximately 40% by weight. The term "equilibrium water content" as used herein refers to maximum water content in the presence of excess water. Thus the cubic phase incorporates water at approximately 35% to approximately 40% by weight. The cubic phase is highly viscous. The viscosity exceeds 1.2 million centipoise (cp) when measured by a Brookfield viscometer; where 1.2 million cp is the maximum measure of viscosity obtainable via the cup and bob configuration of the Brookfield viscometer. In some such embodiments, a therapeutic agent may be incorporated into the semisolid so as to provide a system for sustained, continuous delivery. In some such embodiments, therapeutic agent is a calcium channel blocker. In some such embodiments, therapeutic agent is a dihydropyridine calcium channel blocker. In some such embodiments, therapeutic agent is nimodipine. In some such embodiments, other therapeutic agents, biologically-active agents, drugs, medicaments and inactives may be incorporated into the semisolid for providing a local biological, physiological, or therapeutic effect in the body at various release rates.

In some embodiments, alternative semisolids, modified formulations and methods of production are utilized such that the lipophilic nature of the semisolid is altered, or in the alternative, the aqueous channels contained within the semisolid arc altered. Thus, various therapeutic agents in varying concentrations may diffuse from the semisolid at differing rates, or be released therefrom over time via the aqueous channels of the semisolid. Hydrophilic substances may be utilized to alter semisolid consistency or therapeutic agent release by alteration of viscosity, fluidity, surface tension or the polarity of the aqueous component. For example, glyceryl monostearate (GMS), which is structurally identical to GMO with the exception of a double bond at Carbon 9 and Carbon 10 of the fatty acid moiety rather than a single bond, does not gel upon heating and the addition of an aqueous component, as does GMO. However, because GMS is a surfactant, GMS is miscible in $H_2O$ up to approximately 20% weight/weight. The term "surfactant" as used herein refers to a surface active agent that is miscible in $H_2O$ in limited concentrations as well as polar substances. Upon heating and stirring, the 80% $H_2O$/ 20% GMS combination produces a spreadable paste having a consistency resembling hand lotion. The paste then is combined with melted GMO so as to form the cubic phase gel possessing a high viscosity referred to above.

According to another embodiment, hydrolyzed gelatin, such as commercially available Gelfoam™, is utilized for altering the aqueous component. Approximately 6.25% to 12.50% concentration of Gelfoam™ by weight may be placed in approximately 93.75% to 87.50% respectively by weight $H_2O$ or another aqueous based buffer. Upon heating and stirring, the $H_2O$ (or other aqeuous buffer)/Gelfoam™ combination produces a thick gelatinous substance. The resulting substance is combined with GMO; a product so formed swells and forms a highly viscous, translucent gel being less malleable in comparison to neat GMO gel alone.

According to another embodiment, polyethylene glycols (PEG's) can be utilized for altering the aqueous component to aid in drug solubilization. Approximately 0.5% to 40% concentration of PEG's (depending on PEG molecular weight) by weight can be placed in approximately 99.5% to 60% $H_2O$ or other aqueous based buffer by weight. Upon heating and stirring, the $H_2O$ (or other aqueous buffer)/PEG combination produces a viscous liquid to a semisolid substance. The resulting substance is combined with GMO, whereby a product so formed swells and forms a highly viscous gel.

According to some embodiments, the voltage-gated calcium channel blocker releases from the semisolid through diffusion, conceivably in a biphasic manner. A first phase involves, for example, a lipophilic drug contained within the lipophilic membrane that diffuses therefrom into the aqueous channel. The second phase involves diffusion of the drug from the aqueous channel into the external environment. Being lipophilic, the drug may orient itself inside the GMO gel within its proposed lipid bi-layer structure. Thus, incorporating greater than approximately 7.5% of the drug by weight into GMO causes a loss of the integrity of the three-dimensional structure whereby the gel system no longer maintains the semisolid cubic phase, and reverts to the viscous lamellar phase liquid. In some such embodiments, therapeutic agent is nimodipine. In some such embodiments, therapeutic agent is a calcium channel blocker. In some such embodiments, therapeutic agent is a transient receptor potential protein blocker. According to another embodiment, about 1% to about 45% of therapeutic agent is incorporated by weight into a GMO gel at physiologic temperature without disruption of the normal three- dimensional structure. As a result, this system allows the ability of significantly increased flexibility with drug dosages. Because the delivery system is malleable, it may be delivered and manipulated in an implant site, for example, adjacent to cerebral arteries or the subarachnoid space, so as to adhere and conform to contours of walls, spaces, or other voids in the body as well as completely fill all voids existing. The delivery system ensures drug distribution and uniform drug delivery throughout the implant site. Ease of delivery and manipulation of the delivery system within a space, for example, but not limited to the subarachnoid space, is facilitated via a semisolid delivery apparatus. A semisolid delivery apparatus facilitates targeted and controlled delivery of the delivery system.

Alternatively, the present invention provides a semisolid delivery system, which acts as a vehicle for local delivery of therapeutic agents, comprising a lipophilic, hydrophilic or amphophilic, solid or semisolid substance, heated above its melting point and thereafter followed by inclusion of a warm aqueous component so as to produce a gelatinous composition of variable viscosity based on water content. Therapeutic agent(s) is incorporated and dispersed into the melted lipophilic component or the aqueous buffer component prior to mixing and formation of the semisolid system. The gelatinous composition is placed within the semisolid delivery apparatus for subsequent placement, or deposition. Being malleable, the gel system is easily delivered and manipulated via the semisolid delivery apparatus in an implant site, where it adheres and conforms to contours of the implantation site, spaces, or other voids in the body as well as completely filling all voids existing. Alternatively, a multiparticulate component, comprised of a biocompatible polymeric or non-polymeric system is utilized for producing microspheres having a therapeutic agent entrapped therein. Following final processing methods, the microspheres are incorporated into the semisolid system and subsequently placed within the semisolid delivery apparatus so as to be easily delivered therefrom into an implant site or comparable space, whereby therapeutic agent is subsequently released therefrom by (a) drug release mechanism(s).

## III. Methods

In another aspect, the described invention provides a method for treating a delayed complication associated with a brain injury in a mammal in need thereof, wherein the brain injury includes interruption of at least one cerebral artery, the method comprising:

(a) providing a pharmaceutical composition comprising

(i) a microparticulate formulation of a voltage-gated calcium channel blocker; and optionally

(ii) a pharmaceutically acceptable carrier; and

(b) administering a therapeutic amount of the pharmaceutical composition by a means for administering the therapeutic amount of the pharmaceutical composition into an administration site, wherein the therapeutic amount is effective in reducing signs or symptoms of the at least one delayed complication associated with the brain injury

According to one embodiment, a means for administering the a therapeutic amount of the pharmaceutical composition in step (b) is a surgical injection apparatus, and the site for administration is a site in close proximity to the cerebral artery affected by the brain injury.

According to another embodiment, a means for administering the a therapeutic amount of the pharmaceutical composition in step (b) is a catheter, and the site for administration is locally into a cerebral ventricle so that the microparticle formulation component of the composition is carried by CSF circulation to contact the cerebral artery affected by the brain injury.

According to some such embodiments, the at least one delayed complication is selected from the group consisting of a delayed cerebral ischemia (DCI), an intracerebral hematoma, an intraventricular hemorrhage, a fever, an angiographic vasospasm, a microthromboembolus, a cortical spreading ischemia (CSI), a behavioral deficit, a neurological deficit, a cerebral infarction, neuronal cell death, or a combination thereof. According to one embodiment, the at least one delayed complication is a delayed cerebral ischemia (DCI). According to another embodiment, the at least one delayed complication is an intracerebral hematoma. According to another embodiment, the at least one delayed complication is an intraventricular hemorrhage. According to another embodiment, the at least one delayed complication is a fever. According to another embodiment, the at least one delayed complication is an angiographic vasospasm. According to another embodiment, the at least one delayed complication is a cortical spreading ischemia (CSI). According to another embodiment, the at least one delayed complication is a microthromboembolism. According to another embodiment, the at least one delayed complication is a behavioral deficit. According to another embodiment, the at least one delayed complication is a neurological deficit. According to another embodiment, the at least one delayed complication is a cerebral infarction. According to another embodiment, the at least one delayed complication is neuronal cell death.

According to some embodiments, the brain injury is a result of an underlying condition. Exemplary underlying conditions include, but are not limited to, aneurysm, sudden traumatic head injury, subarachnoid hemorrhage (SAH), etc. According to some such embodiments, the underlying condition is selected from the group consisting of aneurysm, sudden traumatic head injury, subarachnoid hemorrhage (SAH), or a combination thereof. According to one embodiment, the underlying condition is an aneurysm. According to another embodiment, the underlying condition is a sudden traumatic head injury. According to another embodiment, the underlying condition is a subarachnoid hemorrhage (SAH).

According to some embodiments, the pharmaceutical composition, when administered in a therapeutic amount at a site of delivery in the mammal, is effective in preventing or reducing the incidence or severity of at least one delayed complication associated with a brain injury in a mammal in need thereof, wherein the brain injury includes interruption of at least one cerebral artery. According to one embodiment, the site of delivery is a cerebral ventricle. According to some embodiments, the cerebral ventricle is selected from the group consisting of a lateral ventricle, a third ventricle, a fourth ventricle, or a combination thereof. According to one embodiment, the site of delivery is in subarachnoid space. According to one embodiment, the site of delivery is in close proximity to the brain injury. According to another embodiment, the site of delivery is in close proximity to a blood vessel that is affected by the brain injury. According to one embodiment, the blood vessel is at least one cerebral artery. According to another embodiment, the blood vessel is the at least one cerebral artery affected by the brain injury.

According to some embodiments, the pharmaceutical composition is administered by parenteral injection or surgical implantation.

The cerebral ventricles may be cannulatcd or cathctcrizcd as is well-known in the art and as described in various neurosurgical textbooks. This is called insertion of a ventricular catheter or drain or ventriculostomy. A hole of varying size can be drilled in the skull and the outer dura mater covering the brain incised. The pia mater is incised and a catheter (a hollow tube generally made of silicone elastomer of some other biocompatible, nonabsorbable compound) is inserted through the brain into the ventricle of choice. This usually is the lateral ventricle but any ventricle could be catheterized. The catheter can be used to monitor the pressure inside the head, to drain CSF or to administer substances into the CSF.

According to some embodiments, the site of delivery is within 10 mm, less than 10 mm, less than 9.9 mm, less than 9.8 mm, less than 9.7 mm, less than 9.6 mm, less than 9.5 mm, less than 9.4 mm, less than 9.3 mm, less than 9.2 mm, less than 9.1 mm, less than 9.0 mm, less than 8.9 mm, less than 8.8 mm, less than 8.7 mm, less than 8.6 mm, less than 8.5 mm, less than 8.4 mm, less than 8.3 mm, less than 8.2 mm, less than 8.1 mm, less than 8.0 mm, less than 7.9 mm, less than 7.8 mm, less than 7.7 mm, less than 7.6 mm, less than 7.5 mm, less than 7.4 mm, less than 7.3 mm, less than 7.2 mm, less than 7.1 mm, less than 7.0 mm, less than 6.9 mm, less than 6.8 mm, less than 6.7 mm, less than 6.6 mm, less than 6.5 mm, less than 6.4 mm, less than 6.3 mm, less than 6.2 mm, less than 6.1 mm, less than 6.0 mm, less than 5.9 mm, less than 5.8 mm, less than 5.7 mm, less than 5.6 mm, less than 5.5 mm, less than 5.4 mm, less than 5.3 mm, less than 5.2 mm, less than 5.1 mm, less than 5.0 mm, less than 4.9 mm, less than 4.8 mm, less than 4.7 mm, less than 4.6 mm, less than 4.5 mm, less than 4.4 mm, less than 4.3 mm, less than 4.2 mm, less than 4.1 mm, less than 4.0 mm, less than 3.9 mm, less than 3.8 mm, less than 3.7 mm, less than 3.6 mm, less than 3.5 mm, less than 3.4 mm, less than 3.3 mm, less than 3.2 mm, less than 3.1 mm, less than 3.0 mm, less than 2.9 mm, less than 2.8 mm, less than 2.7 mm, less than 2.6 mm, less than 2.5 mm, less than 2.4 mm, less than 2.3 mm, less than 2.2 mm, less than 2.1 mm, less than 2.0 mm, less than 1.9 mm, less than 1.8 mm, less than 1.7 mm, less than 1.6 mm, less than 1.5 mm, less than 1.4 mm, less than 1.3 mm, less than 1.2 mm, less than 1.1 mm, less than 1.0 mm, less than 0.9 mm, less than 0.8 mm, less than 0.7 mm, less than 0.6 mm, less than 0.5 mm, less than 0.4 mm, less than 0.3 mm, less than 0.2 mm, less than 0.1 mm, less than 0.09 mm, less than 0.08 mm, less than 0.07 mm, less than 0.06 mm, less than 0.05 mm, less than 0.04 mm, less than 0.03 mm, less than 0.02 mm, less than 0.01 mm, less than 0.009 mm, less than 0.008 mm, less than 0.007 mm, less than 0.006 mm, less than 0.005 mm, less than 0.004 mm, less than 0.003 mm, less than 0.002 mm, less than 0.001 mm of the brain injury.

According to some embodiments, the site of delivery is within 10 mm, less than 10 mm, less than 9.9 mm, less than 9.8 mm, less than 9.7 mm, less than 9.6 mm, less than 9.5 mm, less than 9.4 mm, less than 9.3 mm, less than 9.2 mm, less than 9.1 mm, less than 9.0 mm, less than 8.9 mm, less than 8.8 mm, less than 8.7 mm, less than 8.6 mm, less than 8.5 mm, less than 8.4 mm, less than 8.3 mm, less than 8.2 mm, less than 8.1 mm, less than 8.0 mm, less than 7.9 mm, less than 7.8 mm, less than 7.7 mm, less than 7.6 mm, less than 7.5 mm, less than 7.4 mm, less than 7.3 mm, less than 7.2 mm, less than 7.1 mm, less than 7.0 mm, less than 6.9 mm, less than 6.8 mm, less than 6.7 mm, less than 6.6 mm, less than 6.5 mm, less than 6.4 mm, less than 6.3 mm, less than 6.2 mm, less than 6.1 mm, less than 6.0 mm, less than 5.9 mm, less than 5.8 mm, less than 5.7 mm, less than 5.6 mm, less than 5.5 mm, less than 5.4 mm, less than 5.3 mm, less than 5.2 mm, less than 5.1 mm, less than 5.0 mm, less than 4.9 mm, less than 4.8 mm, less than 4.7 mm, less than 4.6 mm, less than 4.5 mm, less than 4.4 mm, less than 4.3 mm, less than 4.2 mm, less than 4.1 mm, less than 4.0 mm, less than 3.9 mm, less than 3.8 mm, less than 3.7 mm, less than 3.6 mm, less than 3.5 mm, less than 3.4 mm, less than 3.3 mm, less than 3.2 mm, less than 3.1 mm, less than 3.0 mm, less than 2.9 mm, less than 2.8 mm, less than 2.7 mm, less than 2.6 mm, less than 2.5 mm, less than 2.4 mm, less than 2.3 mm, less than 2.2 mm, less than 2.1 mm, less than 2.0 mm, less than 1.9 mm, less than 1.8 mm, less than 1.7 mm, less than 1.6 mm, less than 1.5 mm, less than 1.4 mm, less than 1.3 mm, less than 1.2 mm, less than 1.1 mm, less than 1.0 mm, less than 0.9 mm, less than 0.8 mm, less than 0.7 mm, less than 0.6 mm, less than 0.5 mm, less than 0.4 mm, less than 0.3 mm, less than 0.2 mm, less than 0.1 mm, less than 0.09 mm, less than 0.08 mm, less than 0.07 mm, less than 0.06 mm, less than 0.05 mm, less than 0.04 mm, less than 0.03 mm, less than 0.02 mm, less than 0.01 mm, less than 0.009 mm, less than 0.008 mm, less than 0.007 mm, less than 0.006 mm, less than 0.005 mm, less than 0.004 mm, less than 0.003 mm, less than 0.002 mm, less than 0.001 mm of the cerebral artery affected by the brain injury.

According to some embodiments, the pharmaceutical composition is capable of releasing the voltage gated calcium channel blocker at the site of delivery within a half-life ranging from 1 day to 30 days. According to one embodiment, the pharmaceutical composition is capable of releasing the voltage gated calcium channel blocker at the site of delivery within a half-life of 1 day. According to another embodiment, the pharmaceutical composition is capable of releasing the voltage gated calcium channel blocker at the site of delivery within a half-life of 2 days. According to another embodiment, the pharmaceutical composition is capable of releasing the voltage gated calcium channel blocker at the site of delivery within a half-life of 3 days. According to another embodiment, the pharmaceutical composition is capable of releasing the voltage gated calcium channel blocker within a half-life of 4 days. According to another embodiment, the pharmaceutical composition is capable of releasing the voltage gated calcium channel blocker at the site of delivery within a half-life of 5 days. According to another embodiment, the pharmaceutical composition is capable of releasing the voltage gated calcium channel blocker at the site of delivery within a half-life of 6 days. According to another embodiment, the pharmaceutical composition is capable of releasing the voltage gated calcium channel blocker at the site of delivery within a half-life of 7 days. According to another embodiment, the pharmaceutical composition is capable of releasing the voltage gated calcium channel blocker at the site of delivery within a half-life of 8 days. According to another embodiment, the pharmaceutical composition is capable of releasing the voltage gated calcium channel blocker at the site of delivery within a half-life of 9 days. According to another embodiment, the pharmaceutical composition is capable of releasing the voltage gated calcium channel blocker at the site of delivery within a half-life of 10 days. According to another embodiment, the pharmaceutical composition is capable of releasing the voltage gated calcium channel blocker at the site of delivery within a half-life of 15 days. According to another embodiment, the pharmaceutical composition is capable of releasing the voltage gated calcium channel blocker at the site of delivery within a half-life of 30 days.

***Therapeutic Effect***

According to another embodiment, implantation of the pharmaceutical composition into the injured brain can improve appetite.

According to another embodiment, implantation of the pharmaceutical composition into the injured brain can improve ataxia or paresis.

According to another embodiment, the release of the voltage-gated calcium channel blocker at the site of delivery can produce a predominantly localized pharmacologic effect over a desired amount of time. According to one embodiment, the release of the voltage-gated calcium channel blocker at the site of delivery produces a predominantly localized pharmacologic effect for 1 day. According to one embodiment, the release of the voltage-gated calcium channel blocker at the site of delivery produces a predominantly localized pharmacologic effect for 2 days. According to one embodiment, the release of the voltage-gated calcium channel blocker at the site of delivery produces a localized pharmacologic effect for 3 days. According to one embodiment, the release of the voltage-gated calcium channel blocker at the site of delivery produces a predominantly localized pharmacologic effect for 4 days. According to one embodiment, the release of the voltage-gated calcium channel blocker at the site of delivery produces a predominantly localized pharmacologic effect for 5 days. According to one embodiment, the release of the voltage-gated calcium channel blocker at the site of delivery produces a predominantly localized pharmacologic effect for 6 days. According to one embodiment, the release of the voltage-gated calcium channel blocker at the site of delivery produces a predominantly localized pharmacologic effect for 7 days. According to one embodiment, the release of the voltage-gated calcium channel blocker at the site of delivery produces a predominantly localized pharmacologic effect for 8 days. According to one embodiment, the release of the voltage-gated calcium channel blocker at the site of delivery produces a predominantly ocalized pharmacologic effect for 15 days. According to one embodiment, the release of the voltage-gated calcium channel blocker at the site of delivery produces a predominantly localized pharmacologic effect for 30 days.

According to another embodiment, the release of the voltage-gated calcium channel blocker at the site of delivery produces a diffuse pharmacologic effect throughout the central nervous system (CNS) over a desired amount of time. According to another embodiment, the release of the voltage-gated calcium channel blocker can produce a diffuse pharmacologic effect throughout the central nervous system (CNS) for 1 day. According to another embodiment, the release of the voltage-gated calcium channel blocker can produce a diffuse pharmacologic effect throughout the central nervous system (CNS) for 2 days. According to another embodiment, the release of the voltage-gated calcium channel blocker can produce a diffuse pharmacologic effect throughout the central nervous system (CNS) for 3 days. According to another embodiment, the release of the voltage-gated calcium channel blocker can produce a diffuse pharmacologic effect throughout the central nervous system (CNS) for 4 days. According to another embodiment, the release of the voltage-gated calcium channel blocker can produce a diffuse pharmacologic effect throughout the central nervous system (CNS) for 5 days. According to another embodiment, the release of the voltage-gated calcium channel blocker can produce a diffuse pharmacologic effect throughout the central nervous system (CNS) for 6 days. According to another embodiment, the release of the voltage-gated calcium channel blocker can produce a diffuse pharmacologic effect throughout the central nervous system (CNS) for 7 days. According to another embodiment, the release of the voltage-gated calcium channel blocker can produce a diffuse pharmacologic effect for throughout the central nervous system (CNS) 8 days. According to another embodiment, the release of the voltage-gated calcium channel blocker can produce a diffuse pharmacologic effect throughout the central nervous system (CNS) for 15 days. According to another embodiment, the release of the voltage-gated calcium channel blocker can produce a diffuse pharmacologic effect for throughout the central nervous system (CNS) 30 days.

According to one embodiment, the localized pharmacologic effect at the site of delivery is a reduction of vasospasm such that internal diameter of the at least one cerebral artery affected by the brain injury is increased compared to a control. According to one embodiment, the pharmaceutical composition is effective to increase the internal diameter of the cerebral artery affected by the brain injury as compared to a control.

According to one embodiment, the diffuse pharmacologic effect is a reduction of vasospasm such that internal diameter of a blood vessel that is within at least 10 mm, at least 9.9 mm, at least 9.8 mm, at least 9.7 mm, at least 9.6 mm, at least 9.5 mm, at least 9.4 mm, at least 9.3 mm, at least 9.2 mm, at least 9.1 mm, at least 9.0 mm, at least 8.9 mm, at least 8.8 mm, at least 8.7 mm, at least 8.6 mm, at least 8.5 mm, at least 8.4 mm, at least 8.3 mm, at least 8.2 mm, at least 8.1 mm, at least 8.0 mm, at least 7.9 mm, at least 7.8 mm, at least 7.7 mm, at least 7.6 mm, at least 7.5 mm, at least 7.4 mm, at least 7.3 mm, at least 7.2 mm, at least 7.1 mm, at least 7.0 mm, at least 6.9 mm, at least 6.8 mm, at least 6.7 mm, at least 6.6 mm, at least 6.5 mm, at least 6.4 mm, at least 6.3 mm, at least 6.2 mm, at least 6.1 mm, at least 6.0 mm, at least 5.9 mm, at least 5.8 mm, at least 5.7 mm, at least 5.6 mm, at least 5.5 mm, at least 5.4 mm, at least 5.3 mm, at least 5.2 mm, at least 5.1 mm, at least 5.0 mm from the site of delivery is increased as compared to a control.

According to some embodiments, the pharmaceutical composition is injected into the cerebral ventricles via a catheter or tube inserted into one of the lateral, third, or fourth ventricles, or the subarachnoid cisterns of the brain.

### *Voltage-gated Calcium Channel Blocker*

According to some embodiments, the voltage-gated channel blocker is selected from the group consisting of L-type voltage-gated calcium channel blocker, N-type voltage-gated calcium channel blocker, P/Q-type voltage-gated calcium channel blocker, or a combination thereof.

Non-limiting examples of the voltage-gated calcium channel blocker that can be formulated into the composition include, but are not limited to, L-type voltage-gated calcium channel blocker, N-type voltage-gated calcium channel blocker, P/Q-type voltage-gated calcium channel blocker, or a combination thereof.

For example, L-type voltage-gated calcium channel blockers include, but are not limited to: dihydropyridine L-type blockers such as nisoldipine, nicardipine and nifedipine, AHF (such as 4aR,9aS)-(+)-4a-Amino-1,2,3,4,4a,9a-hexahydro-4aH-fluorene, HCl), isradipine (such as 4-(4-Benzofurazanyl)-1,-4-dihydro-2,6-dimethyl-3,5-pyridinedicarboxylic acid methyl 1-methhylethyl ester), calciseptine (such as isolated from (Dendroaspis polylepis ploylepis), H-Arg-Ile-Cys-Tyr-Ile-His-Lys-Ala-Ser-Leu-Pro-Arg-Ala-Thr-Lys-Thr-Cys-Val-    Glu-Asn-Thr-Cys-Tyr-Lys-Met-Phe-Ile-Arg-Thr-Gln-Arg-Glu-Tyr-Ile-Ser-Glu-Arg-Gly-Cys-    Gly-Cys-Pro-Thr-Ala-Met-Trp-Pro-Tyr-Gln-Thr-Glu-Cys-Cys-Lys-Gly-Asp-Arg-Cys-Asn-Lys-OH, Calcicludine (such as isolated from Dendroaspis angusticeps (eastern green mamba)),(H-Trp-Gln-Pro-Pro-  Trp-Tyr-Cys-Lys-Glu-Pro-Val-Arg-Ile-Gly-Ser-Cys-Lys-Lys-Gln-Phe-Ser-Ser-Phe-Tyr-  Phe-Lys-Trp-Thr-Ala-Lys-Lys-Cys-Leu-Pro-Phe-Leu-Phe-Ser-Gly-Cys-Gly-Gly-Asn-Ala-Asn-Arg-Phe-Gln-Thr-Ile-Gly-Glu-Cys-Arg-Lys-Lys-Cys-Leu-Gly-Lys-OH, Cilnidipine (such as also FRP-8653, a dihydropyridine-type inhibitor), Dilantizem (such as (2S,3S)-(+)-cis-3-Acetoxy-5-(2-dimethylaminoethyl)-2,3-dihydro-2-(4-methoxyphenyl)-1,5-benzothiazepin-4(5H)-one hydrochloride), diltiazem (such as benzothiazepin-4(5H)-one, 3-(acetyloxy)-5-[2-(dimethylamino)ethyl]-2,3-dihydro-2-(4-methoxyphenyl)--,(+)- cis-, monohydrochloride), Felodipine (such as 4-(2,3-Dichlorophenyl)-1,4-dihydro-2,6-dimethyl-3,5-pyridinecarboxylic acid ethyl methyl ester), FS-2 (such as an isolate from Dendroaspis polylepis polylepis venom), FTX-3.3 (such as an isolate from Agelenopsis aperta), Neomycin sulfate (such as $C_{23}H_{46}N_6O_{13} \cdot 3H_2SO_4$), Nicardipine (such as 1,4- Dihydro-2,6-dimethyl -4-(3-nitrophenypmethyl-2-[methyl(phenylmethypa- mino]-3,5- pyridinedicarboxylic acid ethyl ester hydrochloride, also YC-93, Nifedipine (such as 1,4- Dihydro-2,6-dimethyl-4-(2-nitrophenyl)-3,5-pyridinedicarboxylic acid dimethyl ester), Nimodipine (such as 4-Dihydro-2,6-dimethyl-4-(3-nitrophenyl)-3,5-pyridinedicarboxylic acid 2-methoxyethyl 1-methylethyl ester) or (Isopropyl 2-methoxyethyl 1,4-dihydro-2,6-dimethyl-4-(m¬nitrophenyl)-3,5-pyridinedicarboxylate), Nitrendipine (such as 1,4-Dihydro-2,6-dimethyl-4-(3- nitrophenyl)-3,5-pyridinedicarboxylic acid ethyl methyl ester), S-Petasin (such as (3 S,4aR,5R,6R)-[2,3,4,4a,5,6,7,8-Octahydro-3-(2-propenyl)-4a,5-dimethyl-2-o- xo-6-naphthyl]Z¬3'-methylthio-1'-propenoate), Phloretin (such as 2',4',6'-Trihydroxy-3 -(4-hydroxyphenyl)propiophenone, also 3-(4-Hydroxyphenyl)-1-(2,4,6-trihydroxyphenyl)-1-propanone, also b-(4-Hydroxyphenyl)-2,4,6-trihydroxypropiophenone), Protopine (such as $C_{20}H_{19}NO_5Cl$), SKF-96365 (such as 1-[b-[3-(4-Methoxyphenyl)propoxy]-4-methoxyphenethyl]-1H-imidazole, HCl), Tetrandine (such as 6,6',7,12-Tctramcthoxy-2,2'-dimethylberbaman), (.+-.)-Methoxyverapamil or (+)-Verapamil (such as 54N-(3,4-Dimethoxyphenylethyl)methylamino]-2-(3,4-dimethoxyphenyl)-2-iso- propylvaleronitrile hydrochloride), and (R)-(+)-Bay K8644 (such as R-(+)-1,4-Dihydro-2,6-dimethyl-5-nitro-442- (trifluoromethyl)phenyl]-3-pyridinecarboxylic acid methyl ester). The foregoing examples may be specific to L-type voltage-gated calcium channels or may inhibit a broader range of voltage- gated calcium channels, e.g. N, P/Q, R, and T-type.

According to some embodiments, the voltage-gated calcium channel blocker is a dihydropyridine calcium channel blocker. According to one embodiment, the dihydropyridine calcium channel blocker is nimodipine. According to one embodiment, the nimodipine has a half life of 7-10 days when formulated as described herein, and appropriate lipid solubility.

According to some embodiments, the voltage gated calcium channel blocker is an isolated molecule. The term "isolated molecule" as used herein refers to a molecule that is substantially pure and is free of other substances with which it is ordinarily found in nature or in vivo systems to an extent practical and appropriate for its intended use.

According to some embodiments, the voltage gated calcium channel blocker is admixed with a pharmaceutically-acceptable carrier in a pharmaceutical preparation. According to some such embodiments, the voltage gated calcium channel blocker comprises only a small percentage by weight of the preparation. According to some embodiments, the voltage gated calcium channel blocker is substantially pure.

***Pharmaceutically acceptable carrier***

According to some embodiments, the pharmaceutical composition does not comprise the pharmaceutically acceptable carrier. According to some embodiments, the pharmaceutically acceptable carrier does not comprise hyaluronic acid.

According to one embodiment, the pharmaceutically acceptable carrier is a solid carrier or excipient. According to another embodiment, the pharmaceutically acceptable carrier is a gel-phase carrier or excipient. Examples of carriers or excipients include, but are not limited to, calcium carbonate, calcium phosphate, various monomeric and polymeric sugars (including without limitation hyaluronic acid), starches, cellulose derivatives, gelatin, and polymers. An exemplary carrier can also include saline vehicle, e.g. hydroxyl propyl methyl cellulose (HPMC) in phosphate buffered saline (PBS).

According to some embodiments, the pharmaceutically acceptable carrier imparts stickiness. According to one embodiment, the pharmaceutically acceptable carrier comprises hyaluronic acid. According to some embodiments, the pharmaceutically acceptable carrier comprises 0 % to 5 % hyaluronic acid. According to one embodiment, the pharmaceutically acceptable carrier comprises less than 0.05 % hyaluronic acid. According to another embodiment, the pharmaceutically acceptable carrier comprises less than 0.1 % hyaluronic acid. According to another embodiment, the pharmaceutically acceptable carrier comprises less than 0.2 % hyaluronic acid. According to another embodiment, the pharmaceutically acceptable carrier comprises less than 0.3 % hyaluronic acid. According to another embodiment, the pharmaceutically acceptable carrier comprises less than 0.4 % hyaluronic acid. According to another embodiment, the pharmaceutically acceptable carrier comprises less than 0.5 % hyaluronic acid. According to another embodiment, the pharmaceutically acceptable carrier comprises less than 0.6 % hyaluronic acid. According to another embodiment, the pharmaceutically acceptable carrier comprises less than 0.7 % hyaluronic acid. According to another embodiment, the pharmaceutically acceptable carrier comprises less than 0.8 % hyaluronic acid. According to another embodiment, the pharmaceutically acceptable carrier comprises less than 0.9 % hyaluronic acid. According to another embodiment, the pharmaceutically acceptable carrier comprises less than 1.0 % hyaluronic acid. According to another embodiment, the pharmaceutically acceptable carrier comprises less than 1.1 % hyaluronic acid. According to another embodiment, the pharmaceutically acceptable carrier comprises less than 1.2 % hyaluronic acid. According to another embodiment, the pharmaceutically acceptable carrier comprises less than 1.3% hyaluronic acid. According to another embodiment, the pharmaceutically acceptable carrier comprises less than 1.4 % hyaluronic acid. According to another embodiment, the pharmaceutically acceptable carrier comprises less than 1.5% hyaluronic acid. According to another embodiment, the pharmaceutically acceptable carrier comprises less than 1.6 % hyaluronic acid. According to another embodiment, the pharmaceutically acceptable carrier comprises less than 1.7 % hyaluronic acid. According to another embodiment, the pharmaceutically acceptable carrier comprises less than 1.8% hyaluronic acid. According to another embodiment, the pharmaceutically acceptable carrier comprises less than 1.9% hyaluronic acid. According to another embodiment, the pharmaceutically acceptable carrier comprises less than 2.0 % hyaluronic acid. According to another embodiment, the pharmaceutically acceptable carrier comprises less than 2.1 % hyaluronic acid. According to another embodiment, the pharmaceutically acceptable carrier comprises less than 2.2 % hyaluronic acid. According to another embodiment, the pharmaceutically acceptable carrier comprises less than 2.3% hyaluronic acid. According to another embodiment, the pharmaceutically acceptable carrier comprises less than 2.4 % hyaluronic acid. According to another embodiment, the pharmaceutically acceptable carrier comprises less than 2.5 % hyaluronic acid. According to another embodiment, the pharmaceutically acceptable carrier comprises less than 2.6 % hyaluronic acid. According to another embodiment, the pharmaceutically acceptable carrier comprises less than 2.7 % hyaluronic acid. According to another embodiment, the pharmaceutically acceptable carrier comprises less than 2.8 % hyaluronic acid. According to another embodiment, the pharmaceutically acceptable carrier comprises less than 2.9 % hyaluronic acid. According to another embodiment, the pharmaceutically acceptable carrier comprises less than 3.0 % hyaluronic acid. According to another embodiment, the pharmaceutically acceptable carrier comprises less than 3.5 % hyaluronic acid. According to another embodiment, the pharmaceutically acceptable carrier comprises less than 4.0 % hyaluronic acid. According to another embodiment, the pharmaceutically acceptable carrier comprises less than 4.5 % hyaluronic acid. According to another embodiment, the pharmaceutically acceptable carrier comprises less than 5.0 % hyaluronic acid.

In some embodiments, the pharmaceutically acceptable carrier includes, but is not limited to, a gel, slow-release solid or semisolid compound, optionally as a sustained release gel. In some such embodiments, the voltage-gated calcium channel blocker is embedded into the pharmaceutically acceptable carrier. In some embodiments, the voltage-gated calcium channel blocker is coated on the pharmaceutically acceptable carrier. The coating can be of any desired material, preferably a polymer or mixture of different polymers. Optionally, the polymer can be utilized during the granulation stage to form a matrix with the active ingredient so as to obtain a desired release pattern of the active ingredient. The gel, slow-release solid or semisolid compound is capable of releasing the active agent over a desired period of time. The gel, slow-release solid or semisolid compound can be implanted in a tissue within human brain, for example, but not limited to, in close proximity to a blood vessel, such as a cerebral artery.

According to another embodiment, the pharmaceutically acceptable carrier comprises a slow-release solid compound. According to one such embodiment, the voltage-gated calcium channel blocker is embedded in the slow-release solid compound or coated on the slow-release solid compound. According to yet another embodiment, the pharmaceutically acceptable carrier comprises a slow-release microparticle containing the voltage-gated calcium channel blocker.

According to another embodiment, the pharmaceutically acceptable carrier is a gel compound, such as a biodegradable hydrogel.

## *Microparticle Formulation*

According to some embodiments, the voltage-gated calcium channel blocker is provided in the form of a particle. The term "particle" as used herein refers to nano or microparticles (or in some instances larger) that may contain in whole or in part the calcium channel blocker. According to some embodiments, the microparticulate formulation comprises a plurality of microparticles impregnated with the voltage-gated calcium channel blocker. According to one embodiment, the voltage-gated calcium channel blocker is contained within the core of the particle surrounded by a coating. According to another embodiment, the voltage-gated calcium channel blocker is dispersed throughout the surface of the particle. According to another embodiment, the voltage-gated calcium channel blocker is disposed on or in the microparticle. According to another embodiment, the voltage-gated calcium channel blocker is disposed throughout the surface of the microparticle. According to another embodiment, the voltage-gated calcium channel blocker is adsorbed into the particle.

According to some such embodiments, the microparticles are of uniform size distribution. According to some embodiments, the uniform distribution of microparticle size is achieved by a homogenization process to form a uniform emulsion comprising microparticles. According to some such embodiments, each microparticle comprises a matrix. According to some embodiments, the matrix comprises the voltage gated calcium channel blocker.

According to some embodiments, the pharmaceutical composition is flowable. According to some embodiments, the microparticulate formulation component of the pharmaceutical composition is flowable.

According to some embodiments, the particle is selected from the group consisting of a zero order release, first order release, second order release, delayed release, sustained release, immediate release, and a combination thereof. The particle can include, in addition to the therapeutic agent(s), any of those materials routinely used in the art of pharmacy and medicine, including, but not limited to, erodible, nonerodible, biodegradable, or nonbiodegradable material or combinations thereof

According to some embodiments, the particle is a microcapsule that contains the voltage-gated calcium channel blocker in a solution or in a semi-solid state. According to some embodiments, the particle is a microparticle that contains the voltage-gated calcium channel blocker, in whole or in part. According to some embodiments, the particle is a nanoparticle that contains the voltage-gated calcium channel blocker, in whole or in part. According to some embodiments, the particles can be of virtually any shape.

According to some embodiments, the particle size is between about 25 $\mu$m to about 100 $\mu$m. According to some embodiments, the particle size is between about 30 $\mu$m to about 80 $\mu$m. According to one embodiment, the particle size is at least about 25 $\mu$m. According to another embodiment, the particle size is at least about 30 $\mu$m. According to another embodiment, the particle size is at least about 35 $\mu$m. According to another embodiment, the particle size is at least about 40 $\mu$m. According to another embodiment, the particle size is at least about 45 $\mu$m. According to another embodiment, the particle size is at least about 50 $\mu$m. According to another embodiment, the particle size is at least about 55 $\mu$m. According to another embodiment, the particle size is at least about 60 $\mu$m. According to another embodiment, the particle size is at least about 65 $\mu$m. According to another embodiment, the particle size is at least about 70 $\mu$m. According to another embodiment, the particle size is at least about 75 $\mu$m. According to another embodiment, the particle size is at least about 80 $\mu$m. According to another embodiment, the particle size is at least about 85 $\mu$m. According to another embodiment, the particle size is at least about 90 $\mu$m. According to another embodiment, the particle size is at least about 95 $\mu$m. According to another embodiment, the particle size is at least about 100 $\mu$m.

According to another embodiment, the voltage gated calcium channel blocker can be provided in strings. The strings can contain the voltage-gated calcium channel blocker in a core surrounded by a coating, or the voltage-gated calcium channel blocker can be dispersed throughout the string, or therapeutic agent(s) may be absorbed into the string. The string can be of any order release kinetics, including zero order release, first order release, second order release, delayed release, sustained release, immediate release, etc., and any combination thereof. The string can include, in addition to therapeutic agent(s), any of those materials routinely used in the art of pharmacy and medicine, including, but not limited to, erodible, nonerodible, biodegradable, or nonbiodegradable material or combinations thereof.

According to another embodiment, the voltage-gated calcium channel blocker can be provided in at least one sheet. The sheet can contain the voltage-gated calcium channel blocker and at least one additional therapeutic agent in a core surrounded by a coating, or the voltage-gated calcium channel blocker and at least one additional therapeutic agent can be dispersed throughout the sheet, or the voltage-gated calcium channel blocker can be absorbed into the sheet. The sheet can be of any order release kinetics, including zero order release, first order release, second order release, delayed release, sustained release, immediate release, etc., and any combination thereof. The sheet can include, in addition to the voltage-gated calcium channel blocker and at least one additional therapeutic agent, any of those materials routinely used in the art of pharmacy and medicine, including, but not limited to, erodible, nonerodible, biodegradable, or nonbiodegradable material or combinations thereof.

According to some embodiments, the pharmaceutical composition further comprises a preservative agent. According to some such embodiments, the pharmaceutical composition is presented in a unit dosage form. Exemplary unit dosage forms include, but arc not limited to, ampoules or multi-dose containers.

According to some embodiments, the microparticulate formulation comprises a suspension of microparticles. According to some embodiments, the pharmaceutical composition further comprises at least one of a suspending agent, a stabilizing agent and a dispersing agent. According to some such embodiments, the pharmaceutical composition is presented as a suspension. According to some such embodiments, the pharmaceutical composition is presented as a solution. According to some such embodiments, the pharmaceutical composition is presented as an emulsion.

According to some embodiments, a formulation of the pharmaceutical composition comprises an aqueous solution of the voltage-gated calcium channel blocker in water-soluble form. According to some embodiments, the formulation of the pharmaceutical composition comprises an oily suspension of the voltage-gated calcium channel blocker. Oily suspension of the voltage-gated calcium channel blocker can be prepared using suitable lipophilic solvents. Exemplary lipophilic solvents or vehicles include, but are not limited to, fatty oils such as sesame oil, or synthetic fatty acid esters, such as ethyl oleate or triglycerides. According to some embodiments, the formulation of the pharmaceutical composition comprises an aqueous suspension of the volgate-gated calcium channel blocker. Aqueous injection suspensions can contain substances which increase the viscosity of the suspension, such as sodium carboxymethyl cellulose, sorbitol, or dextran. Optionally, the suspension can also contain suitable stabilizers or agents which increase the solubility of the compounds to allow for the preparation of highly concentrated solutions. Alternatively, the volgate-gated calcium channel blocker can be in powder form for constitution with a suitable vehicle, e.g., sterile pyrogen-free water, before use.

Suitable liquid or solid pharmaceutical preparations include, for example, microencapsulated dosage forms, and if appropriate, with one or more excipients, encochleated, coated onto microscopic gold particles, contained in liposomes, pellets for implantation into the tissue, or dried onto an object to be rubbed into the tissue. As used herein, the term "microencapsulation" refers to a process in which very tiny droplets or particles are surrounded or coated with a continuous film of biocompatible, biodegradable, polymeric or non-polymeric material to produce solid structures including, but not limited to, nonpareils, pellets, crystals, agglomerates, microspheres, or nanoparticlcs. Such pharmaceutical compositions also can be in the form of granules, beads, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, or solubilizers are customarily used as described above. The pharmaceutical compositions are suitable for use in a variety of drug delivery systems. For a brief review of methods for drug delivery, see Langer (1990) Science 249, 1527-1533, which is incorporated herein by reference.

*Microencapsulation Process*

Examples of microencapsulation processes and products; methods for the production of emulsion-based microparticles; emulsion-based microparticles and methods for the production thereof; solvent extraction microencapsulation with tunable extraction rates; microencapsulation process with solvent and salt; a continuous double emulsion process for making microparticles; drying methods for tuning microparticle properties, controlled release systems from polymer blends; polymer mixtures comprising polymers having different non-repeating units and methods for making and using the same; and an emulsion based process for preparing microparticles and workhead assembly for use with same are disclosed and described in U.S. Patent No. 5, 407,609 (entitled Microencapsulation Process and Products Thereof), U.S. Application No. 10/553,003 (entitled Method for the production of emulsion-based microparticles), U.S. Application No. 11/799,700 (entitled Emulsion-based microparticles and methods for the production thereof), U.S. Application No. 12/557,946 (entitled Solvent Extraction Microencapsulation With Tunable Extraction Rates), U.S. Application No. 12/779,138 (entitled Hyaluronic Acid (HA) Injection Vehicle), U.S. Application No. 12/562,455 entitled Microencapsulation Process With Solvent And Salt), U.S. Application No. 12/338,488 (entitled Process For Preparing Microparticles Having A Low Residual Solvent Volume); U.S. Application No. 12/692,027 (entitled Controlled Release Systems From Polymer Blends); U.S. Application No. 12/692,020 (entitled Polymer Mixtures Comprising Polymers Having Different Non-Repeating Units And Methods For Making And Using Same); U.S. Application No. 10/565,401 (entitled "Controlled release compositions"); U.S. Application No. 12/692,029 (entitled "Drying Methods for Tuning Microparticle Properties); U.S. Application No. 12/968,708 (entitled "Emulsion Based Process for Preparing Microparticles and Workhead for Use with Same); and U.S. Application No. 13/074,542 (entitled Composition and Methods for Improved Retention of a Pharmaceutical Composition at a Local Administration Site") The contents of each of these are incorporated herein by reference in its entirety.

According to some embodiments, delivery of the voltage-gated calcium channel blocker using microparticle technology involves bioresorbable, polymeric particles that encapsulate the voltage-gated calcium channel blocker and at least one additional therapeutic agent.

According to one embodiment, the microparticle formulation comprises a polymer matrix, wherein the voltage-gated calcium channel blocker is impregnated in the polymer matrix. According to one embodiment, the polymer is a slow release polymer. According to one embodiment, the polymer is poly (D, L-Lactide-co-glycolide). According to another embodiment, the polymer is poly(orthoester). According to another embodiment, the polymer is poly(anhydride). According to another embodiment, the polymer is polylactide-polyglycolide.

Both non-biodegradable and biodegradable polymeric materials can be used in the manufacture of particles for delivering the voltage-gated calcium channel blocker. Such polymers can be natural or synthetic polymers. The polymer is selected based on the period of time over which release is desired. Bioadhesive polymers of particular interest include, but are not limited to, bioerodible hydrogels as described by Sawhney et al in Macromolecules (1993) 26, 581-587, the teachings of which are incorporated herein. Exemplary bioerodible hydrogels include, but are not limited to, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate). According to one embodiment, the bioadhesive polymer is hyaluronic acid. In some such embodiments, the bioadhesive polymer include less than about 2.3 % of hyaluronic acid.

According to some embodiments, the pharmaceutical composition is formulated for parenteral injection, surgical implantation, or a combination thereof. According to some such embodiments, the pharmaceutical composition is in the form of a pharmaceutically acceptable sterile aqueous or nonaqueous solution, dispersion, suspension or emulsion or a sterile powder for reconstitution into a sterile injectable solution or dispersion. Examples of suitable aqueous and nonaqueous carriers, diluents, solvents or vehicles include, but are not limited to, water, ethanol, dichloromethane, acetonitrile, ethyl acetate, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants. Suspensions can further contain suspending agents, as, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

According to some embodiments, the pharmaceutical composition is formulated in an injectable depot form. Injectable depot forms are made by forming microencapsulated matrices of the voltage gated calcium channel blocker in a biodegradable polymer. Depending upon the ratio of drug to polymer and the nature of the particular polymer employed, the rate of drug release may be controlled. Such long acting formulations can be formulated with suitable polymeric or hydrophobic materials (for example as an emulsion in an acceptable oil) or ion exchange resins, or as sparingly soluble derivatives, for example, as a sparingly soluble salt. Examples of biodegradable polymers include, but are not limited to, polylactide-polyglycolide, poly(orthoesters) and poly(anhydrides). Depot injectable formulations are also prepared by entrapping the drug in liposomes or microemulsions which are compatible with body tissues.

According to some embodiments, the voltage-gated calcium channel blocker is impregnated in or on a polyglycolide (PGA) matrix. PGA is a linear aliphatic polyester developed for use in sutures. Studies have reported PGA copolymers formed with trimethylene carbonate, polylactic acid (PLA), and polycaprolactone. Some of these copolymers may be formulated as microparticles for sustained drug release.

According to some embodiments, the voltage-gated calcium channel blocker is impregnated in or on a polyester-polyethylene glycol matrix. Polyester-polyethylene glycol compounds can be synthesized; these are soft and may be used for drug delivery.

According to some embodiments, the voltage-gated calcium channel blocker is impregnated in or on a poly (amino)-derived biopolymer matrix. Poly (amino)-derived biopolymers can include, but are not limited to, those containing lactic acid and lysine as the aliphatic diamine (see, for example, U.S. Patent 5,399,665), and tyrosine-derived polycarbonates and polyacrylates. Modifications of polycarbonates may alter the length of the alkyl chain of the ester (ethyl to octyl), while modifications of polyarylates may further include altering the length of the alkyl chain of the diacid (for example, succinic to sebasic), which allows for a large permutation of polymers and great flexibility in polymer properties.

According to some embodiments, the voltage-gated calcium channel blocker is impregnated in or on a polyanhydride matrix. Polyanhydrides are prepared by the dehydration of two diacid molecules by melt polymerization (see, for example, U.S. Patent 4,757,128). These polymers degrade by surface erosion (as compared to polyesters that degrade by bulk erosion). The release of the drug can be controlled by the hydrophilicity of the monomers chosen.

According to some embodiments, the voltage-gated calcium channel blocker is impregnated in or on a photopolymerizable biopolymer matrix. Photopolymerizable biopolymers include, but are not limited to, lactic acid/polyethylene glycol/acrylate copolymers.

According to some embodiments, the the voltage-gated calcium channel blocker is impregnated in or on a hydrogel matrix. The term "hydrogel" refers to a substance resulting in a solid, semisolid, pseudoplastic or plastic structure containing a necessary aqueous component to produce a gelatinous or jelly-like mass. Hydrogels generally comprise a variety of polymers, including hydrophilic polymers, acrylic acid, acrylamide and 2-hydroxyethylmethacrylate (HEMA).

According to some embodiments, the voltage-gated calcium channel blocker is impregnated in or on a naturally-occurring biopolymer matrix. Naturally-occurring biopolymers include, but are not limited to, protein polymers, collagen, polysaccharides, and photopolymerizable compounds.

According to some embodiments, the voltage-gated calcium channel blocker is impregnated in or on a protein polymer matrix. Protein polymers have been synthesized from self-assembling protein polymers such as, for example, silk fibroin, elastin, collagen, and combinations thereof.

According to some embodiments, the the voltage-gated calcium channel blocker is impregnated in or on a naturally-occurring polysaccharide matrix. Naturally-occurring polysaccharides include, but are not limited to, chitin and its derivatives, hyaluronic acid, dextran and cellulosics (which generally are not biodegradable without modification), and sucrose acetate isobutyrate (SAIB).

According to some embodiments, the the voltage-gated calcium channel blocker is impregnated in or on a chitin matrix. Chitin is composed predominantly of 2-acetamido-2-deoxy-D-glucose groups and is found in yeasts, fungi and marine invertebrates (shrimp, crustaceous) where it is a principal component of the exoskeleton. Chitin is not water soluble and the deacetylated chitin, chitosan, only is soluble in acidic solutions (such as, for example, acetic acid). Studies have reported chitin derivatives that are water soluble, very high molecular weight (greater than 2 million daltons), viscoelastic, non-toxic, biocompatible and capable of crosslinking with peroxides, gluteraldehyde, glyoxal and other aldehydes and carbodiamides, to form gels.

According to some embodiments, the the voltage-gated calcium channel blocker is impregnated in or on a hyaluronic acid (HA) matrix. Hyaluronic acid (HA), which is composed of alternating glucuronidic and glucosaminidic bonds and is found in mammalian vitreous humor, extracellular matrix of the brain, synovial fluid, umbilical cords and rooster combs from which it is isolated and purified, also can be produced by fermentation processes.

According to some embodiments, the pharmaceutical composition further comprises an adjuvant. Exemplary adjuvants include, but are not limited to, preservative agents, wetting agents, emulsifying agents, and dispersing agents. Prevention of the action of microorganisms can be ensured by various antibacterial and antifungal agents, for example, parabens, chlorobutanol, phenol, sorbic acid, and the like. Isotonic agents, for example, sugars, sodium chloride and the like, can also be included. Prolonged absorption of the injectable pharmaceutical form can be brought about by the use of agents delaying absorption, for example, aluminum monostearate and gelatin.

The formulations can be sterilized, for example, by terminal gamma irradiation, filtration through a bacterial-retaining filter or by incorporating sterilizing agents in the form of sterile solid compositions that may be dissolved or dispersed in sterile water or other sterile injectable medium just prior to use. Injectable preparations, for example, sterile injectable aqueous or oleaginous suspensions may be formulated according to the known art using suitable dispersing or wetting agents and suspending agents. The sterile injectable preparation also may be a sterile injectable solution, suspension or emulsion in a nontoxic, parenterally acceptable diluent or solvent such as a solution in 1,3-butanediol, dichloromethane, ethyl acetate, acetonitrile, etc. Among the acceptable vehicles and solvents that may be employed are water, Ringer's solution, U.S.P. and isotonic sodium chloride solution. In addition, sterile, fixed oils conventionally are employed or as a solvent or suspending medium. For this purpose any bland fixed oil may be employed including synthetic mono- or diglycerides. In addition, fatty acids such as oleic acid are used in the preparation of injectables.

Formulations for parenteral (including but not limited to, subcutaneous, intradermal, intramuscular, intravenous, intraarterial, intrathecal, intraventricular and intraarticular) administration include aqueous and non-aqueous sterile injection solutions that can contain anti-oxidants, buffers, bacteriostats and solutes, which render the formulation isotonic with the blood of the intended recipient; and aqueous and non-aqueous sterile suspensions, which can include suspending agents and thickening agents.

According to another embodiment, the pharmaceutical composition is formulated by conjugating the voltage-gated calcium channel blocker to a polymer that enhances aqueous solubility. Examples of suitable polymers include but are not limited to polyethylene glycol, poly-(d-glutamic acid), poly-(1-glutamic acid), poly-(1-glutamic acid), poly-(d-aspartic acid), poly-(1-aspartic acid), poly-(1-aspartic acid) and copolymers thereof. Polyglutamic acids having molecular weights between about 5,000 to about 100,000, with molecular weights between about 20,000 and about 80,000 may be used and with molecular weights between about 30,000 and about 60,000 may also be used. The polymer is conjugated via an ester linkage to one or more hydroxyls of an inventive epothilone using a protocol as essentially described by U.S. Pat. No. 5,977,163 which is incorporated herein by reference. Particular conjugation sites include the hydroxyl off carbon-21 in the case of 21-hydroxy-derivatives of the present invention. Other conjugation sites include, but are not limited, to the hydroxyl off carbon 3 and/or the hydroxyl off carbon 7.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

As used herein, the singular forms "a", "an" and "the" include plural referents unless the context clearly dictates otherwise. For example, reference to a "polypeptide" means one or more polypeptides.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and described the methods and/or materials in connection with which the publications are cited.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

## EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g., amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

**Example 1. Pilot Study 1 - Effect of Nimodipine formulation on cerebral vasospasm in a subarachnoid hemorrhage (SAH) Dog model**

**Materials and Methods**

*Formulation*

The test formulation of the microparticulate nimodipine formulation containing a uniform distribution of microparticle size was prepared by combining a polymer solution (e.g., a 50-50 glycolide-lactide blend) with a solvent in the presence of nimodipine. The mixture was added to a surfactant containing aqueous solution to form an emulsion and the solvent extracted to produce the flowable microparticulate nimodipine formulation. The initial drug load was 65%, i.e., 65% nimodipine and 35% polymer. The mean particle size was about 52 $\mu$.

The microparticulate nimodipine formulation was combined with a pharmaceutical carrier to form the pharmaceutical composition of the described invention. When the means for delivery is a surgical injection apparatus and the delivery site is within close proximity of a cerebral artery in a subarachnoid space, a vehicle (e.g. saline (hydroxyl propyl methyl cellulose (HPMC) in phosphate buffered saline (PBS))) is mixed with the microparticulate nimodipine formulation. The placebo formulation contained the microparticles created with no nimodipine plus vehicle.

*Treatment Groups*

A total of 6 male dogs were assigned to the study as presented in Table 1.

**Table 1. Treatment group assignments**

| Table 1. Group Assignments (Study 1) | | |
|---|---|---|
| Group Number | Treatment | Number of Male Animals |
| 1 | Intracisternal administration of Microparticulate Placebo Formulation plus saline vehicle | 2 |
| 2 | Intracisternal administration of Microparticulate Nimodipine Formulation at low dose[a] plus saline vehicle | 2 |
| 3 | Intracisternal administration of Microparticulate Nimodipine Formulation at high dose[b] plus saline vehicle | 2 |
| [a]10 mg delivered dose [b]30 mg delivered dose | | |

*Administration*

The control (Microparticulate Placebo Formulation) and test articles (Microparticulate Nimodipine Formulation at Low Dose or Microparticulate Nimodipine Formulation at High Dose) were administered once during surgery on Day 1 via injection into the cisterna magna (the enlarged subarachnoid space between the caudal surface of the cerebellum and the dorsal surface of the medulla oblongata). The dose levels for the treated groups were 10 mg or 30 mg at a fixed dose volume of 0.25 mL (Microparticulate Placebo Formulation), 0.17 mL or 0.18 mL (Microparticulate Nimodipine Formulation at Low Dose), or 0.46 mL (Microparticulate Nimodipine Formulation at High Dose). The syringes provided were loaded with 16 and 40 mg of Microparticulate Nimodipine Formulation at Low Dose and Microparticulate Nimodipine Formulation at High Dose, respectively; this took into account the overfill needed to fill the dead volume in the delivery system. As the materials were administered per the reconstitution/injection procedure the delivered doses were approximately 10 mg and 30 mg. The control group received the control article (Microparticulate Placebo Formulation) in the same manner as the treated groups.

For reconstitution/injection, a syringe comprising diluent was attached via a connector to a syringe comprising the microparticulate nimodipine formulation. A plunger is cycled to draw the vehicle into the microparticulate formulation. The resulting pharmaceutical composition is then pushed into the left syringe, which is disconnected from the connector. For delivery, the composition is injectable either through a surgical needle or can be fitted with or injected through a cannula or catheter of any appropriate size.

**Surgical Procedures**

On Day 1, the dogs were weighed, baseline blood was collected and blood pressure, temperature, heart rate oxygen, and blood gases were monitored throughout the surgical procedure. Cerebral angiography was performed through one vertebral artery. Images were captured using identical exposure factors and magnification for every angiogram. An internal magnification standard was included in every angiogram.

Following angiography, the animals were turned prone and the cisterna magna was punctured percutaneously with an 18 gauge spinal needle. A targeted volume of 0.3 mL/kg CSF was allowed to drain spontaneously, after which 0.5 mL/kg of fresh, autologous, arterial, non-heparinized blood was withdrawn from the femoral catheter and injected into the cisterna magna at a rate of approximately 5 mL/minute. Approximately one half of the blood volume was injected, then the placebo or nimodipine formulation was administered at a rate of approximately 5 mL/minute. Upon completion of administration of microparticulate placebo or microparticulate nimodipine formulations (low dose and high dose), the remaining blood was injected. The needle was withdrawn immediately after the injection. The animal was tilted 30° head down during cisternal blood injection and remained in that position for 15 minutes following completion of injection. The animal was then turned supine, the femoral catheter was removed, and the femoral artery was ligated. The incision was closed in a standard fashion.

On day 3, the dogs were placed under general anesthesia and the cisternal blood injection was repeated. On days 8 and 15, the animals were anesthetized, and angiography and removal of CSF from the cistern magna was repeated. After angiography on day 15, animals were not recovered from anesthesia. They were euthanized under anesthesia, perfused with phosphate-buffered saline and then neutral buffered formalin, and the brains subjected to histological analysis as described above.

Vasospasm was assessed by comparing the diameters of the basilar arteries on days 1, 8, and 15. The angiographic data was independently analyzed by four reviewers who were blinded to the animal group. The five averaged lumen diameters for each animal were then averaged to obtain a mean lumen diameter for each animal at each time point. Individual percent vasospasm was determined for each animal for Days 8 and 15 using formula (1):

$$\frac{[Follow-up(Day8or15)MeanLumenDiameter]-[Baseline(Day1)MeanLumenDiameter]}{BaselineMeanLumenDiameter}\times100 \quad \textbf{(1)}$$

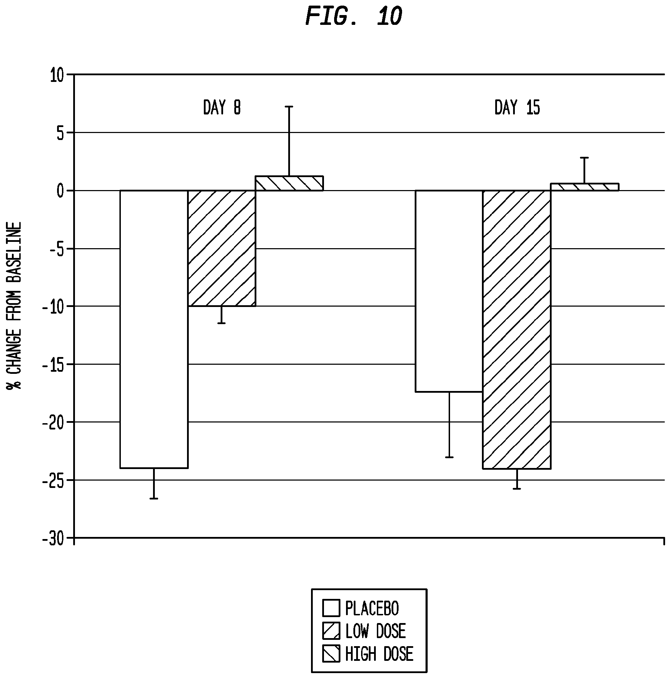

Average percent vasospasm for Days 8 and 15 was also determined for each group. Figure 10 shows percent (%) changes in mean basilar arterial diameters from baseline following treatment in the cisterna magna of the subarachnoid space with a low dose (10 mg) nimodipine formulation, a high dose (30 mg) formulation, and a placebo. Table 2 summarizes the mean, standard error, median and standard deviation for percent vasospasm.

**Table 2 Summary of percent vasospasm data from review of angiograms**

| Table 2: Summary Data from Review of Angiograms (Study 1) | | | | | | |
|---|---|---|---|---|---|---|
| | Placebo Day 8 | Low Dose Day 8 | High Dose Day 8 | Placebo Day 15 | Low Dose Day 15 | High Dose Day 15 |
| Mean | -24 | -9.925 | 1.225 | -17.325 | -24 | 0.65 |
| Standard Error | 2.543947064 | 1.5665115 | 5.999774301 | 5.735761 | 1.807392 | 2.128575 |
| Median | -24.95 | -10.6 | -3.15 | -13.8 | -25.6 | -1.2 |
| Standard Deviation | 5.087894129 | 3.133023 | 11.9995486 | 11.47152 | 3.614784 | 4.257151 |

At day 8, the mean basilar artery diameter decreased 24% in control animals when compared to baseline. Animals treated with low dose of nimodipinc microparticles had a -9.9% mean decrease in basilar artery diameter when compared to baseline. Animals treated with the high dose of nimodipine microparticles had a mean basilar artery diameter increase of 1.2% when compared to baseline.

At day 15, the mean basilar artery diameter decrease for the control animals (placebo-treated) was -17.3% when compared to baseline. Animals treated with the low dose of nimodipine microparticles had a -24% mean decrease in basilar artery diameter when compared to baseline. Animals treated with the high dose of nimodipine microparticles had a mean basilar artery diameter increase of 0.7% when compared to baseline. Statistical analysis was not performed due to the small number of animals studied.

This Example shows that: (1) at day 8, narrowing of basilar artery was highest for the control group, followed by the low dose group and lowest for the high dose group; and (2) at day 15, narrowing of basilar artery was highest the control, low dose groups and lowest for the high dose group. Due to a small number of animals in the study, the change in basilar artery in the lose dose group at day 15 was within expected statistical variation.

Clinical findings that were considered to be associated with the study procedures were limited to observations of decreased activity and inappetence. Decreased activity was noted in all 6 animals during the first week of the study and in one placebo treated and one low dose nimodipine microparticle treated animal during the second week. Inappetence was noted in 5 of 6 animals during the first week and 4 of 6 animals during the second week. These findings were present in animals from all dose groups and therefore considered to be study procedure related.

*Behavioral Observations*

Observations for morbidity, mortality, injury, and the availability of food and water were conducted twice daily for all animals. Body weights were measured and recorded prior to randomization and weekly during the study. A complete physical examination was conducted on all animals by a staff veterinarian pretest.

Behavioral observations were conducted by a staff veterinarian on a daily basis for each animal enrolled on study. Behavior of each animal was examined by a staff veterinarian daily. Behavior pertaining to behavior categories of apetite, activity and neurological defect were given behavioral scores according to Tables 3-5.

Table 3 provides behavioral scores given for appetite.

**Table 3. Behavioral Score for Appetite**

| Appetite | |
|---|---|
| Score | Observation |
| 2 | Finished meal |
| 1 | Left meal unfinished |
| 0 | Scarcely ate |

Table 4 provides behavioral scores for activity.

**Table 4. Behavioral scores for Activity**

| Activity | |
|---|---|
| Score | Observation |
| 2 | Active, barking or standing |
| 1 | Lying down, will stand and walk with some stimulation |
| 0 | Almost always lying down |

Table 5 provides behavioral scores for neurological defects. Neurological defect scored was ability to walk because of ataxia or paresis.

**Table 5. Behavioral scores for neurological defects**

| Neurological Defects | |
|---|---|
| Score | Observation |
| 2 | No deficit |
| 1 | Unable to walk because of ataxia or paresis |
| 0 | Impossible to walk or stand because of ataxia or paresis |

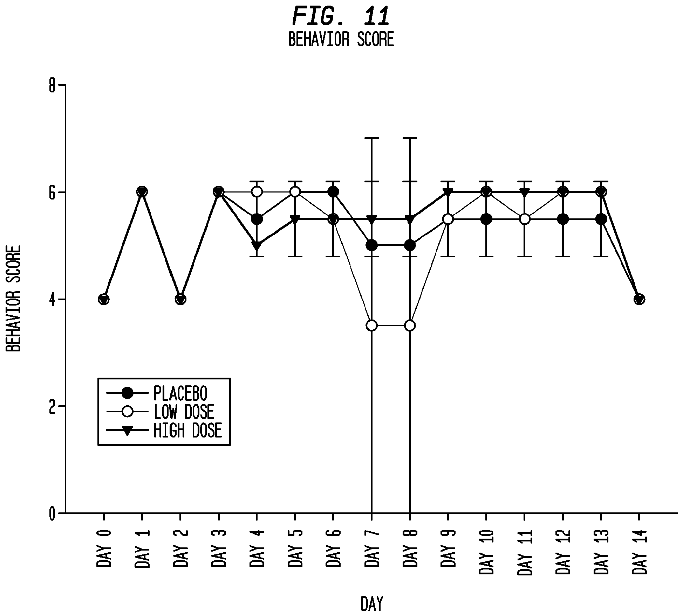

Figure 11 shows a plot of averaged behavioral scores of dogs subjected to subarachnoid hemorrhage, which are treated with a placebo, a low dose (10 mg) of the microparticulate nimodipine formulation, or a high dose (30 mg) of the microparticulate nimodipine formulation.
There were no consistent or marked changes in appetite or activity and no changes in neurological function.
Neither the study procedures nor treatment with placebo or nimodipine microparticles was associated with any substantial change in body weight. There were no apparent differences in hematology parameters between either nimodipine microparticle dose and placebo.

***Serum Analysis***

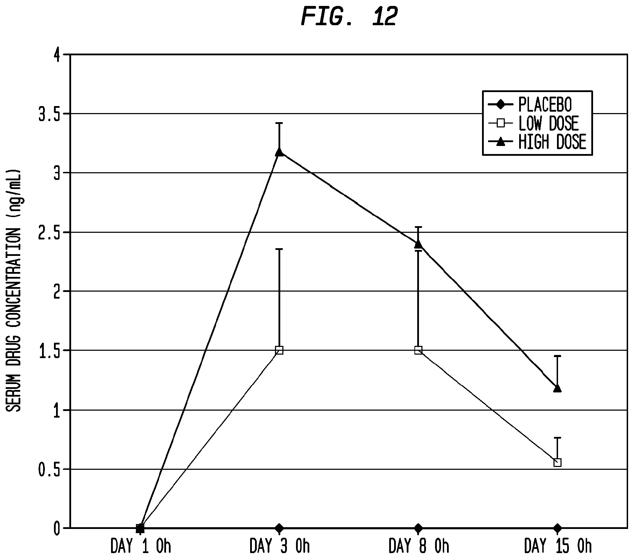

Analysis of serum samples for nimodipine showed higher concentrations on day 3 with detectable levels of nimodipine still present on day 15 (Figure 7, Table 6). Table 6 lists the serum drug concentrations (ng/mL) in dogs subjected to subarachnoid hemorrhage, when treated with placebo, low dose (10 mg) microparticulate nimodipine formulation or a high dose (30 mg) microparticulate nimodipine formulation. Figure 12 shows a plot of the serum drug concentrations (ng/mL) over time in dogs subjected to subarachbnoid hemorrhage, when treated with placebo, a low dose (10 mg) microparticulate nimodipine formulation or a high dose (30 mg) microparticulate nimodipine formulation.
Serum concentrations of nimodipine were higher in animals treated with high-dose nimodipine microparticles. Nimodipine was not detected at any time point in the placebo animals.

**Table 6. Serum drug concentrations (ng/mL)**

| Table 6: Serum Drug Concentrations (ng/mL)(Study 1) | | | | | |
|---|---|---|---|---|---|
| Group | | Day 1 0h | Day 3 0h | Day 8 0h | Day 15 0h |
| Placebo | Mean | 0 | 0 | 0 | 0 |

(continued)

| Table 6: Serum Drug Concentrations (ng/mL)(Study 1) | | Day 1 0h | Day 3 0h | Day 8 0h | Day 15 0h |
|---|---|---|---|---|---|
| Group | | | | | |
| | SD | NA | NA | NA | NA |
| Formulation Dose 1 | Mean | 0 | 1.51 | 1.5 | 0.559 |
| | SD | NA | 0.184 | 0.148 | 0.257 |
| Formulation Dose 2 | Mean | 0 | 3.18 | 2.4 | 1.19 |
| | SD | NA | 0.856 | 0.834 | 0.219 |
| Below limit of quantitation <0.200 ng/mL | | | | | |
| Above limit of quantitation >200 ng/mL | | | | | |
| N = 2 per measurement | | | | | |

### *Cerebrospinal Fluid (CSF) Analysis*

Analysis of CSF samples found sustained high concentrations ofnimodipine on days 3 and 8 after administration of the low dose of nimodipine microparticles with lower concentrations present on day 15. Table 7 lists the drug concentrations (ng/mL) in CSF from dogs subjected to subarachbnoid hemorrhage, when treated with placebo, a low dose (10 mg) microparticulate nimodipine formulation or a high dose (30 mg) microparticulate nimodipine formulation.

CSF nimodipine concentrations were significantly higher than serum concentrations with administration of low and high dose of nimodipine microparticles and detectable concentrations were still present at day 15. One of the high dose day 3 samples was above the limit of quantitation (>500 ng/mL) and was not re-testable due to lack of additional sample. Statistical significance could not be determined because of low sample number. Nimodipine was not detected at any time points in the Placebo animals.

### Table 7. CSF nimodipine concentrations (ng/mL) for each treatment group

| Table 7: CSF Nimodipine Concentrations (ng/mL)(Study 1) | | Day 1 0h | Day 3 0h | Day 8 0h | Day 15 0h |
|---|---|---|---|---|---|
| Group | | | | | |
| Placebo | Mean | 0 | 0 | 0 | 0 |
| | SD | NA | NA | NA | NA |
| Formulation Dose 1 | Mean | 0 | 380 | 379 | 156 |
| | SD | NA | 151 | NA | 132 |
| Formulation Dose 2 | Mean | 0 | 5.78* | 126 | 63.6 |
| | SD | NA | NA | 168 | 88.2 |
| Below limit of quantitation <0.500 ng/mL | | | | | |
| Above limit of quantitation >500 ng/mL | | | | | |
| * One of the two samples of Formulation Dose 2 was above the limit of quantitation and not enough sample remained for additional testing | | | | | |
| N = 2 per measurement | | | | | |

### *Microscopic*

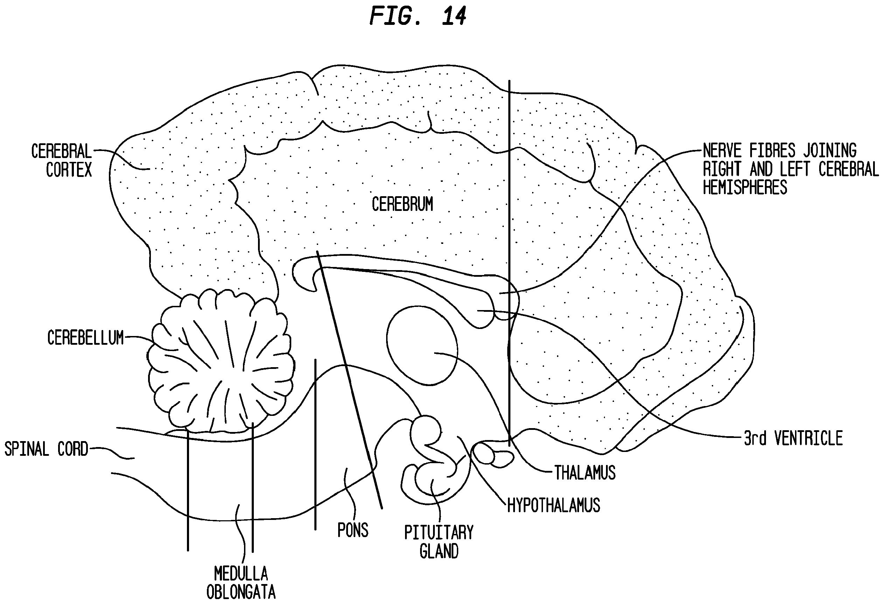

Figure 13 shows histopathology of dogs subjected to subarachnoid hemorrhage (SAH) when treated with placebo (A) and when treated with the low dose microparticulate nimodipine formulation (B). Figure 14 shows sectional planes used in canine model experiments. The only microscopic findings consisted of minimal to mild granulomatous inflammation within the subarachnoid space of the pons and/or medulla in both animals from the low-dose microparticulate nimodipine formulation group and one of 2 animals from the high-dose microparticulate nimodipine formulation group. Inflammation was characterized by aggregates of giant cells that engulfed foreign material. Minimal subacute inflammation or lymphocytic perivascular infiltrate was also noted in a few animals across groups. The latter finding was closely associated with the granulomatous inflammation.

Minimal to mild degeneration was noted in one of two animals from the placebo group and both animals from the low-dose nimodipine microparticle group. Degeneration was present in the ventral portion of the pons and/or medulla and was characterized by cavitating spaces partially filled with hemorrhage, proliferating small vessels, and increased numbers of glial/astrocytic cells. Foamy vacuolated cells were occasionally present. Axonal swelling/degeneration was present in the adjacent brain tissue. This finding was considered related to the injection procedure and was not an effect of the injected composition.

Meningeal hemorrhage and/or fibroplasia were noted in most animals examined and were likely related to the necropsy and/or the injection procedure.

The microscopic studies showed minimal to mild granulomatous inflammation within the subarachnoid space, minimal to mild degeneration and meningeal hemorrhage and/or fibroplasia in all treatment groups, i.e., placebo, low dose (10 mg) microparticulate nimodipine formulation and high dose (30 mg) microparticulate nimodipine formulation.

**Example 2. Study 2 - Effect of Nimodipine formulations on cerebral vasospasm in a subarachnoid hemorrhage (SAH) Dog model**

**Materials and Methods**

*Formulation*

The test formulation of the microparticulate nimodipine formulation containing a uniform distribution of microparticle size was prepared by combining a polymer solution (e.g., a 50-50 glycolide-lactide blend) with a solvent in the presence of nimodipine. The mixture was added to a surfactant containing aqueous solution to form an emulsion and the solvent extracted to produce the flowable microparticulate nimodipine formulation. The initial drug load was 65%, i.e., 65% nimodipine and 35% polymer. The mean particle size was about 52 μ.

The microparticulate nimodipine formulation is combined with a pharmaceutical carrier to form the pharmaceutical composition of the described invention. When the means for delivery is a surgical injection apparatus and the delivery site is within close proximity of a cerebral artery in a subarachnoid space, a vehicle that imparts stickiness (e.g. hyaluronic acid) is mixed with the microparticulate nimodipine formulation ("nimodipine formulation 1"). For microparticulate nimodipine formulation 2 ("nimodipine formulations 2"), the pharmaceutically acceptable carrier a vehicle that imparts stickiness was not used as the pharmaceutically acceptable carrier. The placebo formulation contained the microparticles plus vehicle, but no nimodipine.

*Treatment Groups*

A total of 30 dogs were assigned to the study as presented in Table 8.

**Table 8. Treatment group assignments**

| Table 8. Group Assignments (Study 2) | | |
|---|---|---|
| Group Number | Treatment | Number of Animals |
| 1 | Intracisternal administration of Microparticulate Placebo Formulation with HA followed by Oral Nimodipine | 4 males + 4 females |
| 2 | Intracisternal administration of Microparticulate Nimodipine Formulation 1 (with HA) at dose 1[a] | 4 males + 4 females |
| 3 | Intracisternal administration of Microparticulate Nimodipine Formulation 1 (with HA) at dose 2[b] | 3 males + 3 females |
| 4 | Intraventricular administration of Microparticulate Nimodipine Formulation 2 (without HA) at dose 2[c] | 4 males + 4 females |
| [a]40 mg delivered dose | | |
| [b]100 mg delivered dose | | |
| [c]100 mg delivered dose | | |

*Administration*

The micropaticulate nimodipine formulation (Formulation 1) was administered to treatment groups 2 and 3 with a vehicle (e.g. hyaluronic acid) by surgical injection within the cisterna magna of the subarachnoid space. The microparticulate nimodipine formulation (Formulation 2) was administered to treatment group 4 without the vehicle by syringe through a catheter (14 gauge to 18 gauge) into a cerebral ventricle. The microparticulate placebo formulation was administered to treatment group 1 (Oral Control) with a vehicle (e.g. hyaluronic acid) by surgical injection within the cisterna magna of the subarachnoid space. Following administration of microparticulate placebo formulation on day 1, treatment group 1 (Oral Control) then received oral nimodipine capsules (0.86mg/kg) six times per day till day 21. A placebo control group was also administered a microparticulate placebo formulation with a vehicle (e.g. hyaluronic acid) by surgical injection within the cisterna magna of the subarachnoid space. (Data not shown). The delivered dose levels for the treated groups 2, 3 and 4 are as shown in Table 8 above. The syringes were loaded with the pharmaceutical compositions taking into account the overfill needed to fill the dead volume in the delivery system. The delivered doses were approximately 40 mg (dose 1) and 100 mg (dose 2). The oral control and placebo groups received the control article in the same manner as the treated groups.

For reconstitution/injection, a syringe comprising diluent was attached via a connector to a syringe comprising the microparticulate nimodipine formulation. In case of treatment groups 1, 2, and 3, a plunger is cycled to draw the vehicle into the microparticulate formulation. The resulting pharmaceutical composition is then pushed into the left syringe, which is disconnected from the connector. For delivery, the composition is injectable either through a surgical needle or can be fitted with and injected through a cannula or catheter of any appropriate size.

**Surgical Procedures**

On Day 1, the dogs were weighed, baseline blood was collected and blood pressure, temperature, heart rate oxygen, and blood gases were monitored throughout the surgical procedure. Cerebral angiography was performed through one vertebral artery. Images were captured using identical exposure factors and magnification for every angiogram. An internal magnification standard was included in every angiogram.

In case of treatment groups 1, 2, and 3, following angiography, the animals were turned prone and the cisterna magna was punctured percutaneously with an 14 gauge spinal needle. A targeted volume of 0.3 mL/kg CSF was allowed to drain spontaneously, after which 0.5 mL/kg of fresh, autologous, arterial, non-heparinized blood was withdrawn from the femoral catheter and injected into the cisterna magna at a rate of approximately 5 mL/minute. Approximately one half of the blood volume was injected, then the microparticulate placebo formulation or microparticulate nimodipine formulation was administered at a rate of approximately 5 mL/minute. Upon completion of placebo or nimodipine formulation (dose 1 and dose 2) administration, the remaining blood was injected. The needle was withdrawn immediately after the injection. The animal was tilted 30° head down during cisternal blood injection and remained in that position for 15 minutes following completion of injection. The animal was then turned supine, the femoral catheter was removed, and the femoral artery was ligated. The incision was closed in a standard fashion.

In case of treatment group 4, the micropaticulate nimodipine formulation was administered as a single injection through a catheter (14 gauge) into the lateral ventricle at a rate of 5 mL/minute.

On day 3, the dogs were placed under general anesthesia and the cisternal blood injection (in case of treatment groups 1, 2, and 3) or the intraventricular administration (in case of treatment group 4) was repeated. On days 8 and 15, the animals were anesthetized, and angiography and removal of CSF from the cistern magna was repeated. After angiography on day 15, animals were not recovered from anesthesia. They were euthanized under anesthesia, perfused with phosphate-buffered saline and then neutral buffered formalin, and the brains subjected to histological analysis as described above.

Vasospasm was assessed by comparing the diameters of the basilar arteries on days 1,8, and 15. The angiographic data was independently analyzed by two reviewers who were blinded to the animal group. The five averaged lumen diameters for each animal were then averaged to obtain a mean lumen diameter for each animal at each time point. Individual percent vasospasm was determined for each animal for Days 8 and 15 using formula (1):

$$\frac{\left[Follow-up(Day8or15)MeanLumenDiameter\right]-\left[Baseline(Day1)MeanLumenDiameter\right]}{BaselineMeanLumenDiameter}\times100 \quad \textbf{(1)}$$

Average percent vasospasm for Days 8 and 15 was also determined for each group. Figure 15 shows percent (%) changes in mean basilar arterial diameters from baseline following treatment in the cisterna magna of the subarachnoid space with nimodipine formulation 1, dose 1 (40 mg), nimodipine formulation 1, dose 2 (100 mg), and oral control, as well as following administration of nimodipine formulation 2 dose 2 (100 mg) to a cerebral ventricle. Table 9 summarizes the mean, standard error, median and standard deviation for percent vasospasm.

**Table 9 Summary of percent vasospasm data from review of angiograms**

| Table 9: Summary Data from Review of Angiograms (Study 2) | | | | |
|---|---|---|---|---|
| | Control Day 8 | Formulation 1 40mg Day 8 | Formulation 1 100 mg Day 8 | Formulation 2 100 mg Day 8 |
| Mean | -27.89% | -13.27% | -9.38% | -8.08% |
| Standard Error | 0.0300 | 0.0529 | 0.0558 | 0.0392 |
| | Control Day 15 | Formulation 1 40mg Day 15 | Formulation 1 100mg Day 15 | Formulation 2 100mg Day 15 |
| Mean | -11.67% | -3.60% | -12.73% | 1.13% |
| Standard Error | 0.0454 | 0.0430 | 0.0355 | 0.0326 |

This example shows that on day 8 control animals treated with oral nimodipine had the highest reduction in basilar artery diameter compared to baseline followed by animals treated with formulation 1 at 40 mg dose, followed by animals treated with formulation 1 at 100 mg dose, and followed by animals treated formulation 2 at 100mg dose via intraventricular delivery. Animals treated with formulation 2 at 100mg dose via intraventricular delivery showed the lowest reduction in basilar artery diameter. All three formulations were statistically significant on day 8 when compared to control. On day 15, control and formulation 1 100 mg dose had similar reductions in basilar artery diameter, formulation 1 at 40 mg dose was lower, and formulation 2 at 100 mg dose had no reduction or a slight dilation of basilar artery diameter compared to baseline. On day 15, only formulation 2 at 100 mg dose was statistically significant compared to control. This example showed that all three formulations were able to reduce vasospasm compared to oral nimodipine control on day 7 and formulation 2 100 mg dose was able to reduce vasospasm compared to oral nimodipine control on day 15.

*Behavioral Observations (Study 2)*

Observations for morbidity, mortality, injury, and the availability of food and water were conducted twice daily for all animals. Body weights were measured and recorded prior to randomization and weekly during the study. A complete physical examination was conducted on all animals by a staff veterinarian pretest.
Behavioral observations were conducted by a staff veterinarian on a daily basis for each animal enrolled on study. Behavior of each animal was examined by a staff veterinarian daily. Behavior pertaining to behavior categories of apetite, activity and neurological defect were given behavioral scores according to Tables 3-5 above.
There were no consistent or marked changes in appetite or activity and no changes in neurological function.

*Serum Analysis (Study 2)*

Tables 10a-10g list the serum drug concentrations (ng/mL) in dogs subjected to subarachbnoid hemorrhage, when treated with nimodipine formulation 1, dose 1 (40 mg), nimodipine formulation 1, dose 2 (100 mg), and oral control, as well as following administration of nimodipine formulation 2 dose 2 (100 mg) to a cerebral ventricle. Figure 16 shows a plot of the serum drug concentrations (ng/mL) over time in dogs subjected to subarachbnoid hemorrhage, when treated with nimodipine formulation 1, dose 1 (40 mg), nimodipine formulation 1, dose 2 (100 mg), and oral control, as well as following administration of nimodipine formulation 2 dose 2 (100 mg) to a cerebral ventricle.
Figure 16 and Tables 10a-10g show that formulation 1 at 40 mg dose and at 100 mg dose both showed peak plasma concentration on day 3, formulation 2 at 100 mg dose showed peak plasma concentration on day 4, and control oral nimodipine had a peak plasma concentration on day 7. Formulation 1 at 40 mg had the lowest peak concentration (8.3 ng/mL) while formulation 1 at 100mg, formulation 2 at 100 mg, and control with oral nimodipine all had approximately equal peak concentrations (13.6 ng/mL, 14.6 ng/mL, and 14.6 ng/mL, respectively). This example showed that all formulations had plasma concentrations at or below the maximum plasma concentrations seen in control with oral nimodipine.

**Table 10. Serum drug concentrations (ng/mL)(Study 2)**

| Table 10a: Plasma Nimodipine Concentrations (ng/mL)(Study 2) | | | | | |
|---|---|---|---|---|---|
| Group | | Day 1 0h | Day 1 1h | Day 1 3h | Day 1 6h |
| Control | Mean | BLQ<0.200 | BLQ<0.200 | BLQ<0.200 | 0.3 |
| | SD | NA | NA | NA | 0.6 |
| | # | 8 | 8 | 8 | 8 |
| Formulation 1 40 mg | Mean | BLQ<0.200 | 2.6 | 2.4 | 2.3 |
| | SD | NA | 0.7 | 1.4 | 0.9 |
| | # | 8 | 8 | 7 | 8 |
| Formulation 1 100 mg | Mean | BLQ<0.200 | 6.3 | 5.0 | 4.9 |
| | SD | NA | 2.6 | 2.5 | 2.1 |
| | # | 6 | 6 | 6 | 6 |
| Formulation 2 100 mg | Mean | BLQ<0.200 | 5.4 | 4.2 | 4.5 |
| | SD | NA | 1.0 | 0.8 | 0.9 |
| | # | 8 | 8 | 8 | 8 |
| Table 10b: Plasma Nimodipine Concentrations (ng/mL)(Study 2) | | | | | |
| Group | | Day 1 12h | Day 1 18h | Day 1 24h | Day 1 36h |
| Control | Mean | 0.9 | 1.8 | 3.4 | 6.5 |
| | SD | 0.5 | 0.8 | 1.9 | 5.5 |
| | # | 8 | 8 | 8 | 8 |
| Formulation 1 40 mg | Mean | 3.6 | 4.4 | 5.4 | 7.0 |
| | SD | 1.9 | 2.3 | 2.7 | 3.4 |
| | # | 8 | 8 | 8 | 8 |
| Formulation 1 100 mg | Mean | 5.7 | 7.5 | 8.3 | 10.8 |
| | SD | 2.1 | 2.7 | 3.2 | 4.7 |
| | # | 6 | 6 | 6 | 6 |
| Formulation 2 100 mg | Mean | 6.0 | 8.3 | 9.6 | 12.2 |
| | SD | 1.9 | 3.1 | 3.8 | 5.8 |
| | # | 8 | 8 | 8 | 8 |
| Table 10c: Plasma Nimodipine Concentrations (ng/mL)(Study 2) | | | | | |
| Group | | Day 3 0h | Day 4 0h | Day 5 0h | Day 6 0h |
| Control | Mean | 8.9 | 9.4 | 7.0 | 10.5 |
| | SD | 4.8 | 7.7 | 6.4 | 6.4 |
| | # | 8 | 8 | 8 | 8 |
| Formulation 1 40 mg | Mean | 8.3 | 6.4 | 5.2 | 4.5 |
| | SD | 4.0 | 2.0 | 1.8 | 1.4 |
| | # | 8 | 8 | 8 | 8 |
| Formulation 1 100 mg | Mean | 13.6 | 11.6 | 10.4 | 9.0 |
| | SD | 6.1 | 5.2 | 4.7 | 4.5 |
| | # | 6 | 6 | 6 | 6 |
| Formulation 2 100 mg | Mean | 13.0 | 14.6 | 12.8 | 11.8 |
| | SD | 5.4 | 5.1 | 3.7 | 3.7 |
| | # | 8 | 8 | 8 | 8 |

(continued)

| Table 10d: Plasma Nimodipine Concentrations (ng/mL)(Study 2) | | | | | |
|---|---|---|---|---|---|
| Group | | Day 7 0h | Day 8 0h | Day 9 0h | Day 10 0h |
| Control | Mean | 14.6 | 8.1 | 7.0 | 6.3 |
| | SD | 18.8 | 10.5 | 4.8 | 5.4 |
| | # | 8 | 8 | 8 | 8 |
| Formulation 1 40 mg | Mean | 3.9 | 3.9 | 3.3 | 2.9 |
| | SD | 0.9 | 0.9 | 1.1 | 0.8 |
| | # | 8 | 8 | 8 | 8 |
| Formulation 1 100 mg | Mean | 8.1 | 7.8 | 6.4 | 5.4 |
| | SD | 3.9 | 3.9 | 3.0 | 2.6 |
| | # | 6 | 6 | 6 | 6 |
| Formulation 2 100 mg | Mean | 11.7 | 12.7 | 13.6 | 13.7 |
| | SD | 4.5 | 4.2 | 4.1 | 4.3 |
| | # | 8 | 8 | 8 | 8 |
| Table 10e: Plasma Nimodipine Concentrations (ng/mL)(Study 2) | | | | | |
| Group | | Day 12 0h | Day 14 0h | Day 16 0h | Day 18 0h |
| Control | Mean | 3.5 | 2.6 | 4.3 | 5.2 |
| | SD | 2.3 | 1.2 | 3.8 | 4.6 |
| | # | 8 | 8 | 8 | 8 |
| Formulation 1 40 mg | Mean | 2.0 | 1.4 | 1.4 | 0.9 |
| | SD | 0.8 | 0.7 | 0.6 | 0.4 |
| | # | 8 | 8 | 6 | 6 |
| Formulation 1 100 mg | Mean | 4.0 | 3.4 | 3.0 | 2.1 |
| | SD | 2.3 | 1.8 | 1.2 | 1.0 |
| | # | | | 4 | |
| Formulation 2 100 mg | Mean | 10.7 | 8.1 | 5.1 | 3.8 |
| | SD | 4.2 | 3.4 | 2.4 | 2.0 |
| | # | 8 | 8 | 6 | 6 |
| Table 10f: Plasma Nimodipine Concentrations (ng/mL)(Study 2) | | | | | |
| Group | | Day 21 0h | Day 28 0h | Day 38 0h | Day 42 0h |
| Control | Mean | 4.2 | BLQ<0.200 | BLQ<0.200 | BLQ<0.200 |
| | SD | 4.3 | NA | NA | NA |
| | # | 8 | 4 | 3 | 3 |
| Formulation 1 40 mg | Mean | 0.5 | 0.1 | BLQ<0.200 | BLQ<0.200 |
| | SD | 0.3 | 0.2 | NA | NA |
| | # | 6 | 6 | 5 | 5 |
| Formulation 1 100 mg | Mean | 1.3 | 0.2 | BLQ<0.200 | BLQ<0.200 |
| | SD | 0.7 | 0.3 | NA | NA 2 |
| | # | 4 | 4 | 2 | 2 |
| Formulation 2 100 mg | Mean | 1.6 | 0.7 | BLQ<0.200 | BLQ<0.200 |
| | SD | 1.0 | 1.0 | NA | NA |
| | # | 6 | 3 | 1 | 1 |

(continued)

| Table 10g: Plasma Nimodipine Concentrations (ng/mL)(Study 2) | | | |
|---|---|---|---|
| Group | | Day 49 0h | |
| Control | Mean | BLQ<0.200 | |
| | SD | NA | |
| | # | 3 | |
| | | | |
| | | | |
| | | | |
| Formulation 1 40 mg | Mean | BLQ<0.200 | |
| | SD | NA | |
| | # | 5 | |
| Formulation 1 100 mg | Mean | BLQ<0.200 | |
| | SD | NA | |
| | # | 2 | |
| Formulation 2 100 mg | Mean | BLQ<0.200 | |
| | SD | NA | |
| | # | 1 | |

### Cerebrospinal Fluid (CSF) Analysis (Study 2)

Tables 11a-11b list the CSF drug concentrations (ng/mL) in dogs subjected to subarachbnoid hemorrhage, when treated with nimodipine formulation 1, dose 1 (40 mg), nimodipine formulation 1, dose 2 (100 mg), and oral control, as well as following administration of nimodipine formulation 2 dose 2 (100 mg) to a cerebral ventricle. Figure 17 shows a plot of the CSF drug concentrations (ng/mL) over time in dogs subjected to subarachbnoid hemorrhage, when treated with nimodipine formulation 1, dose 1 (40 mg), nimodipine formulation 1, dose 2 (100 mg), and oral control, as well as following administration of nimodipine formulation 2 dose 2 (100 mg) to a cerebral ventricle.

Figure 17 and Tables 11a-11b show that control with oral nimodipine" formulation 0 mg dose, formulation 1 100 mg dose, and formulation 2 100 mg dose all had peak CSF nimodipine concentrations at day 3. Control with oral nimodipine had a peak CSF nimodipine concentrations of 1.6 ng/mL, formulation 1 40 mg dose had a peak CSF nimodipine concentration of 1763.5 ng/mL, formulation 1 100 mg dose had a peak CSF nimodipine concentration of 3028.5 ng/mL, and formulation 2 100 mg dose had a peak CSF nimodipine concentration of 1896.5 ng/mL. Control with oral nimodipine had detectable CSF concentrations up to day 8, formulation 1 40 mg and 100 mg doses had detectable CSF concentrations up to day 35, and formulation 2 100 mg dose had detectable CSF concentrations up to day 42. This example showed that both formulation 1 and 2 were able to have significantly higher CSF nimodipine concentrations than control with oral nimodipine and were able to maintain these concentrations for a longer period of time. This example shows that both formulation 1 and formulation 2 were able to generate high CSF nimodipine concentrations while keeping plasma nimodipine concentrations at or below maximal levels seen with control oral nimodipine. This example shows that high levels of CSF nimodipine concentrations achieved with the microparticulate nimodipine formulations when administered locally (either by intracisternal or by intraventricular administration) are able to reduce vasospasm in dogs with subarachnoid hemorrhage.

**Table 11. CSF nimodipine concentrations (ng/mL)for each treatment group (Study 2)**

| Table 11a: CSF Nimodipine Concentrations (ng/mL) (Study 2) | | | | | |
|---|---|---|---|---|---|
| Group | | Day 1 0h | Day 3 0h | Day 8 0h | Day 15 0h |
| Control | Mean | BLQ<0.500 | 1.6 | 0.5 | BLQ<0.500 |
| | SD | NA | 1.2 | 0.7 | NA |
| | # | 8 | 7 | 8 | 7 |
| Formulation 1 40 mg | Mean | BLQ<0.500 | 1763.5 | 476.0 | 127.6 |

(continued)

| Table 11a: CSF Nimodipine Concentrations (ng/mL) (Study 2) | | | | | |
|---|---|---|---|---|---|
| Group | | Day 1 0h | Day 3 0h | Day 8 0h | Day 15 0h |
| | SD | NA | 1122.2 | 311.8 | 94.4 |
| | # | 8 | 8 | 8 | 8 |
| Formulation 1 100 mg | Mean | BLQ<0.500 | 3028.5 | 1210.6 | 325.0 |
| | SD | NA | 2347.0 | 648.2 | 342.0 |
| | # | 6 | 6 | 5 | 6 |

| Table 11a: CSF Nimodipine Concentrations (ng/mL)(Study 2) | | | | | |
|---|---|---|---|---|---|
| | | Day 1 0h | Day 3 0h | Day 8 0h | Day 15 0h |
| Formulation 2 100 mg | Mean | BLQ<0.500 | 1896.5 | 1254.8 | 273.8 |
| | SD | NA | 803.4 | 697.7 | 245.8 |
| | # | 8 | 8 | 8 | 8 |

| Table 11b: CSF Nimodipine Concentrations (ng/mL)(Study 2) | | | | | |
|---|---|---|---|---|---|
| Group | | Day 28 0h | Day 35 0h | Day 42 0h | Day 49 0h |
| Control | Mean | BLQ<0.500 | BLQ<0.500 | BLQ<0.500 | BLQ<0.500 |
| | SD | NA | NA | NA | NA |
| | # | 7 | 4 | 3 | 2 |
| Formulation 1 40 mg | Mean | 9.0 | 1.3 | BLQ<0.500 | BLQ<0.500 |
| | SD | 7.4 | 1.1 | NA | NA |
| | # | 6 | 5 | 5 | 4 |
| Formulation 1 100 mg | Mean | 30.5 | 1.3 | BLQ<0.500 | BLQ<0.500 |
| | SD | 30.6 | 1.9 | NA | NA |
| | # | 4 | | 2 | 2 |
| Formulation 2 100 mg | Mean | 14.6 | 10.9 | 1.6 | BLQ<0.500 |
| | SD | 13.5 | NA | NA | NA |
| | # | 3 | 1 | 1 | 1 |

**Equivalents**

While the present invention has been described with reference to the specific embodiments thereof it should be understood by those skilled in the art that various changes may be made and equivalents may be substituted without departing from the true spirit and scope of the invention. In addition, many modifications may be made to adopt a particular situation, material, composition of matter, process, process step or steps, to the objective spirit and scope of the present invention. All such modifications are intended to be within the scope of the claims appended hereto.

The following statements of invention summarise aspects and embodiments of the present invention.

1. A method for treating a delayed complication associated with a brain injury in a mammal in need thereof, wherein the brain injury includes interruption of at least one cerebral artery, the method comprising:

(a) providing a pharmaceutical composition comprising

(i) a microparticulate formulation of a voltage-gated calcium channel blocker; and optionally
(ii) a pharmaceutically acceptable carrier; and

(b) administering a therapeutic amount of the pharmaceutical composition by a means for administration of the therapeutic amount of the pharmaceutical composition at a site of administration,

wherein the therapeutic amount is effective in reducing signs or symptoms of the at least one delayed complication associated with the brain injury, and
wherein the at least one delayed complication is at least one of delayed cerebral ischemia (DCI), a plurality of microthromboemboli, a cortical spreading ischemia (CSI), and an angiographic vasospasm.

2. The method according to statement 1 wherein in step (b), the means for administration is a surgical injection apparatus and the site of administration is in close proximity to at least one cerebral artery affected by the brain injury.

3. The method according to statement 1, wherein in step (b), the means for administration is a surgical injection apparatus and the site of administration is a cerebral ventricle.

4. The method according to statement 2, wherein the surgical injection apparatus is a needle, a cannula, a catheter, or a combination thereof.

5. The method according to statement 3, wherein the microparticulate formulation is carried by cerebrospinal fluid to contact the cerebral artery affected by the brain injury.

6. The method according to statement 1, wherein the therapeutic amount is effective to increase the internal diameter of the cerebral artery affected by the brain injury, as compared to a control.

7. The method according to statement 1, wherein the brain injury is a result of an aneurysm, sudden traumatic head injury, subarachnoid hemorrhage (SAH), or a combination thereof.

8. The method according to statement 7, wherein the brain injury is a result of subarachnoid hemorrhage.

9. The method according to statement 1, wherein the at least one delayed complication associated with the brain injury further comprises at least one of an intracerebral hematoma, an intraventricular hemorrhage, a fever, a behavioral deficit, a neurological deficit, a cerebral infarction, and neuronal cell death.

10. The method according to statement 9, wherein the behavioral deficit is improved such that the improved behavioral deficit comprises an increase in appetite.

11. The method according to statement 9, wherein the neurological deficit is improved such that the improved neurological deficit comprises an improvement of ataxia or paresis.

12. The method according to statement 1, wherein the pharmaceutical composition exerts a predominantly localized pharmacologic effect in treating the at least one delayed complication associated with brain injury.

13. The method according to statement 1, wherein the pharmaceutical composition exerts a diffuse pharmacologic effect throughout the brain in treating the at least one delayed complication associated with brain injury.

14. The method according to statement 1, wherein the microparticulate formulation comprises a plurality of microparticles, wherein the microparticles are of uniform size distribution, and wherein each microparticle comprises a matrix.

15. The method according to statement 1, wherein the microparticulate formulation further comprises a plurality of microparticles impregnated with the voltage-gated calcium channel blocker.

16. The method according to statement 1, wherein the microparticle formulation comprises a liquid suspension of microparticles.

17. The method according to statement 1, wherein the cerebral ventricle is a lateral ventricle, a third ventricle, a fourth ventricle, or a combination thereof.

18. The method according to statement 1, wherein the microparticulate formulation of the voltage-gated calcium channel blocker comprises a slow-release compound.

19. The method according to statement 18, wherein the slow release compound is a polymer.

20. The method according to statement 19, wherein the voltage-gated calcium channel blocker is disposed on or in the slow-release polymer.

21. The method according to statement 20, wherein the microparticulate formulation of the voltage-gated calcium channel blocker comprises poly (D, L-lactide-co-glycolide).

22. The method according to statement 20, wherein the microparticulate formulation of a voltage-gated calcium channel blocker comprises poly(orthoester).

23. The method according to statement 20, wherein the microparticulate formulation of the voltage-gated calcium channel blocker comprises polyanhydride.

24. The method according to statement 1, wherein the voltage-gated calcium channel blocker is selected from the group consisting of an L-type voltage-gated calcium channel blocker, an N-type voltage-gated calcium channel blocker, a P/Q-type voltage-gated calcium channel blocker, or a combination thereof.

25. The method according to statement 1, wherein the voltage-gated calcium channel blocker is a dihydropyridine calcium channel blocker.

26. The method according to statement 25, wherein the dihydropyridine calcium channel blocker is nimodipine.

27. The method according to statement 1, wherein the pharmaceutically acceptable carrier comprises a gel compound.

28. The method according to statement 27, wherein the gel compound is a biodegradable hydrogel.

29. The method according to statement 1, wherein the pharmaceutical composition does not comprise the pharmaceutically acceptable carrier.

30. The method according to statement 1, wherein the pharmaceutically acceptable carrier does not comprise hyaluronic acid.

31. The method according to statement 1, wherein the pharmaceutically acceptable carrier comprises hyaluronic acid.

32. The method according to statement 31, wherein the pharmaceutically acceptable carrier ranges between 0 % to 5 % hyaluronic acid.

33. The method according to statement 32, wherein the pharmaceutically acceptable carrier comprises less than 2.3 % hyaluronic acid.

34. The method according to statement 33, wherein the pharmaceutically acceptable carrier comprises less than 5 % hyaluronic acid.

35. A semisolid multiparticulate delivery system for treating a delayed complication associated with a brain injury in a mammal in need thereof, wherein the brain injury includes interruption of at least one cerebral artery, the system comprising:

(a) a pharmaceutical composition comprising

(i) a microparticulate formulation of a voltage-gated calcium channel blocker; and optionally
(ii) a pharmaceutically acceptable carrier; and

(b) a means for administering a therapeutic amount of the pharmaceutical composition at a site for administration

wherein the therapeutic amount is effective in reducing signs or symptoms of the at least one delayed complication associated with the brain injury.

36. The system according to statement 35, wherein the at least one delayed complication is at least one of delayed cerebral ischemia (DCI), a plurality of microthromboemboli, a cortical spreading ischemia (CSI), and an angiographic vasospasm. The system according to statement 35, wherein the means for administration is a surgical injection apparatus and the site of administration is in close proximity to at least one cerebral artery affected by the brain injury.

37. The system according to statement 35, wherein the means for administration is a surgical injection apparatus and the site of administration is a cerebral ventricle.

38. The system according to statement 37, wherein the surgical injection apparatus is a needle, a cannula, a catheter, or a combination thereof.

39. The system according to statement 35, wherein the microparticulate formulation is capable of being carried by cerebrospinal fluid to contact the cerebral artery affected by the brain injury.

40. The system according to statement 35, wherein the therapeutic amount is effective to increase the internal diameter of the cerebral artery affected by the brain injury, as compared to a control.

41. The system according to statement 35, wherein the brain injury is a result of an aneurysm, sudden traumatic head injury, subarachnoid hemorrhage (SAH), or a combination thereof.

42. The system according to statement 41, wherein the brain injury is a result of subarachnoid hemorrhage.

43. The system according to statement 35, wherein the at least one delayed complication associated with the brain injury further comprises at least one of an intracerebral hematoma, an intraventricular hemorrhage, a fever, a behavioral deficit, a neurological deficit, a cerebral infarction, and neuronal cell death.

44. The system according to statement 43, wherein the behavioral deficit is improved such that the improved behavioral deficit comprises an increase in appetite.

45. The system according to statement 43, wherein the neurological deficit is improved such that the improved neurological deficit comprises an improvement of ataxia or paresis.

46. The system according to statement 35, wherein the pharmaceutical composition exerts a predominantly localized pharmacologic effect in treating the at least one delayed complication associated with brain injury.

47. The system according to statement 35, wherein the pharmaceutical composition exerts a diffuse pharmacologic effect throughout the brain in treating the at least one delayed complication associated with brain injury.

48. The system according to statement 35, wherein the microparticulate formulation comprises a plurality of microparticles, wherein the microparticles are of uniform size distribution, and wherein each microparticle comprises a matrix.

49. The system according to statement 35, wherein the microparticulate formulation further comprises a plurality of microparticles impregnated with the voltage-gated calcium channel blocker.

50. The system according to statement 35, wherein the microparticle formulation comprises a liquid suspension of microparticles.

51. The system according to statement 35, wherein the cerebral ventricle is a lateral ventricle, a third ventricle, a fourth ventricle, or a combination thereof.

52. The system according to statement 35, wherein the microparticulate formulation of the voltage-gated calcium channel blocker comprises a slow-release compound.

53. The system according to statement 52, wherein the slow release compound is a polymer.

54. The system according to statement 53, wherein the voltage-gated calcium channel blocker is disposed on or in the slow-release polymer.

55. The system according to statement 52, wherein the microparticulate formulation of a voltage-gated calcium channel blocker comprises poly (D, L-lactide-co-glycolide).

56. The system according to statement 55, wherein the microparticulate formulation of a voltage-gated calcium channel blocker comprises poly( orthoester).

57. The system according to statement 55, wherein the microparticulate formulation of a voltage-gated calcium channel blocker comprises polyanhydride.

58. The system according to statement 35, wherein the voltage-gated calcium channel blocker is selected from the group consisting of an L-type voltage-gated calcium channel blocker, an N-type voltage-gated calcium channel blocker, a P/Q-type voltage-gated calcium channel blocker, or a combination thereof.

59. The system according to statement 35, wherein the voltage-gated calcium channel blocker is a dihydropyridine calcium channel blocker.

60. The system according to statement 59, wherein the dihydropyridine calcium channel blocker is nimodipine.

61. The system according to statement 35, wherein the pharmaceutically acceptable carrier comprises a gel compound.

62. The system according to statement 61, wherein the gel compound is a biodegradable hydrogel.

63. The system according to statement 35, wherein the pharmaceutical composition does not comprise the pharmaceutically acceptable carrier.

64. The system according to statement 35, wherein the pharmaceutically acceptable carrier does not comprise hyaluronic acid.

65. The system according to statement 35, wherein the pharmaceutically acceptable carrier is hyaluronic acid.

66. The system according to statement 64, wherein the pharmaceutically acceptable carrier ranges between 0 % to 5 % hyaluronic acid.

67. The method according to statement 66, wherein the pharmaceutically acceptable carrier comprises less than 2.3 % hyaluronic acid.

68. The method according to statement 66, wherein the pharmaceutically acceptable carrier comprises less than 5 % hyaluronic acid.

**Claims**

1. A pharmaceutical composition for use in a method of treating a delayed complication of a brain injury in a mammal by administration of the composition to a site of administration in close proximity to at least one cerebral artery in the brain, wherein the at least one delayed complication is at least one of delayed cerebral ischemia (DCI), a plurality of microthromboemboli, a cortical spreading ischemia (CSI), and an angiographic vasospasm; said composition comprising :

   (i) a voltage-gated calcium channel blocker which is provided in polymeric microparticles; and
   (ii) a pharmaceutically acceptable carrier.

2. A pharmaceutical composition for use according to claim 1, wherein the composition is formulated for parenteral injection or implantation.

3. A pharmaceutical composition for use according to claim 1 or claim 2, wherein the composition comprises a suspension of microparticles.

4. A pharmaceutical composition for use as claimed in any preceding claim, wherein the microparticles comprise poly (D, L-Lactide-co-glycolide).

5. A pharmaceutical composition for use according to any preceding claim, wherein the pharmaceutically acceptable carrier comprises hyaluronic acid.

6. A pharmaceutical composition for use as claimed in claim 5, wherein the pharmaceutically acceptable carrier comprises less than 2.3% hyaluronic acid.

7. A pharmaceutical composition for use according to any preceding claim, wherein the at least one delayed complication is selected from the group consisting of a microthromboembolism, a cortical spreading ischemia, and a delayed cerebral ischemia caused by cortical spreading ischemia or the formation of microthromboemboli.

8. A pharmaceutical composition for use as claimed in any preceding claim, wherein the brain injury is subarachnoid haemorrhage.

9. A pharmaceutical composition for use as claimed in any preceding claim, wherein said method comprises administration of the composition to a cerebral ventricle or to the subarachnoid space.

10. A pharmaceutical composition for use as claimed in claim 9, wherein said method comprises administration of the composition by injection into the lateral, third and/or fourth ventricles of the brain or into the subarachnoid cisterns of the brain.

11. A pharmaceutical composition for use as claimed in any preceding claim, wherein the voltage-gated calcium channel blocker is a dihydropyridine calcium channel blocker such as nimodipine.

12. A pharmaceutical composition for use as claimed in any preceding claim, wherein the composition is adapted for sustained or delayed release of the voltage-gated calcium channel blocker.

13. A pharmaceutical composition for use as claimed in any preceding claim, wherein the composition is adapted to exert a predominantly localized pharmacologic effect.

14. A pharmaceutical composition for use as claimed in any preceding claim, wherein the treating involves preventing or reducing the incidence or severity of the delayed complication in the mammal.

# FIG. 1

## FIG. 2A

## FIG. 2B

## FIG. 2C

FIG. 3

**FIG. 4A**

**FIG. 4B**

## FIG. 5
### ARTERIES OF BRAIN (FRONTAL VIEW AND SECTION)

CORPUS CALLOSUM

MEDIAL AND LATERAL LENTICULOSTRIATE aa.

LATERAL ORBITOFRONTAL a.

ASCENDING FRONTAL (CANDELABRA) BRANCH

PRECENTRAL (PREROLANDIC) AND CENTRAL (ROLANDIC) BRANCHES

ANT. AND POST. PARIETAL BRANCHES

ANGULAR BRANCH

TEMPORAL BRANCHES (POST., MIDDLE, ANT.)

MIDDLE CEREBRAL ARTERY AND BRANCHES, DEEP IN LATERAL CEREBRAL (SYLVIAN) FISSURE

ANT. COMMUNICATING a.

POST. COMMUNICATING a.

SUP. CEREBELLAR a.

ANT. INF. CEREBELLAR a.

POST. SPINAL a.

PARACENTRAL a.

FRONTAL BRANCHES

PERICALLOSAL a.

CALLOSOMARGINAL a.

FRONTOPOLAR a.

ANTERIOR CEREBRAL ARTERIES

MEDIAL ORBITOFRONTAL a.

RECURRENT a. (OF HEUBNER)

INT. CAROTID a.

ANT. CHOROIDAL a.

POSTERIOR CEREBRAL ARTERY

BASILAR a.

INT. AUDITORY (LABYRINTHINE) a.

VERTEBRAL a.

POST. INT. CEREBELLAR a.

ANT. SPINAL a.

EP 3 222 284 A1

## FIG. 6

INTERTHALAMIC ADHESION

FORNIX

CENTRAL PART OF LATERAL VENTRICLE

SUPRAPINEAL RECESS

INTERVENTRICULAR FORAMEN

PINEAL RECESS

THIRD VENTRICLE

PINEAL BODY

CORPUS CALLOSUM

CEREBRAL AQUEDUCT

ANTERIOR HORN OF LATERAL VENTRICLE

COLLATERAL TRIGONE

SUPRAOPTIC RECESS

OPTIC CHIASM

POSTERIOR HORN OF LATERAL VENTRICLE

INFUNDIBULAR RECESS

PITUITARY

INFERIOR HORN OF LATERAL VENTRICLE

FOURTH VENTRICLE

LATERAL RECESS, ENDS IN LATERAL APERTURE OF FOURTH VENTRICLE

MEDIAN APERTURE OF FOURTH VENTRICLE

CENTRAL CANAL

## FIG. 7

ARACHNOID GRANULATIONS

CHOROID PLEXUS OF LATERAL VENTRICLE

CHOROID PLEXUS OF THIRD VENTRICLE

SUPERIOR SAGITTAL SINUS

AMBIENT CISTERN

INTER-HEMISPHERIC CISTERN

STRAIGHT SINUS

INTER-VENTRICULAR FORAMEN

CONFLUENCE OF THE SINUSES

CISTERN OF LAMINA TERMINALIS

VERMIAN CISTERN

BASAL CISTERN { CHIASMATIC CISTERN

INTERPEDUNCULAR CISTERN }

CHOROID PLEXUS OF FOURTH VENTRICLE

CEREBRAL AQUEDUCT

CEREBELLOMEDULLARY CISTERN (CISTERNA MAGNA)

PONTOMEDULLARY CISTERN

MEDIAN APERTURE

CENTRAL CANAL

EP 3 222 284 A1

FIG. 8A

FIG. 8B

MIDPUPILLARY
LINE

FIG. 8C

# FIG. 9

# FIG. 10

FIG. 11
BEHAVIOR SCORE

## FIG. 12

A                    B

**FIGURE 13**

## FIG. 14

CEREBRAL CORTEX

NERVE FIBRES JOINING RIGHT AND LEFT CEREBRAL HEMISPHERES

CEREBRUM

CEREBELLUM

SPINAL CORD

3rd VENTRICLE

THALAMUS

HYPOTHALAMUS

PONS

PITUITARY GLAND

MEDULLA OBLONGATA

EP 3 222 284 A1

FIG. 15

## FIG. 16

## FIG. 17

Europäisches
Patentamt

European
Patent Office

Office européen
des brevets

**EUROPEAN SEARCH REPORT**

Application Number

EP 17 16 6614

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOCH MACDONALD R ET AL: "Site-specific, Sustained-release Nimodipine to Prevent Vasospasm", INTERNET CITATION, 4 October 2010 (2010-10-04), pages 1-2, XP002730578, Retrieved from the Internet: URL:http://www.edgetherapeutics.com/wp-content/uploads/2010/10/Site-specific-Sustained-release-Nimodipine-to-Prevent-Vasospasm.pdf [retrieved on 2014-10-01] * second paragraph on page 2 * * first two paragraphs on the page 1 * | 1-14 | INV. A61K38/16 A61K9/16 A61K9/00 A61K31/4422 A61K9/06 |
| X | HÄNGGI D ET AL: "The effect of an intracisternal nimodipine slow-release system on cerebral vasospasm after experimental subarachnoid haemorrhage in the rat", 1 January 2008 (2008-01-01), CERABRAL VASOSPASM. NEW STRATEGIES IN RESEARCH AND TREATMENT; [ACTA NEUROCHIRURGICA SUPPLEMENTUM; ISSN 0065-1419; VOL. 104], SPRINGER, AT, PAGE(S) 103 - 107, XP008176257, ISBN: 978-3-211-75717-8 * table 1 * | 1-14 | |
| | | | TECHNICAL FIELDS SEARCHED (IPC) |
| | | | A61K |
| A | US 2004/105888 A1 (PRATT DANIEL [US] ET AL) 3 June 2004 (2004-06-03) * paragraph [0069]; examples 2-3 * | 1-14 | |
| A | US 5 993 855 A (YOSHIMOTO TAKASHI [JP] ET AL) 30 November 1999 (1999-11-30) * column 4 - column 6; example 2 * | 1-14 | |

-/--

The present search report has been drawn up for all claims

| Place of search | Date of completion of the search | Examiner |
|---|---|---|
| The Hague | 16 August 2017 | Frelichowska, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 17 16 6614

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2008/305147 A1 (MACDONALD R LOCH [CA] ET AL) 11 December 2008 (2008-12-11) * paragraph [0187] - paragraph [0192]; claims 1, 27 * | 1-14 | |

TECHNICAL FIELDS
SEARCHED (IPC)

The present search report has been drawn up for all claims

| Place of search | Date of completion of the search | Examiner |
|---|---|---|
| The Hague | 16 August 2017 | Frelichowska, J |

## ANNEX TO THE EUROPEAN SEARCH REPORT
## ON EUROPEAN PATENT APPLICATION NO.

EP 17 16 6614

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2017

| Patent document cited in search report | | Publication date | Patent family member(s) | | Publication date |
|---|---|---|---|---|---|
| US 2004105888 | A1 | 03-06-2004 | AU | 2003298749 A1 | 18-06-2004 |
| | | | CA | 2547531 A1 | 10-06-2004 |
| | | | EP | 1575562 A2 | 21-09-2005 |
| | | | US | 2004105888 A1 | 03-06-2004 |
| | | | US | 2011176994 A1 | 21-07-2011 |
| | | | WO | 2004047768 A2 | 10-06-2004 |
| US 5993855 | A | 30-11-1999 | EP | 0765659 A1 | 02-04-1997 |
| | | | JP | 2909418 B2 | 23-06-1999 |
| | | | JP | H0977657 A | 25-03-1997 |
| | | | US | 5993855 A | 30-11-1999 |
| US 2008305147 | A1 | 11-12-2008 | AU | 2008261668 A1 | 18-12-2008 |
| | | | CA | 2690600 A1 | 18-12-2008 |
| | | | CN | 101873858 A | 27-10-2010 |
| | | | CN | 105055300 A | 18-11-2015 |
| | | | CN | 105963242 A | 28-09-2016 |
| | | | EP | 2164497 A1 | 24-03-2010 |
| | | | IL | 202647 A | 26-02-2015 |
| | | | JP | 5576790 B2 | 20-08-2014 |
| | | | JP | 2010529205 A | 26-08-2010 |
| | | | KR | 20100055385 A | 26-05-2010 |
| | | | KR | 20150015514 A | 10-02-2015 |
| | | | KR | 20150061031 A | 03-06-2015 |
| | | | NZ | 581836 A | 27-07-2012 |
| | | | NZ | 599430 A | 28-03-2014 |
| | | | US | 2008305147 A1 | 11-12-2008 |
| | | | US | 2013022660 A1 | 24-01-2013 |
| | | | WO | 2008154585 A1 | 18-12-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61441695 A
- US 13732008 A
- US 97690207 P
- US 94312407 P
- US 5407609 A
- US 553003 A
- US 799700 A
- US 557946 A
- US 779138 A
- US 562455 A
- US 338488 A
- US 692027 A
- US 692020 A
- US 565401 A
- US 692029 A
- US 968708 A
- US 074542 A
- US 5399665 A
- US 4757128 A
- US 5977163 A
- US 5747058 A
- US 5968542 A

**Non-patent literature cited in the description**

- **ERIC R. KANDEL ; JAMES H. SCHWARTZ.** Principles of Neural Sciences. Elsevier Science Publishing Co., Inc, 1985, 854-56
- **MACDONALD, R.L. ; WEIR, B.** In Cerebral Vasospasm. Academic Press, 2001
- **SANDS, Z. et al.** Voltage-gated ion channels. *Current Biology,* 2005, vol. 15 (2), R44-R47
- **VAN PETEGEM F. et al.** *Biochemical Society Transactions,* 2006, vol. 34 (5), 887-893
- **CATTERALL W. A. et al.** International Union of Pharmacology. XLVIII. Nomenclature and structure-function relationships of voltage-gated calcium channels. *Pharmacol. Rev.,* 2005, vol. 57 (4), 411-25
- **YAMAKAGE M. et al.** Calcium channels--basic aspects of their structure, function and gene encoding; anesthetic action on the channels--a review. *Can. J. Anaesth.,* 2002, vol. 49 (2), 151-64
- **DOLPHIN A. C.** A short history of voltage-gated calcium channels. *Br. J. Pharmacol.,* 2006, vol. 147 (1), 56-62
- **CHU P. J. et al.** Calcium channel gamma subunits provide insights into the evolution of this gene family. *Gene,* 2002, vol. 280 (1-2), 37-48
- **WINDS, R. et al.** *J. Physiol. (Lond.),* 1980, vol. 305, 171-95
- **LLINDS, R. et al.** *Proc. Natl. Acad. Sci. U.S.A.,* 1989, vol. 86 (5), 1689-93
- **YAMAKAGE M. et al.** Calcium channels-basic aspects of their structure, function and gene encoding; anesthetic action on the channels--a review. *Can. J. Anaesth.,* 2002, vol. 49 (2), 151-64
- *J. Biol. Chem.,* 2000, vol. 275, 21309
- *Biochemistry,* 1998, vol. 37, 15353
- *Peptides,* 1999, vol. 1998, 748
- *J. Neurosci.,* 2000, vol. 20, 171
- *J. Neurosci.,* 1999, vol. 19, 9235
- The CIBA Collection of Medical Illustrations. **NETTER FH.** Nervous System. CIBA, 1986, vol. 1, 256
- **SCHUENKE M ; SCHULTE E ; SCHUMACHER U.** Thieme Atlas of Anatomy. Georg Thieme Verlag, 2006, 192, , 541
- **SCHUENKE M ; SCHULTE E ; SCHUMACHER U.** Thieme Atlas of Anatomy. Georg Thieme Verlag, 2006, 194, , 541
- Techniques of CSF diversion. **MCCOMB JG.** Hydrocephalus. Williams & Wilkins, 1990, vol. 3, 48, , 128
- Cerebrospinal fluid. **POLLAY M.** The Practice of Neurosurgery. Williams & Wilkins, 1996, vol. 1, 36, , 1381
- **DREIER, J.P. et al.** *Brain,* 2009, vol. 132, 1866-81
- FUNDAMENTAL IMMUNOLOGY. Lippincott-Raven Publishers, 1999, 1051-1053
- **COPLIN et al.** *Arch Neurol,* 1999, vol. 56, 1348-1352
- **LANGER.** *Science,* 1990, vol. 249, 1527-1533
- **SAWHNEY et al.** *Macromolecules,* 1993, vol. 26, 581-587